(19) Europäisches Patentamt / European Patent Office / Office européen des brevets

(11) **EP 3 733 204 A1**

(12) **EUROPEAN PATENT APPLICATION**
published in accordance with Art. 153(4) EPC

(43) Date of publication:
**04.11.2020 Bulletin 2020/45**

(21) Application number: **18895064.6**

(22) Date of filing: **27.12.2018**

(51) Int Cl.:
*A61K 45/00* (2006.01)   *A61K 31/55* (2006.01)
*A61K 31/553* (2006.01)   *A61P 1/12* (2006.01)
*A61P 13/00* (2006.01)   *A61P 43/00* (2006.01)

(86) International application number:
**PCT/JP2018/048229**

(87) International publication number:
**WO 2019/131902 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.12.2017 JP 2017250938**
**30.03.2018 JP 2018067231**

(71) Applicant: **Takeda Pharmaceutical Company Limited**
**Osaka-shi,**
**Osaka 541-0045, (JP)**

(72) Inventors:
• **KAMO Izumi**
  **Fujisawa-shi, Kanagawa 251-0012 (JP)**
• **MUELLER-PLOCK Nele**
  **78464 Konstanz (DE)**

(74) Representative: **Jones, Nicholas Andrew**
**Withers & Rogers LLP**
**4 More London Riverside**
**London, SE1 2AU (GB)**

(54) **THERAPEUTIC AGENT FOR STRESS URINARY INCONTINENCE AND FECAL INCONTINENCE**

(57)    The present invention provides a medicament for use in treating stress urinary incontinence with fewer adverse effects including a body weight lowering effect. The present invention also provides a medicament for use in treating a disease such as incontinence of feces and further provide a medicament for use in treating a disease such as incontinence of feces with fewer adverse effects including a body weight lowering effect.

A medicament for use in treating stress urinary incontinence, comprising a 5-HT$_{2C}$ receptor agonist, wherein the medicament is administered at a dosage lower than the minimum dosage of the agonist as an anti-obesity drug. A medicament for use in treating incontinence of feces, etc., comprising a 5-HT$_{2C}$ receptor agonist. A medicament for use in treating incontinence of feces, comprising a 5-HT$_{2C}$ receptor agonist, wherein the medicament is administered at a dosage lower than the minimum dosage of the agonist as an anti-obesity drug.

[Figure 2]

**Description**

[Technical Field]

The present invention relates to a therapeutic agent for incontinence of feces. The present invention also relates to a therapeutic agent for stress urinary incontinence with fewer adverse effects including a body weight lowering effect. The present invention further relates to a therapeutic agent for a disease such as incontinence of feces and a therapeutic agent for incontinence of feces with fewer adverse effects including a body weight lowering effect.

[Background Art]

Serotonin receptors are classified into 10 or more subtypes, most of which are expressed in the central nervous system and known as targets for antidepressants. $5\text{-HT}_{2C}$ receptors included in one of these subtypes (hereinafter also referred to as "serotonin $5\text{-HT}_{2C}$ receptor") are expressed mainly in the central nervous system, and phenotype alterations exhibited in their knockout mice imply that these receptors are involved in phenomena such as feeding, sexual functions, and social activities. Particularly, the activation of $5\text{-HT}_{2C}$ receptors in the hypothalamus is considered to suppress feeding and to lower body weight (Non Patent Literature 1).

For example, *N*-methyl-*N*-(1-methylethyl)-6,7,8,9-tetrahydropyrazino[2,3-*f*][1,4]oxazepine-3-amine (hereinafter, also referred to as "Compound A"), (*R*)-8-chloro-1-methyl-2,3,4,5-tetrahydro-1H-3-benzazepine (hereinafter, also referred to as "Compound B") (Patent Literatures 1 to 5), and compounds described in technical literatures below are known as selective agonists for $5\text{-HT}_{2C}$ receptors.

The hydrochloride salt of Compound B (hereinafter, this hydrochloride salt of Compound B is also referred to as "Compound B'") is sold as an anti-obesity drug in the US. Compound B', however, is known to manifest severe adverse effects, such as headache and nausea, with increasing dose, whereas its administration at a dosage giving no adverse effects has been found to be insufficient in terms of an anti-obesity effect (Non Patent Literatures 2 to 4).

Additionally, $5\text{-HT}_{2C}$ receptor agonists are known to exert a urethral resistance increasing effect as a consequence of enlarging the urethral-closing reflex (Patent Literatures 1, 2, 4, and 5). It is clarified that when a $5\text{-HT}_{2C}$ agonist enhances urethra-closing responses, at least it increases contractile responses of pelvic floor muscles (Patent Literatures 1, 4 and 6). Pelvic floor muscle training exercises, which strengthen the pelvic floor muscles, are known to be effective for diseases such as incontinence of feces, prolapse of various organs, and dribbling after urination (Non Patent Literatures 5, 6 and 7). It is known that the urethra and the rectum-the anus are closed upon the pelvic floor muscles contraction (Non Patent Literature 7).

[Citation List]

[Patent Literature]

[Patent Literature 1] WO2010/147226
[Patent Literature 2] WO2006/022420
[Patent Literature 3] WO2003/086306
[Patent Literature 4] WO2007/132841
[Patent Literature 5] WO2011/111817
[Patent Literature 6] WO2008/108445

[Non Patent Literature]

[Non Patent Literature 1] Expert Opinion on Investigational Drugs, 2006, Vol. 15, p. 257-266
[Non Patent Literature 2] LORCASERIN HYDROCHLORIDE, Briefing Document for FDA Advisory Committee Meeting, published on April 6, 2012, published by Center for Drug Evaluation and Research, Food and Drug Administration (http://www.fda.gov/downloads/AdvisoryCommittees/CommitteesMeetingMaterials/ Drugs/EndocrinologicandMetabolicDrugsAdvisoryCommittee/UCM303200.pdf)
[Non Patent Literature 3] Clinical Therapeutics 2016, Vol. 38, No. 10, p2227-2238 [Non Patent Literature 4] FDA Briefing Document : NDA 22529, September 16, 2010, published by FDA
[Non Patent Literature 5] World Journal of Urology 2012, Vol. 30, p. 437-43

[Non Patent Literature 6] British Journal of Urology 1997, Vol. 79, p. 892-7
[Non Patent Literature 7] Central European Journal of Urology 2011, Vol. 64, p. 110-9

[Summary of Invention]

[Technical Problem]

An object of the present invention is to provide a therapeutic agent for stress urinary incontinence. Another object of the present invention is to provide a therapeutic agent for stress urinary incontinence that can be administered even to stress urinary incontinence patients having no obesity. A further object of the present invention is to provide a therapeutic agent for stress urinary incontinence with fewer adverse effects such as headache and nausea.
A further object of the present invention is to provide a therapeutic agent for a disease such as incontinence of feces. A further object of the present invention is to provide a therapeutic agent for incontinence of feces that can be administered even to patients of incontinence of feces having no obesity. A further object of the present invention is to provide a therapeutic agent for incontinence of feces with fewer adverse effects such as headache and nausea.

[Solution to Problem]

The present inventors have found that the urethral resistance increasing effect of 5-HT$_{2C}$ receptor agonists, unlike their anti-obesity effect, originates in the spinal cord. The present inventors have also found that unexpected lower plasma concentrations of 5-HT$_{2C}$ receptor agonists than their concentrations in plasma (hereinafter also referred to as "plasma concentration" or "concentration in blood") exerting a meaningful anti-obesity effect (body weight lowering effect) suffice to increase urethral resistance based on the activation of 5-HT$_{2C}$ receptors in the spinal cord, hence, 5-HT$_{2C}$ receptor agonists are capable of treating stress urinary incontinence at unexpectedly low dosages that fall below the dosages prescribed as anti-obesity drugs.
Further, the 5-HT$_{2C}$ receptor agonist may be useful for treating not only stress urinary incontinence but a disease such as incontinence of feces, prolapse of various organs, or dribbling after urination, whose pathogenesis is considered to partly involve a weakened pelvic floor muscles and the present inventors have found that diseases such as incontinence of feces can be treated with 5-HT$_{2C}$ receptor agonists. Tube organs such as the urethra, the rectum-the anus, and the vagina can close simultaneously by the same mechanism in which reflex pelvic floor muscle contractions are mediated, and 5-HT$_{2C}$ receptor agonists enhance the reflex pelvic floor muscle contractions. Therefore, 5-HT$_{2C}$ receptor agonists such as compound A' and compound B' can simultaneously enhance the closures of the tube organs such as the urethra, the rectum-the anus, and the vagina via a pelvic floor muscle contraction. Based on these findings, the inventors further discover that prolapse of various organs and incontinence of feces can be treated with 5-HT$_{2C}$ receptor agonists at the same plasma level as the effective plasma level for stress urinary incontinence.
The present invention is based on these findings.
Specifically, the present invention provides the following aspects:

[1] A medicament for use in treating or preventing incontinence of feces, comprising a 5-HT$_{2C}$ receptor agonist.
[2] The medicament according to above [1], wherein the 5-HT$_{2C}$ receptor agonist is *N*-methyl-*N*-(1-methylethyl)-6,7,8,9-tetrahydropyrazino[2,3-*f*][1,4 ]oxazepine-3-amine or a salt thereof.
[3] The medicament according to above [1], wherein the 5-HT$_{2C}$ receptor agonist is (*R*)-8-chloro-1-methyl-2,3,4,5-tetrahydro-1H-3-benzazepine or a salt thereof.
[4] A medicament for use in treating stress urinary incontinence, comprising a 5-HT$_{2C}$ receptor agonist, wherein the medicament is administered at a dosage lower than the minimum dosage of the agonist as an anti-obesity drug.
[5] The medicament according to above [4], wherein the 5-HT$_{2C}$ receptor agonist is *N*-methyl-*N*-(1-methylethyl)-6,7,8,9-tetrahydropyrazino[2,3-*f*][1,4]oxazepine-3-amine or a salt thereof.
[6] The medicament according to above [5], wherein the medicament is administered such that the plasma concentration of the 5-HT$_{2C}$ receptor agonist is more than 2 ng/mL and less than 203 ng/mL for the duration from 1 hour to 24 hours.
[7] The medicament according to above [5], wherein the medicament is administered such that the plasma concentration of the 5-HT$_{2C}$ receptor agonist is more than 2 ng/mL and equal to or less than 116 ng/mL for the duration from 1 hour to 24 hours.
[8] The medicament according to above [4], wherein the 5-HT$_{2C}$ receptor agonist is (*R*)-8-chloro-1-methyl-2,3,4,5-tetrahydro-1H-3-benzazepine or a salt thereof.
[9] The medicament according to above [8], wherein the medicament is administered such that the plasma concentration of the 5-HT$_{2C}$ receptor agonist is more than 0.32 ng/mL and less than 43 ng/mL for the duration from 1 hour to 24 hours.

[10] The medicament according to above [8], wherein the medicament is administered such that the plasma concentration of the 5-HT$_{2C}$ receptor agonist is more than 0.32 ng/mL and equal to or less than 24 ng/mL for the duration from 1 hour to 24 hours.

[11] The medicament according to above [8], wherein the medicament is administered at a daily dose ranging between 0.1 mg and 10 mg.

[12] A medicament for use in treating stress urinary incontinence, comprising a therapeutically effective amount of a 5-HT$_{2C}$ receptor agonist for treating stress urinary incontinence, wherein the therapeutically effective amount of the agonist shows no body weight lowering effect.

[13] The medicament according to above [12], wherein the 5-HT$_{2C}$ receptor agonist is N-methyl-N-(1-methylethyl)-6,7,8,9-tetrahydropyrazino[2,3-f][1,4]oxazepine-3-amine or a salt thereof.

[14] The medicament according to above [13], wherein the medicament is administered such that the plasma concentration of the 5-HT$_{2C}$ receptor agonist is more than 2 ng/mL and less than 203 ng/mL for the duration from 1 hour to 24 hours.

[15] The medicament according to above [13], wherein the medicament is administered such that the plasma concentration of the 5-HT$_{2C}$ receptor agonist is more than 2 ng/mL and equal to or less than 116 ng/mL for the duration from 1 hour to 24 hours.

[16] The medicament according to above [12], wherein the 5-HT$_{2C}$ receptor agonist is (R)-8-chloro-1-methyl-2,3,4,5-tetrahydro-1H-3-benzazepine or a salt thereof.

[17] The medicament according to above [16], wherein the medicament is administered such that the plasma concentration of the 5-HT$_{2C}$ receptor agonist is more than 0.32 ng/mL and less than 43 ng/mL for the duration from 1 hour to 24 hours.

[18] The medicament according to above [16], wherein the medicament is administered such that the plasma concentration of the 5-HT$_{2C}$ receptor agonist is more than 0.32 ng/mL and equal to or less than 24 ng/mL for the duration from 1 hour to 24 hours.

[19] The medicament according to above [16], wherein the 5-HT$_{2C}$ receptor agonist is administered at a total daily dose ranging between 0.1 mg and 10 mg.

[20] A medicament for use in treating incontinence of feces, comprising a 5-HT$_{2C}$ receptor agonist.

[21] A medicament for use in treating incontinence of feces, comprising a 5-HT$_{2C}$ receptor agonist, wherein the medicament is administered at a dosage lower than the minimum dosage of the agonist as an anti-obesity drug.

[22] The medicament according to above [21], wherein the 5-HT$_{2C}$ receptor agonist is N-methyl-N-(1-methylethyl)-6,7,8,9-tetrahydropyrazino[2,3-f][1,4]oxazepine-3-amine or a salt thereof.

[23] The medicament according to above [22], wherein the medicament is administered such that the plasma concentration of the 5-HT$_{2C}$ receptor agonist is more than 2 ng/mL and less than 203 ng/mL for the duration from 1 hour to 24 hours.

[24] The medicament according to above [22], wherein the medicament is administered such that the plasma concentration of the 5-HT$_{2C}$ receptor agonist is more than 2 ng/mL and equal to or less than 116 ng/mL for the duration from 1 hour to 24 hours.

[25] The medicament according to above [21], wherein the 5-HT$_{2C}$ receptor agonist is (R)-8-chloro-1-methyl-2,3,4,5-tetrahydro-1H-3-benzazepine or a salt thereof.

[26] The medicament according to above [25], wherein the medicament is administered such that the plasma concentration of the 5-HT$_{2C}$ receptor agonist is more than 0.32 ng/mL and less than 43 ng/mL for the duration from 1 hour to 24 hours.

[27] The medicament according to above [25], wherein the medicament is administered such that the plasma concentration of the 5-HT$_{2C}$ receptor agonist is more than 0.32 ng/mL and equal to or less than 24 ng/mL for the duration from 1 hour to 24 hours.

[28] The medicament according to above [25], wherein the 5-HT$_{2C}$ receptor agonist is administered at a total daily dose ranging between 0.1 mg and 10 mg.

[29] A medicament for use in treating incontinence of feces, comprising a therapeutically effective amount of a 5-HT$_{2C}$ receptor agonist for treating incontinence of feces, wherein the therapeutically effective amount of the agonist shows no body weight lowering effect.

[30] The medicament according to above [29], wherein the 5-HT$_{2C}$ receptor agonist is N-methyl-N-(1-methylethyl)-6,7,8,9-tetrahydropyrazino[2,3-f][1,4]oxazepine-3-amine or a salt thereof.

[31] The medicament according to above [30], wherein the medicament is administered such that the plasma concentration of the 5-HT$_{2C}$ receptor agonist is more than 2 ng/mL and less than 203 ng/mL for the duration from 1 hour to 24 hours.

[32] The medicament according to above [30], wherein the medicament is administered such that the plasma concentration of the 5-HT$_{2C}$ receptor agonist is more than 2 ng/mL and equal to or less than 116 ng/mL for the duration from 1 hour to 24 hours.

[33] The medicament according to above [29], wherein the 5-HT$_{2C}$ receptor agonist is (R)-8-chloro-1-methyl-2,3,4,5-tetrahydro-1H-3-benzazepine or a salt thereof.

[34] The medicament according to above [33], wherein the medicament is administered such that the plasma concentration of the 5-HT$_{2C}$ receptor agonist is more than 0.32 ng/mL and less than 43 ng/mL for the duration from 1 hour to 24 hours.

[35] The medicament according to above [34], wherein the medicament is administered such that the plasma concentration of the 5-HT$_{2C}$ receptor agonist is more than 0.32 ng/mL and equal to or less than 24 ng/mL for the duration from 1 hour to 24 hours.

[36] The medicament according to above [33], wherein the medicament is administered at a daily dose ranging between 0.1 mg and 10 mg.

[37] A method of treating or preventing incontinence of feces in a subject in need thereof, comprising administering to the subject an effective amount of a 5-HT$_{2C}$ receptor agonist.

[38] A method of treating or preventing stress urinary incontinence or incontinence of feces in a subject in need thereof, comprising administering to the subject an effective amount of a 5-HT$_{2C}$ receptor agonist, wherein the effective amount is a dosage lower than the minimum dosage of the agonist as an anti-obesity drug.

[39] A method of treating or preventing stress urinary incontinence or incontinence of feces in a subject in need thereof, comprising administering to the subject an effective amount of a 5-HT$_{2C}$ receptor agonist, wherein the effective amount of the agonist shows no body weight lowering effect.

[40] A 5-HT$_{2C}$ receptor agonist for use in a method of treating or preventing incontinence of feces.

[41] A 5-HT$_{2C}$ receptor agonist for use in a method of treating or preventing stress urinary incontinence or incontinence of feces, characterized in that the agonist is administered at a dosage lower than the minimum dosage of the agonist as an anti-obesity drug.

[42] A 5-HT$_{2C}$ receptor agonist for use in a method of treating or preventing stress urinary incontinence or incontinence of feces, characterized in that the agonist is administered at a dosage that show no body weight lowering effect.

[43] Use of a 5-HT$_{2C}$ receptor agonist in a manufacture of a medicament for use in treating or preventing incontinence of feces.

[44] Use of a 5-HT$_{2C}$ receptor agonist in a manufacture of a medicament for use in treating or preventing stress urinary incontinence or incontinence of feces.

[45] Use of a 5-HT$_{2C}$ receptor agonist in a manufacture of a medicament for use in treating or preventing stress urinary incontinence or incontinence of feces, characterized in that the agonist is administered at a dosage that show no body weight lowering effect.

[Advantageous Effects of Invention]

According to the present invention, 5-HT$_{2C}$ receptor agonists can be administered at doses producing substantially no body weight lowering effect and can therefore be administered even to stress urinary incontinence patients having no obesity. Moreover, the medicament of the present invention may elicit reduced adverse effects in comparison to that associated with the administration of 5-HT$_{2C}$ receptor agonists as an anti-obesity drug and may therefore prevent reduction in quality of life (QOL) of patients during the treatment of their stress urinary incontinence. Furthermore, according to the present invention, diseases such as incontinence of feces may be treated. According to the present invention, 5-HT$_{2C}$ receptor agonists can be administered at doses producing substantially no body weight lowering effect and can therefore be administered even to patients of incontinence of feces having no obesity. Moreover, the medicament of the present invention may elicit reduced adverse effects in comparison to that associated with the administration of 5-HT$_{2C}$ receptor agonists as an anti-obesity drug and may therefore prevent reduction in quality of life (QOL) of patients during the treatment of their incontinence of feces.

[Brief Description of Drawings]

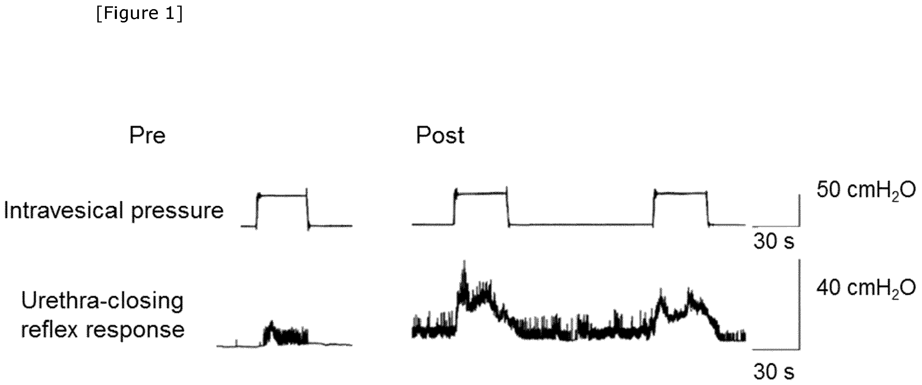

[Figure 1] Figure 1 shows results of an experiment in which the degree of urethra-closing reflex response to increased intravesical pressure before administration of hydrochloride salt of Compound A (hereinafter, this hydrochloride salt of Compound A is also referred to as "Compound A'") (Pre) was compared with that after administration of Compound A' (Post).

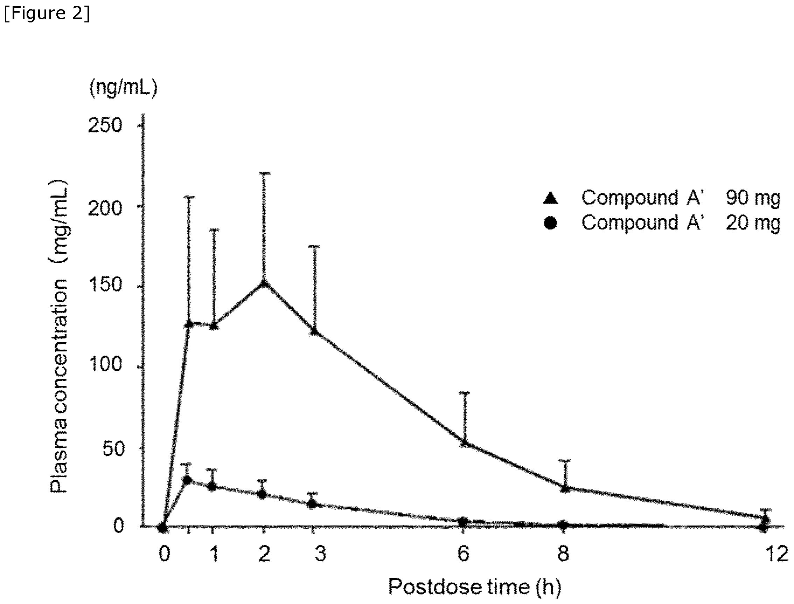

[Figure 2] Figure 2 shows the time-dependent change in the plasma concentration of Compound A in healthy female volunteers who received a single dose of 20 mg or 90 mg of Compound A'.

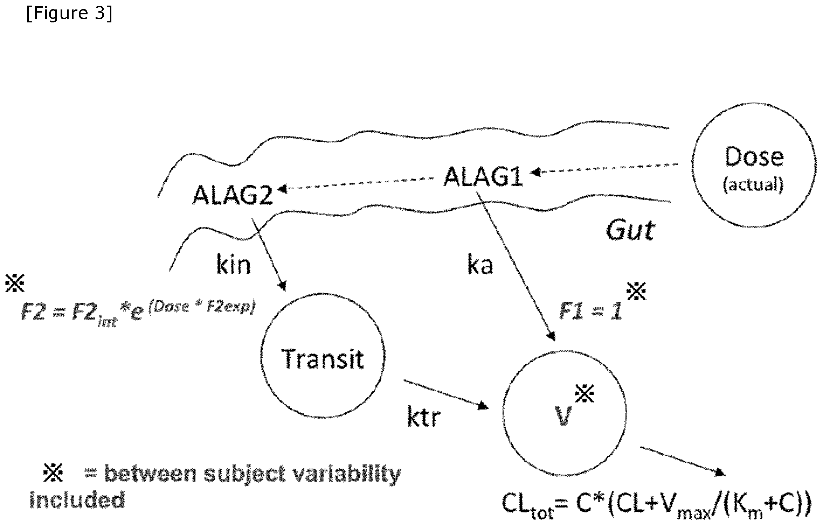

[Figure 3] Figure 3 shows a schematic overview of the final PK model of Compound A. In Figure 3, the terms are defined as follows. Dose: dosing compartment (i.e., actual free base dose); V: Central volume of distribution (i.e., observation compartment Compound A); ka: first order absorption rate constant; kin and ktr: first order absorption rate constants; CL: Clearance; Vmax: maximum elimination rate; Km: concentration at half the Vmax; C: concentration Compound A; ALAG1 and ALAG2: lag times; F2int: base bioavailability fraction; F2exp: exponent of bioavailability fraction.

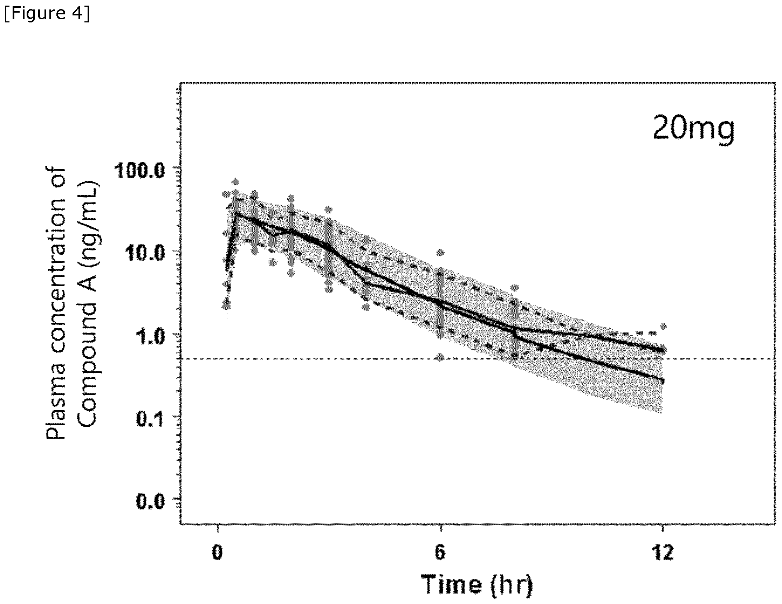

[Figure 4] Figure 4 shows a diagram for visual predictive check of Compound A plasma concentration-time profile after a single dose of 20 mg in the Japanese population. In Figure 4, the dots depict observed values. The blue solid line depicts the median of the observed values. The red dotted line depicts 5 percentile values and 95 percentile values. The black solid line depicts a predicted median. The shaded area depicts 90% prediction interval.

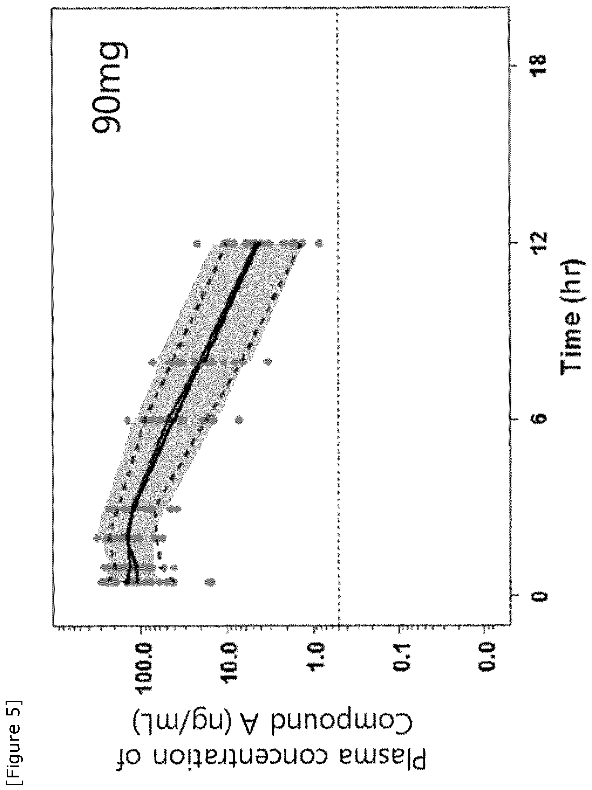

[Figure 5] Figure 5 shows a diagram for visual predictive check of Compound A plasma concentration-time profile after a single dose of 90 mg in the Japanese population. In Figure 5, the dots depict observed values. The blue solid line depicts the median of the observed values. The red dotted line depicts 5 percentile values and 95 percentile values. The black solid line depicts a predicted median. The shaded area depicts 90% prediction interval.

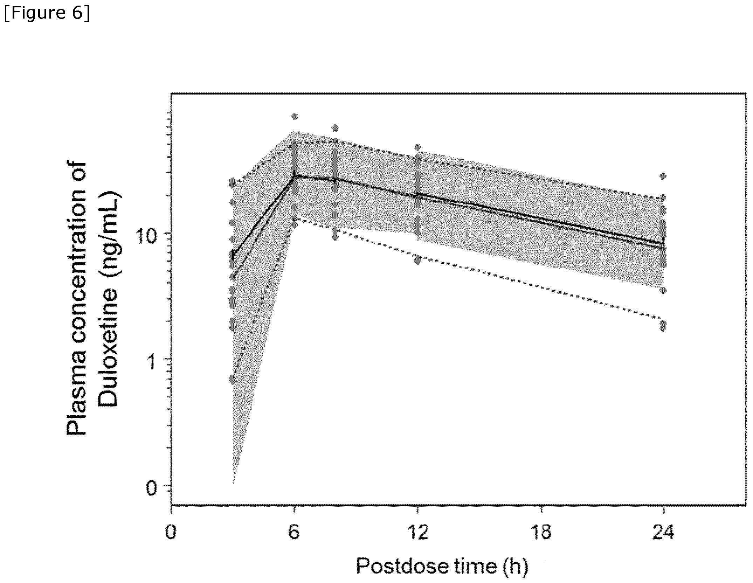

[Figure 6] Figure 6 shows a diagram for visual predictive check of Duloxetine plasma concentration-time profile after a single dose of 40 mg in the Japanese population. In Figure 6, the dots depict observed values. The blue solid line depicts the median of the observed values. The red dotted line depicts 5 percentile values and 95 percentile values. The black solid line depicts a predicted median. The shaded area depicts 90% prediction interval.

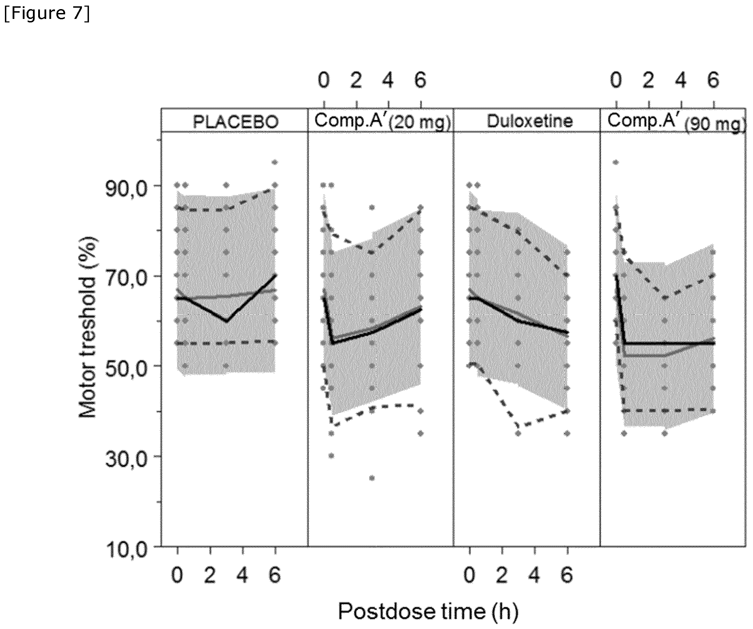

[Figure 7] Figure 7 shows predicted % MT-time profiles obtained from single doses of a placebo, 20 mg or 90 mg of Compound A', and duloxetine hydrochloride salt (hereinafter also abbreviated as "duloxetine", while the term "duloxetine" in the context of concentration in blood indicates "the free form of duloxetine"). In Figure 7, the dots depict observed values. The blue solid line depicts the median of the observed values. The red dotted line depicts 5 percentile values and 95 percentile values. The black solid line depicts a predicted median. The shaded area depicts 90% prediction interval.

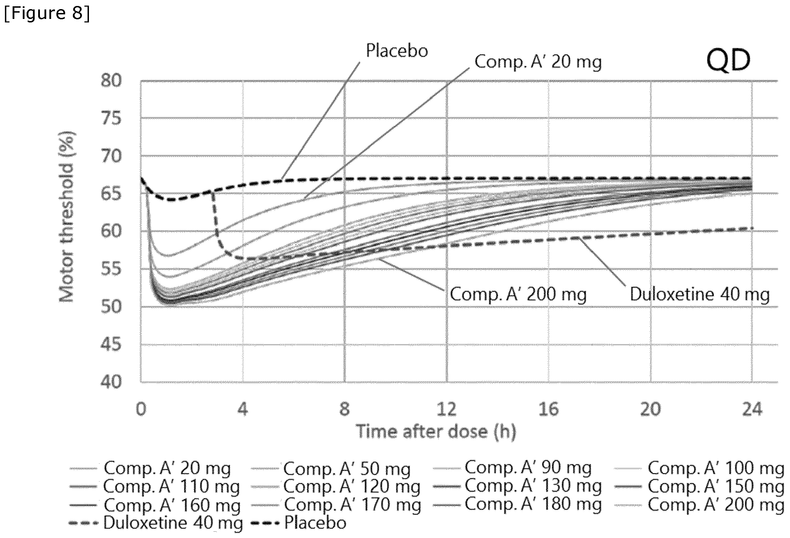

[Figure 8] Figure 8 shows results of a simulation of the %MT-time profile following 40 mg Duloxetine and following the indicated dose of Compound A' (QD) administration for the US population. In this figure, "Comp. A'" indicates Compound A'.

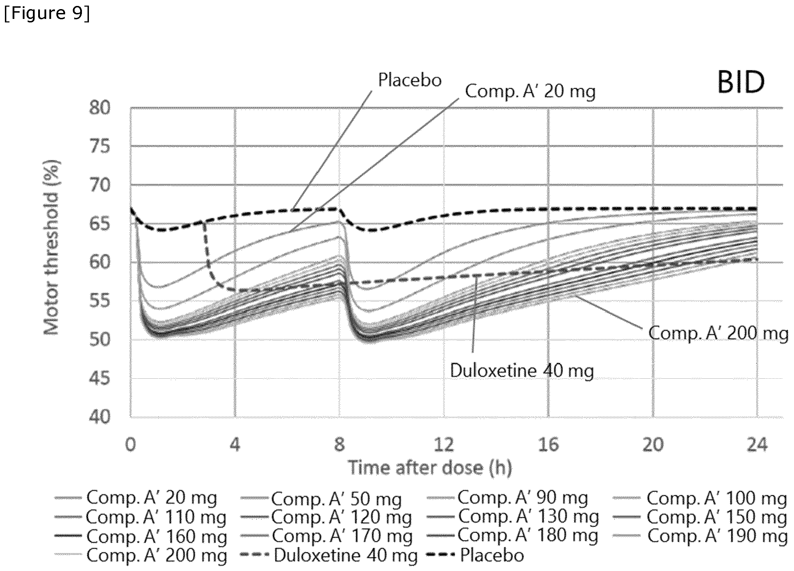

[Figure 9] Figure 9 shows results of a simulation of the %MT-time profile following 40 mg Duloxetine and following the indicated dose of Compound A' (BID) administration for the US population. In this figure, "Comp. A'" indicates Compound A'.

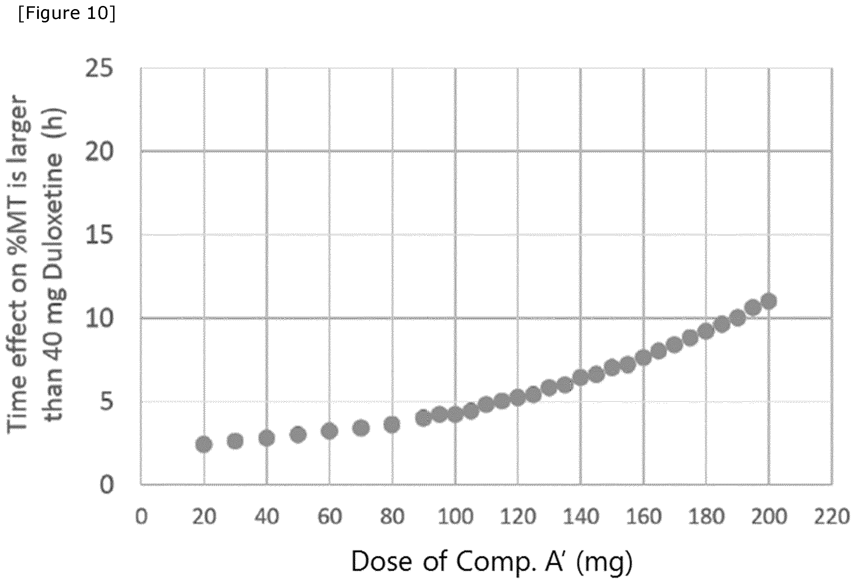

[Figure 10] Figure 10 shows results of a simulation of the effect on the %MT following the indicated dose of Compound A' (QD) administration. The effect on the %MT in this figure was calculated by subtracting the %MT after 40 mg Duloxetine administration from that after the indicated Compound A' administration.

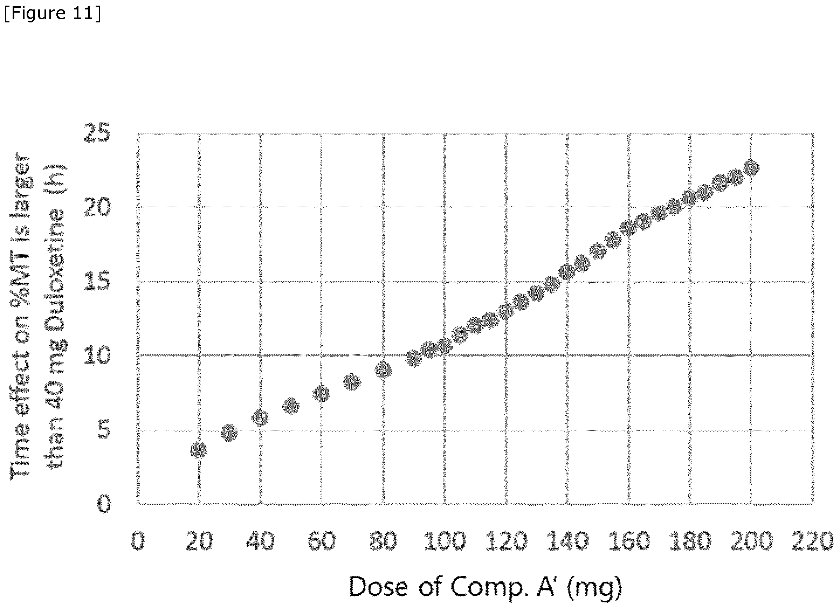

[Figure 11] Figure 11 shows results of a simulation of the effect on the %MT following the indicated dose of Compound A' (BID) administration. The effect on the %MT in this figure was calculated by subtracting the %MT after 40 mg Duloxetine administration from that after the indicated Compound A' administration.

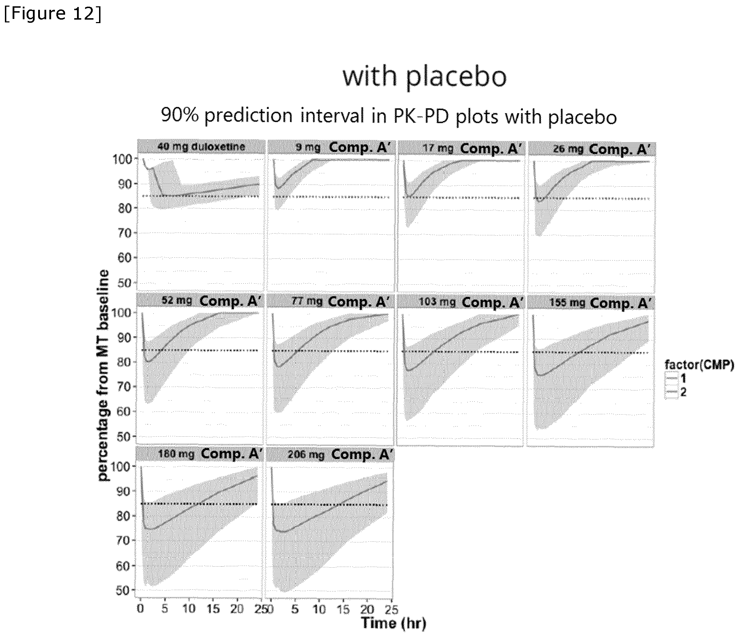

[Figure 12] Figure 12 shows the results of the simulation of effect-time profile for Compound A' administration in the US population with an additional placebo effect. Gray area shows 90% prediction interval for %MT from the baseline. Solid lines show predicted median %MT from the base line per dose group. Horizontal intermittent lines show 50th percentile Duloxetine effect at its Cmax.

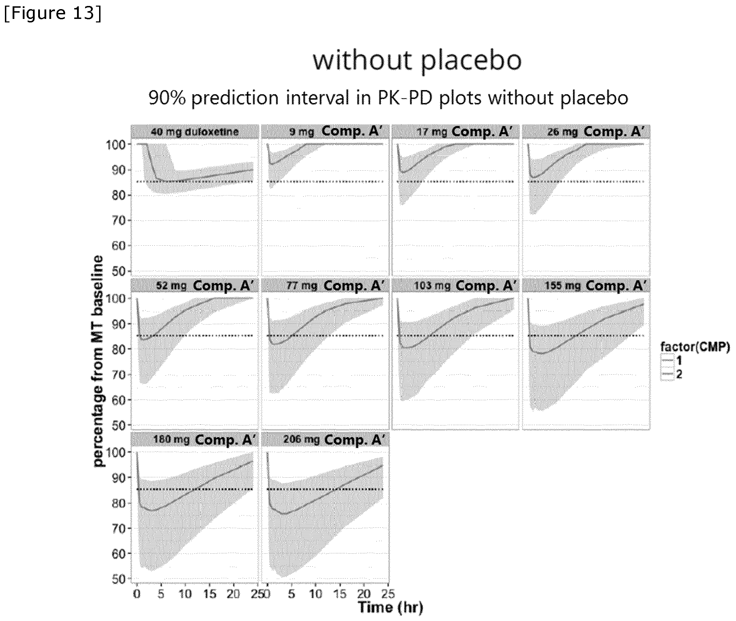

[Figure 13] Figure 13 shows the results of the simulation of effect-time profile for Compound A' administration in the US population without additional placebo effect. Gray area shows 90% prediction interval for %MT from the baseline. Solid lines show predicted median %MT from the base line per dose group. Horizontal intermittent lines show 50th percentile Duloxetine effect at its Cmax.

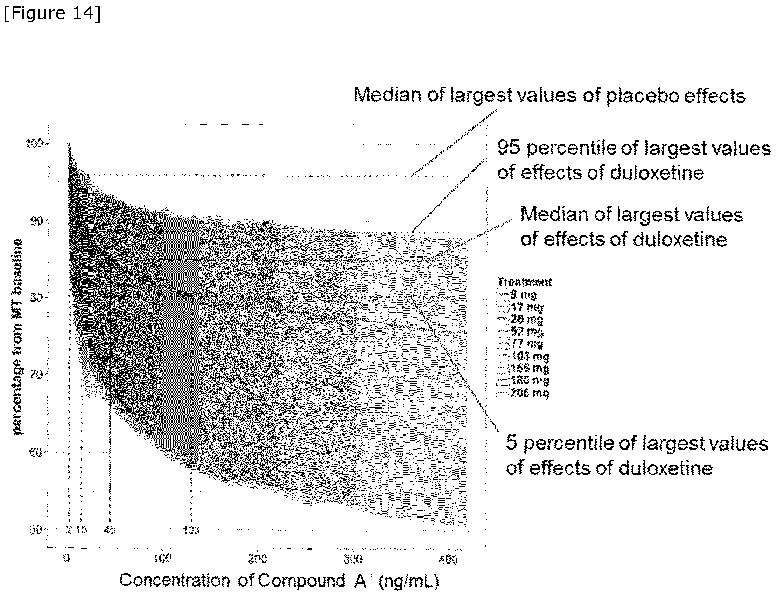

[Figure 14] Figure 14 shows a concentration-effect profile of Compound A'. The shaded area depicts the 90% prediction interval of % MT changes from the baseline. The solid line depicts a predicted median of % MT changes from the baseline in each dosage group. The horizontal blue dotted line depicts the median of the largest values of placebo effects. The horizontal grey dotted line or black solid line depicts the median of the largest effects of duloxetine. The dotted line depicts the 5 percentile and 95 percentile values thereof.

[Description of Embodiments]

In the present specification, the term "5-HT$_{2C}$ receptor" means 5-HT$_{2C}$, a subtype of 5-HT$_2$ in the receptor family of 5-hydroxytryptamine (or 5-HT).

In the present specification, the term "agonist" also includes partial agonists and selective agonists, unless otherwise specified.

In the present specification, the term "5-HT$_{2C}$ receptor agonist" means a substance activating the 5-HT$_{2C}$ receptor in order to initiate one or more intracellular activities or pathways known to those skilled in the art.

6

In the present specification, the term "subject" means a human subject.

In the present specification, the subject particularly may refer to a subject having stress urinary incontinence and more particularly a subject having stress urinary incontinence who has no obesity.

In the present specification, the subject particularly may also refer to a subject having fecal incontinence and more particularly a subject having fecal incontinence who has no obesity.

Subject having stress urinary incontinence is mainly found in females. Therefore, a subject having stress urinary incontinence may be a female.

In the present specification, the term "stress urinary incontinence" means that urine leaks involuntarily when the abdomen is placed under pressure. A subject with stress urinary incontinence experiences urine leakage due to pressure applied to the abdomen, for example, when the subject sneezes, coughs, exercises, stands up, and/or lifts a heavy weight. Those skilled in the art may also refer to stress urinary incontinence as "stress urinary incontinence", "stress incontinence" or "urinary stress incontinence" or "incontinence stress" or "urethral sphincter incontinence" or "urethral sphincter incompetence". The term "stress urinary incontinence" used herein is also referred to as "SUI". Further, those skilled in the art may also refer to incontinence of feces as "incontinence of feces", or "fecal incontinence".

In the present specification, the term "body mass index" or "BMI" refers to an index calculated from body weight (unit: kg) / (height m)$^2$ and is used for assessing obesity. According to the diagnostic criteria for obesity provided by the Japan Society for the Study of Obesity (see Diagnosis criteria for obesity and obesity disease, 2011), the obesity is classified as class I obesity to class IV obesity. The specific relationship between BMI values and obesity is as shown in the table below.

[Table 1]

Table 1: Relationship between BMI value and obesity

| BMI value | Determination of status |
| --- | --- |
| Lower than 18.5 | Underweight |
| 18.5 or higher to lower than 25 | Normal body weight |
| 25 or higher to lower than 30 | Obese (Class I) |
| 30 or higher to lower than 35 | Obese (Class II) |
| 35 or higher to lower than 40 | Obese (Class III) |
| 40 or higher | Obese (Class IV) |

Similarly, the World Health Organization (WHO) utilizes BMI in their definition of obesity for adults over 20 years of age, stating that BMI provides the most useful population-level measure of overweight and obesity as it is the same for both sexes and for all ages of adults. However, it should be considered a rough guide because it may not correspond to the same degree of fatness in different individuals. Overweight and obesity are defined as abnormal or excessive fat accumulation that may impair health.

The WHO definition is:

a BMI greater than or equal to 25 is overweight or Pre-obesity

a BMI greater than or equal to 30 is obesity.

The WHO defined relationship between BMI value and obesity is as follows:

[Table 1-1]

| BMI Value | Nutritional Status |
| --- | --- |
| Below 18.5 | Underweight |
| 18.5 or higher to lower than 25 | Normal weight |
| 25 or higher to lower than 30 | Pre-obesity |
| 30 or higher to lower than 35 | Obesity class I |

(continued)

| BMI Value | Nutritional Status |
| --- | --- |
| 35 or higher to lower than 40 | Obesity class II |
| Above 40 | Obesity class III |
| Source: Adapted from WHO, 1995, WHO, 2000 and WHO 2004 | |

In the present specification, the term "body weight lowering effect" means that the body weight of a subject is substantially lowered by a treatment to a therapeutically beneficial level as compared with the body weight of a subject without the treatment. In a specific embodiment, the term "body weight lowering effect" means that the weight loss is equal to or more than 3.8 % (in another embodiment, more than 2.6%) by calculating the ratio of the weight loss by treatment with placebo and a drug, respectively, and subtracting the weight loss ratio in the placebo treatment from that in the drug treatment. In the present specification, the term "anti-obesity effect" has the same meaning as the body weight lowering effect and is used interchangeably therewith.

In the present specification, the term "show no body weight lowering effect" is used to include the case where the body weight of a subject is not substantially lowered by a treatment to a therapeutically beneficial level as compared with the body weight of a subject without the treatment. In particular, the term "show no body weight lowering effect" means the weight loss below 3.8 % (in another embodiment, equal to or less than 2.6%) by calculating the ratio of the weight loss by treatment with placebo and a drug, respectively, and subtracting the weight loss ratio in the placebo treatment from that in the drug treatment.

In the present specification, the term "treatment" conceptually includes not only therapy but prevention.

In the present specification, the term "therapeutically effective amount" means an amount that brings about the therapy, curing, prevention, or amelioration of a disease or a disorder, or reduction in the rate of progression of a disease or a disorder in a subject who has received this amount as compared with a subject who has not received this amount.

In the present specification, the term "minimum dosage as an anti-obesity drug" means the lower limit of a dosage range producing a therapeutically effective anti-obesity effect (or body weight lowering effect), or the lower limit of a dosage range satisfying criteria for drug approval provided by a pharmaceutical authority. Those skilled in the art can appropriately determine the minimum dosage as an anti-obesity drug. The "amount that shows no body weight lowering effect" can be determined as a medication dosage that does not produce a therapeutically effective body weight lowering effect by administration of that dosage or does not produce a body weight lowering effect satisfying the criteria for drug approval provided by a pharmaceutical authority (for example, a dosage which shows a body weight decreasing effect by less than 3.8% (in another embodiment, equal to or less than 2.6%) compared to the average body weight in a placebo group). Such a dosage may be determined under an exercise regimen and/or a nutritional management necessary for keeping body weight constant without the administration of 5-HT$_{2C}$ receptor agonists for a subject.

In the present specification, the term "pharmaceutically acceptable salt" means an acid-addition salt or a base-addition salt that is accepted for administration to an organism.

In the present specification, the term "equal to or less than", "equal to or lower than", etc., includes the specified numerical value and a numerical value range that falls below the specified numerical value. Thus, in the present specification, the term " equal to or less than", " equal to or lower than", etc., is used to include the term "less than", "lower than", etc.

According to the present invention, stress urinary incontinence in a subject suffering from stress urinary incontinence can be treated by administrating to the subject a 5-HT$_{2C}$ receptor agonist at a dosage that shows no body weight lowering effect. In the present invention, stress urinary incontinence in a subject suffering from stress urinary incontinence can also be treated by administrating to the subject a 5-HT$_{2C}$ receptor agonist at a dosage lower than the minimum dosage of the agonist as an anti-obesity drug.

Thus, the present invention provides a medicament for use in treating stress urinary incontinence, comprising a 5-HT$_{2C}$ receptor agonist, wherein the medicament is administered at an amount that shows no body weight lowering effect. The present invention also provides a medicament for use in treating stress urinary incontinence, comprising a 5-HT$_{2C}$ receptor agonist, wherein the medicament is administered at a dosage lower than the minimum dosage of the 5-HT$_{2C}$ receptor agonist as an anti-obesity drug.

The present invention provides a medicament for use in treating stress urinary incontinence, comprising a therapeutically effective amount of a 5-HT$_{2C}$ receptor agonist, wherein the therapeutically effective amount of the agonist shows no body weight lowering effect. The present invention also provides a medicament for use in treating stress urinary incontinence, comprising a therapeutically effective amount of a 5-HT$_{2C}$ receptor agonist, wherein the therapeutically effective amount of the agonist is lower than the therapeutically effective amount of the agonist as an anti-obesity drug. The present invention further provides a medicament for use in treating stress urinary incontinence, comprising a therapeutically effective amount of a 5-HT$_{2C}$ receptor agonist, wherein the medicament is administered to a subject so as to show no body weight lowering effect. Therefore, the present invention allows the 5-HT$_{2C}$ receptor agonist to be administered even to subjects having stress urinary incontinence without obesity. The administration of the 5-HT$_{2C}$ receptor agonist at a dosage that shows the body weight lowering effect causes adverse effects, such as nausea and headache, at high frequency. By contrast, the present invention permits the administration of the agonist at a lower dosage, which can reduce the frequency of adverse effects and thus may improve QOL of subjects during the treatment of their stress urinary incontinence.

According to the present invention, incontinence of feces in a subject suffering from incontinence of feces can be treated by administrating to the subject a 5-HT$_{2C}$ receptor agonist {for example, the 5-HT$_{2C}$ receptor agonist may be (R)-8-chloro-1-methyl-2,3,4,5-tetrahydro-1H-3-benzazepine or a salt thereof. According to the present invention, incontinence of feces in a subject suffering from incontinence of feces can be treated by administrating to the subject a 5-HT$_{2C}$ receptor agonist at a dosage where adverse effects such as nausea and headache will be reduced. According to the present invention, incontinence of feces in a subject suffering from incontinence of feces can be treated by administrating to the subject a 5-HT$_{2C}$ receptor agonist at a dosage that shows no body weight lowering effect. In the present invention, incontinence of feces in a subject suffering from incontinence of feces can also be treated by administrating to the subject a 5-HT$_{2C}$ receptor agonist at a dosage lower than the minimum dosage of the agonist as an anti-obesity drug.

Thus, the present invention provides a medicament for use in treating incontinence of feces, comprising a 5-HT$_{2C}$ receptor agonist, wherein the medicament is administered at an amount that shows no body weight lowering effect. The present invention also provides a medicament for use in treating incontinence of feces, comprising a 5-HT$_{2C}$ receptor agonist, wherein the medicament is administered at a dosage lower than the minimum dosage of the 5-HT$_{2C}$ receptor agonist as an anti-obesity drug.

The present invention provides a medicament for use in treating incontinence of feces, comprising a therapeutically effective amount of a 5-HT$_{2C}$ receptor agonist, wherein the therapeutically effective amount of the agonist shows no body weight lowering effect. The present invention also provides a medicament for use in treating incontinence of feces, comprising a therapeutically effective amount of a 5-HT$_{2C}$ receptor agonist, wherein the therapeutically effective amount of the agonist is lower than the therapeutically effective amount of the agonist as an anti-obesity drug. The present invention further provides a medicament for use in treating incontinence of feces, comprising a therapeutically effective amount of a 5-HT$_{2C}$ receptor agonist, wherein the medicament is administered to a subject so as to show no body weight lowering effect. Therefore, the present invention allows the 5-HT$_{2C}$ receptor agonist to be administered even to subjects of incontinence of feces without obesity. The administration of the 5-HT$_{2C}$ receptor agonist at a dosage that shows the body weight lowering effect causes adverse effects, such as nausea and headache, at high frequency. By contrast, the present invention permits the administration of the agonist at a lower dosage, which can reduce the frequency of adverse effects and thus may improve QOL of subjects during the treatment of their incontinence of feces.

In the present invention, the subject may have any body mass index (BMI) and can be a subject having BMI < 18.5 (i.e., a subject having "underweight"), a subject having BMI of 18.5 or higher and lower than 25 (i.e., a subject having "normal body weight"), or a subject having BMI of 25 or higher (class I to IV "obese" subject).

The present invention provides a medicament for use in treating stress urinary incontinence, comprising a therapeutically effective amount of a 5-HT$_{2C}$ receptor agonist, wherein at least one adverse effect selected from nausea and headache is reduced. The present invention also provides a medicament for use in treating stress urinary incontinence, comprising a therapeutically effective amount of a 5-HT$_{2C}$ receptor agonist, wherein the medicament is administered at a dosage at which at least one adverse effect selected from nausea and headache is reduced.

The present invention provides a medicament for use in treating incontinence of feces, comprising a therapeutically effective amount of a 5-HT$_{2C}$ receptor agonist, wherein at least one adverse effect selected from nausea and headache is reduced. The present invention also provides a medicament for use in treating incontinence of feces, comprising a therapeutically effective amount of a 5-HT$_{2C}$ receptor agonist, wherein the medicament is administered at a dosage at which at least one adverse effect selected from nausea and headache is reduced.

In the present invention, as a 5-HT$_{2C}$ receptor agonist, for example, a 5-HT$_{2C}$ receptor agonist having an *in vitro* agonist activity of approximately 1000 nM or lower, preferably approximately 100 nM or lower, in terms of concentration at which a test agonist exhibits 50% effect *in vitro* (EC$_{50}$) may be used. Examples of the 5-HT$_{2C}$ receptor agonist include compounds described in EP0572863, EP0863136, EP1213017, USP3253989, USP3676558, USP3652588, USP4082844, USP4971969, USP5494928, USP5646173, USP6310208, WO97/42183, WO98/30546, WO98/30548, WO98/33504, WO99/02159, WO99/43647 (USP6281243), WO00/12475 (USP6380238), WO00/12502 (USP6365598), WO00/12510 (USP6433175), WO00/12475, WO00/12481 (USP6552062), WO00/12482, WO00/12502, WO00/16761, WO00/17170, WO00/28993, WO00/35922 (USP6372745), WO00/44737, WO00/44753, WO00/64899, WO00/77001, WO00/77002, WO00/77010, WO00/76984 (USP6465467), WO01/09111, WO01/09122, WO01/09123 (USP6638936), WO01/09126, WO01/12602, WO01/12603 (USP6706750), WO01/40183, WO01/66548 (USP6583134), WO01/70207, WO01/70223, WO01/72752 (USP6734301), WO01/83487, WO02/04456, WO02/04457, WO02/08178, WO02/10169, WO02/24700, WO02/24701, WO02/36596, WO02/40456, WO02/40457, WO02/42304, WO02/44152 (USP6479534), WO02/48124, WO02/51844 (USP6610685), WO02/59124, WO02/59127, WO02/59129, WO02/72584, WO02/74746, WO02/83863, WO02/98350, WO02/98400, WO02/98860, WO03/00663, WO03/00666, WO03/04501, WO03/06466, WO03/11281, WO03/14118, WO03/14125, WO03/24976, WO03/28733, WO03/33497, WO03/57161, WO03/57213, WO03/57673, WO03/57674, WO03/62205, WO03/64423, WO03/86306, WO03/87086, WO03/89409, WO03/91250, WO03/91251, WO03/91257, WO03/97636, WO04/00829, WO04/00830 (USP6667303), WO04/56324, WO04/78718, WO04/81010, WO04/087156, WO04/87662, WO04/87692, WO04/89897, WO04/096196, WO04/96201, WO04/112769, US2004/192754, WO05/00849, WO05/03096, EP1500391, WO05/16902, WO05/19180, US2005/080074, WO05/40146, WO05/41856, WO05/42490, WO05/42491, WO05/44812, WO05/082859, WO05/000309, WO05/019179, WO05/121113, WO05/049623, WO05/105082, WO05/109987, WO05/121113, WO05/113535, US2006/003990, US2006/014777, US2006/014778, WO06/000902, WO06/028961, WO06/020817, WO06/020049, WO06/019940, WO06/004931, US2006/025601, WO2006/022420WO06/044762, WO06/047032, WO06/050007, WO06/052887, WO06/077025, WO06/065600, WO06/103511, WO06/116165, WO06/047228, WO06/117304, US2006/241172, US2006/241176, WO06/116136, WO06/116148, WO06/116151, WO06/116171, WO06/116170, WO06/116218, WO06/116169, WO06/077025, US2007/032481, WO07/025144, WO07/028082, WO07/028132, WO07/028131, WO07/028083, WO07/030150, US2007/0049613, WO2007/132841, WO2011/111817.

Any of these compounds may preferably be used in the present invention. Examples of the 5-HT$_{2C}$ receptor agonist also include Compound A and its salts (for example, the hydrochloride salt) and Compound B and its salts (for example, the hydrochloride salt). Any of these compounds or salts, diastereomer, or enantiomer thereof may be used in the present invention.

The 5-HT$_{2C}$ receptor agonists include, for example, a compound selected from a group consisting of:

6,8-dichloro-1-methyl-2,3,4,5-tetrahydro-1H-3-benzazepine;
6-chloro-1-methyl-2,3,4,5-tetrahydro-1H-3-benzazepine;
8-chloro-9-fluoro-1-methyl-2,3,4,5-tetrahydro-1H-3-benzazepine;
8,9-dichloro-1-methyl-2,3,4,5-tetrahydro-1H-3-benzazepine; and
9-bromo-8-chloro-1-methyl-2,3,4,5-tetrahydro-1H-3-benzazepine,
diastereomer, or enantiomer thereof, or pharmaceutically acceptable salt, hydrate, or solvate thereof. These compounds are disclosed in US8404675.

The 5-HT$_{2C}$ receptor agonists include, for example, a compound selected from a group consisting of:

N-methyl-6,8-dichloro-1-methyl-2,3,4,5-tetrahydro-1H-3-benzazepine;
N-methyl-6-chloro-1-methyl-2,3,4,5-tetrahydro-1H-3-benzazepine;
N-methyl-8-chloro-9-fluoro-1-methyl-2,3,4,5-tetrahydro-1H-3-benzazepine;
N-methyl-8,9-dichloro-1-methyl-2,3,4,5-tetrahydro-1H-3-benzazepine; and
N-methyl-9-bromo-8-chloro-1-methyl-2,3,4,5-tetrahydro-1H-3-benzazepine, diastereomer, or enantiomer thereof, or pharmaceutically acceptable salt, hydrate, or solvate thereof. These compounds are disclosed in US8404675.

The 5-HT$_{2C}$ receptor agonists include, for example, a compound selected from a group consisting of:

8-bromo-7-hydroxy-1-methyl-2,3,4,5-tetrahydro-1H-3-benzazepine;
7-allyloxy-8-bromo-1-methyl-2,3,4,5-tetrahydro-1H-3-benzazepine;
7-benxyloxy-8-bromo-1-methyl-2,3,4,5-tetrahydro-1H-3-benzazepine;
8-bromo-7-ethoxy-1-methyl-2,3,4,5-tetrahydro-1H-3-benzazepine;
8-bromo-7 -isopropoxy-1-methyl-2,3,4,5-tetrahydro-1H-3-benzazepine;
N-propyl-8-bromo-7-methoxy-1-methyl-2,3,4,5-tetrahydro-1H-3-benzazepine;
7-hydroxy-8-iodo-1-methyl-2,3,4,5-tetrahydro-1H-3-benzazepine;
7-allyloxy-8-iodo-1-methyl-2,3,4,5-tetrahydro-1H-3-benzazepine;
3,5-dimethyl-6,7,8,9-tetrahydro-5H-1-oxa-7-azacycloheptaindene;
7-allyloxy-8-chloro-1-methyl-2,3,4,5-tetrahydro-1H-3-benzazepine;
7-methoxy-1-methyl-8-(2-thienyl)-2,3,4,5-tetrahydro-1H-3-benzazepine;
8-cyano-7-methoxy-1-methyl-2,3,4,5-tetrahydro-1H-3-benzazepine;
8-bromo-1-cyclopropyl-7-methoxy-2,3,4,5-tetrahydro-1H-3-benzazepine;
8-bromo-1-hydroxy-methyl-7-methoxy-2,3,4,5-tetrahydro-1H-3-benzazepine;
8-bromo-1-isopropyl-7-methoxy-2,3,4,5-tetrahydro-1H-3-benzazepine;
8-bromo-7-hydroxy-1-isopropyl-2,3,4,5-tetrahydro-1H-3-benzazepine;
7-allyloxy-8-bromo-1-isopropyl-2,3,4,5-tetrahydro-1H-3-benzazepine;
8-bromo-7-methoxy-1,4-dimethyl-2,3,4,5-tetrahydro-1H-3-benzazepine;

7-allyloxy-8-bromo-1,4-dimethyl-2,3,4,5-tetrahydro-1H-3-benzazepine;
7-(2-methyl-2H-pyrazol-3-yl)-1-methyl-2,3,4,5-tetrahydro-1H-3-benzazepine;
7-(4-bromo-2-methyl-2H-pyrazol-3-yl)-1-methyl-2,3,4,5-tetrahydro-1H-3-benzazepine;
7-(3-chlorophenyl)-1-methyl-2,3,4,5-tetrahydro-1H-3-benzazepine;
7-(2-chlorophenyl)-1-methyl-2,3,4,5-tetrahydro-1H-3-benzazepine;
8-chloro-1-hydroxy-2,3,4,5-tetrahydro-1H-3-benzazepine;
8-bromo-1-methyl-2,3,4,5-tetrahydro-1H-3-benzazepine;
8-fluoro-1-methyl-2,3,4,5-tetrahydro-1H-3-benzazepine;
7-fluoro-1-methyl-2,3,4,5-tetrahydro-1H-3-benzazepine;
7,8-dichloro-1-methyl-2,3,4,5-tetrahydro-1H-3-benzazepine;
N-methyl-8-chloro-1-methyl-2,3,4,5-tetrahydro-1H-3-benzazepine;
1-methyl-7-trifluoromethoxy-2,3,4,5-tetrahydro-1H-3-benzazepine;
8-iodo-1-methyl-7-trifluoromethoxy-2,3,4,5-tetrahydro-1H-3-benzazepine;
N-propyl-8-iodo-7-methoxy-1-methyl-2,3,4,5-tetrahydro-1H-3-benzazepine;
1-ethyl-8-iodo-7-methoxy-2,3,4,5-tetrahydro-1H-3-benzazepine;
7-(3-methoxyphenyl)-1-methyl-2,3,4,5-tetrahydro-1H-3-benzazepine;
7-(2,6-difluoro-phenyl)-1-methyl-2,3,4,5-tetrahydro-1H-3-benzazepine;
7-(2-fluoro-phenyl)-8-chloro-1-methyl-2,3,4,5-tetrahydro-1H-3-benzazepine;
7-(2-trifluoromethylphenyl)-1-methyl-2,3,4,5-tetrahydro-1H-3-benzazepine;
7-(3-trifluoromethylphenyl)-1-methyl-2,3,4, 5-tetrahydro-1H-3-benzazepine;
7-(4-trifluoromethylphenyl)-1-methyl-2,3,4,5-tetrahydro-1H-3-benzazepine;
8-(2-chlorophenyl)-1-methyl-2,3,4,5-tetrahydro-1H-3-benzazepine; and
8-bromo-1-methoxymethyl-7-methoxy-2,3,4,5-tetrahydro-1H-3-benzazepine, diastereomer, or enantiomer thereof,
or pharmaceutically acceptable salt, hydrate, or solvate thereof. These compounds are disclosed in US8846906.

The 5-HT$_{2C}$ receptor agonists include, for example, a compound selected from a group consisting of:

8-bromo-7 -methoxy-1-methyl-2,3,4,5-tetrahydro-1H-3-benzazepine;
8-chloro-7-methoxy-1-methyl-2,3,4,5-tetrahydro-1H-3-benzazepine;
8-iodo-7-methoxy-1-methyl-2,3,4,5-tetrahydro-1H-3-benzazepine;
N-methyl-8-bromo-7-methoxy-1-methyl-2,3,4,5-tetrahydro-1H-3-benzazepine;
8-bromo-1-ethyl-7-methoxy-2,3,4,5-tetrahydro-1H-3-benzazepine;
8-chloro-1-ethyl-7-methoxy-2,3,4,5-tetrahydro-1H-3-benzazepine;
8-iodo-1-ethyl-7-methoxy-2,3,4,5-tetrahydro-1H-3-benzazepine;
7-methoxy-1-methyl-8-trifluoromethyl-2,3,4,5-tetrahydro-1H-3-benzazepine; and
7-methoxy-1-methyl-8-pentafluoroethyl-2,3,4,5-tetrahydro-1H-3-benzazepine, diastereomer, or enantiomer thereof,
or pharmaceutically acceptable salt, hydrate, or solvate thereof. These compounds are disclosed in US8846906.

The 5-HT$_{2C}$ receptor agonists include, for example, 8-chloro-1-methyl-2,3,4,5-tetrahydro-1H-3-benzazepine, or diastereomer or enantiomer thereof, or pharmaceutically acceptable salt, solvate, or hydrate thereof. These compounds are disclosed in US8846906.

The 5-HT$_{2C}$ receptor agonists include, for example, a compound selected from a group consisting of:

8-iodo-1-methyl-2,3,4,5-tetrahydro-1H-3-benzazepine;
8-trifluoromethyl-1-methyl-2,3,4,5-tetrahydro-1H-3-benzazepine;
8-trifluoromethyl-1-ethyl-2,3,4,5-tetrahydro-1H-3-benzazepine;
8-chloro-1-ethyl-2,3,4,5-tetrahydro-1H-3-benzazepine;
8-bromo-1-ethyl-2,3,4,5-tetrahydro-1H-3-benzazepine;
8-iodo-1-ethyl-2,3,4,5-tetrahydro-1H-3-benzazepine;
7,8-dichloro-1-methyl-2,3,4,5-tetrahydro-1H-3-benzazepine;
7,8-dichloro-1-ethyl-2,3,4,5-tetrahydro-1H-3-benzazepine;
8-chloro-7-fluoro-1-methyl-2,3,4,5-tetrahydro-1H-3-benzazepine;
8-chloro-7-fluoro-1-ethyl-2,3,4,5-tetrahydro-1H-3-benzazepine,

or diastereomer or enantiomer thereof, or pharmaceutically acceptable salt, solvate, or hydrate thereof. These compounds are disclosed in US8846906.

The 5-HT$_{2C}$ receptor agonists include, for example, a compound selected from a group consisting of:

1-(2,3-difluoro-phenyl)-2 -ethyl-piperazine;
1-(3-fluoro-phenyl)-2-ethyl-piperazine;
1-(4-fluoro-phenyl)-2-ethyl-piperazine;
(R)-1-(3-chloro-4-fluoro-phenyl)-2-methyl-piperazine;
(S)-1-(3-chloro-4-fluoro-phenyl)-2-methyl-piperazine;
(R)-1-(3,4-difluoro-phenyl)-2-methyl-piperazine;
(S)-1-(3,4-difluoro-phenyl)-2-methyl-piperazine;
(R)-1-(3-chloro-2-fluoro-phenyl)-2-methyl-piperazine;
(S)-1-(3-chloro-2-fluoro-phenyl)-2-methyl- piperazine;
(R)-1-(4-fluoro-phenyl)-2-methyl-piperazine;
(S)-1-(4-fluoro-phenyl)-2-methyl-piperazine;
(R)-1-(3,4-dichloro-phenyl)-2-methyl-piperazine;
(S)-1-(3,4-dichloro-phenyl)-2-methyl-piperazine;
(R)-1-(3-chloro-4-methyl-phenyl)-2- methyl-piperazine;
(S)-1-(3-chloro-4-methyl-phenyl)-2-methyl-piperazine;
(R)-1-(3,4-difluoro-phenyl)-2-methyl-piperazine;
(S)-1-(3,4-difluoro-phenyl)-2-methyl-piperazine;
(R)-1-(3,5-dichloro-phenyl)-2-methyl-piperazine;
(S)-1-(3,5-dichloro-phenyl)-2-methyl-piperazine;
(R)-1-(2,5-difluoro-phenyl)-2-methyl-piperazine;
(S)-1-(2, 5-difluoro-phenyl)-2-methyl-piperazine;
(R)-1-(4-chloro-3-fluoro-phenyl)-2-methyl-piperazine;
(S)-1-(4-chloro-3 -fluoro-phenyl)-2-methyl-piperazine;
(R)-1-(3-chloro-2-methyl-phenyl)-2-methyl-piperazine;
(S)-1-(3-chloro-2-methyl-phenyl)-2-methyl-piperazine;
(R)-1-(5-chloro-2-fluoro-phenyl)-2-methyl-piperazine;
(S)-1-(5-chloro-2-fluoro-phenyl)-2-methyl-piperazine;
(R)-1-(5-chloro-2-methyl-phenyl)-2-methyl-piperazine;
(S)-1-(5-chloro-2-methyl-phenyl)-2-methyl-piperazine;
1-(3-chloro-4-fluoro-phenyl)-2-ethyl-piperazine;
1-(3-chloro-phenyl)-2-ethyl-piperazine;
1-(4-chloro-phenyl)-2-ethyl-piperazine;
1-(3,4-difluoro-phenyl)-2-ethyl-piperazine; and
(R)-1-(5-chloro-2-fluoro-phenyl)-2-ethyl-piperazine,
or diastereomer or enantiomer thereof, or pharmaceutically acceptable salt, solvate,
or hydrate thereof. These compounds are disclosed in US20070179155.

The 5-HT$_{2C}$ receptor agonists include, for example, a compound selected from a group consisting of:

(R)-1-(2-fluoro-5-trifluoromethyl-phenyl)-2-methyl-piperazine;
(S)-1-(2-fluoro-5-trifluoromethyl-phenyl)-2-methyl-piperazine;
(R)-1 -(4-chloro-2-fluoro-phenyl)-2-methyl-piperazine;
(S)-1-(4-chloro-2-fluoro-phenyl)-2-methyl-piperazine;
(R)-1-(3-chloro-5-fluoro-phenyl)-2-methyl-piperazine;
(S)-1-(3-chloro-5-fluoro-phenyl)-2-methyl-piperazine;
(R)-1-(3-fluoro-phenyl)-2-methyl-piperazine;
(S)-1-(3-fluoro-phenyl)-2-methyl-piperazine;
(R)-1-(2-fluoro-4-trifluoromethyl-phenyl)-2-methyl-piperazine;
(S)-1-(2-fluoro-4-trifluoromethyl-phenyl)-2-methyl-piperazine;
(R)-1-(2-chloro-3-fluoro-phenyl)-2-methyl-piperazine;
(S)- 1-(2-chloro-3 -fluoro-phenyl)-2-methyl-piperazine;
(R)-1-(2-fluoro-5-methyl-phenyl)-2-methyl-piperazine;
(S)-1-(2-fluoro-5-methyl-phenyl)-2-methyl-piperazine;
(R)-1-(4-fluoro-biphenyl-3 -yl)-2-methyl-piperazine;
(S)-1-(4-fluoro-biphenyl-3-yl)-2-methyl-piperazine;
(R)-1-(2,5-difluoro-4-methoxy-phenyl)-2-methyl-piperazine;
(S)-1-(2,5-difluoro-4-methoxy-phenyl)-2-methyl-piperazine;
(R)-1-(2-fluoro-4-methyl-phenyl)-2-methyl-piperazine;

(S)-1-(2-fluoro-4-methyl-phenyl)-2-methyl-piperazine;
(R)-1-(2-chloro-5-fluoro-phenyl)-2-methyl-piperazine;
(S)-1-(2-chloro-5-fluoro-phenyl)-2-methyl-piperazine;
(R)-1-(2-chloro-4-fluoro-phenyl)-2-methyl-piperazine;
(S)-l-(2-chloro-4-fluoro-phenyl)-2-methyl-piperazine;
(R)-1 -(2,4-dichloro-phenyl)-2-methyl-piperazine;
(S)-1-(2,4-dichloro-phenyl)-2-methyl-piperazine;
(R)-1-(2,5-dichloro-phenyl)-2-methyl-piperazine;
(S)-1-(2,5-dichloro-phenyl)-2-methyl-piperazine;
(R)-1-(3,5-ビス-trifluoromethyl-phenyl)-2-methyl-piperazine;
(S)-1-(3,5-ビス-trifluoromethyl-phenyl)-2-methyl-piperazine;
(R)-1-(4-fluoro-2-methyl-phenyl)-2-methyl-piperazine;
(S)-1-(4-fluoro-2-methyl-phenyl)-2-methyl-piperazine;
(R)-1-(2-chloro-phenyl)-2-methyl-piperazine;
(S)-1-(2-chloro-phenyl)-2-methyl-piperazine;
(R)-1-(2,3-dichloro-phenyl)-2-methyl-piperazine;
(R)-1-(2,3-dichloro-phenyl)-2-methyl-piperazine;
(R)-1 -(2,6-dichloro-phenyl)-2-methyl-piperazine;
(R)-1 -(2,6-dichloro-phenyl)-2-methyl-piperazine;
(R)-1-(2-chloro-5-trifluoromethyl-phenyl)-2-methyl-piperazine;
(R)-1-(2-chloro-5-trifluoromethyl-phenyl)-2-methyl-piperazine;
(R)-2-methyl-1-(4-trifluoromethyl-phenyl)-piperazine;
(S)-2-methyl-1-(4-trifluoromethyl-phenyl)-piperazine;
(R)-1-(2-fluoro-3 -trifluoromethyl-phenyl)-2-methyl-piperazine;
(S)-1-(2-fluoro-3-trifluoromethyl-phenyl)-2-methyl-piperazine;
(R)-1-(3-fluoro-5-trifluoromethyl-phenyl)-2-methyl-piperazine;
(R)-1-(3-fluoro-5-trifluoromethyl-phenyl)-2-methyl-piperazine;
(R)-1-(4-chloro-3-trifluoromethyl-phenyl)-2-methyl-piperazine;
(S)-1-(4-chloro-3-trifluoromethyl-phenyl)-2-methyl-piperazine;
(R)-2,4-dimethyl-1-(3-trifluoromethyl-phenyl)-piperazine
or diastereomer or enantiomer thereof, or pharmaceutically acceptable salt, solvate,
or hydrate thereof. These compounds are disclosed in US20070179155.

The 5-HT$_{2C}$ receptor agonists include, for example, a compound selected from a group consisting of:

1-(2-bromo-phenyl)-2-vinyl-piperazine;
1-(4-chloro-phenyl)-2-vinyl-piperazine;
1-(3-fluoro-phenyl)-2-vinyl-piperazine;
1-(3-chloro-4-fluoro-phenyl)-2-vinyl-piperazine;
1-(3-chloro-phenyl)-2-vinyl-piperazine;
1-(3-bromo-phenyl)-2-vinyl-piperazine;
1-(3,5-dichloro-phenyl)-2-vinyl-piperazine;
1-(2-bromo-4-isopropyl-phenyl)-2-vinyl-piperazine;
1-(2-bromo-4-trifluoromethoxy-phenyl)-2-vinyl-piperazine;
1-(2-bromo-4-trifluoromethyl-phenyl)-2-vinyl-piperazine;
3-(2-vinyl-piperazine-1-yl)-benzonitrile;
1-(3,5-difluoro-phenyl)-2-vinyl-piperazine;
1-o-tolyl-2-vinyl-piperazine;
1-(2,3-difluoro-phenyl)-2-vinyl-piperazine; and
(R)-1-(4-chloro-phenyl)-2-methyl-piperazine;
or diastereomer or enantiomer thereof, or pharmaceutically acceptable salt, solvate,
or hydrate thereof. These compounds are disclosed in US20070179155.

The 5-HT$_{2C}$ receptor agonists include, for example, a compound selected from a group consisting of:

7-benzyloxy-1-methyl-2,3,4,5-tetrahydro-1H-benzo[d]azepine;
1-methyl-7-(1-phenyl-ethoxy)-2,3,4,5-tetrahydro-1H-benzo[d]azepine;

1-methyl-7-phenethyloxy-2,3,4,5-tetrahydro-1H-benzo[d]azepine;
1-methyl-7-(3-phenyl-propoxy)-2,3,4,5-tetrahydro-1H-benzo[d]azepine;
benzyl-(5-methyl-2,3,4,5-tetrahydro-1H-benzo[d]azepin-7-yl)-amine;
(5-methyl-2,3,4,5-tetrahydro-1H-benzo[d]azepin-7-yl)-(1'-phenyl-ethyl)-amine;
benzyl-methyl-(5-methyl-2,3,4,5-tetrahydro-1H-benzo[d]azepin-7-yl)-amine;
(5-methyl-2,3,4,5-tetrahydro-1H-benzo[d]azepin-7-yl)-phenethyl-amine;
(5-methyl-2,3,4,5-tetrahydro-1H-benzo[d]azepin-7-yl)-(3-phenyl-propyl)-amine;
(5-methyl-2,3,4,5-tetrahydro-1H-benzo[d]azepin-7-yl)-phenyl-amine;
1-methyl-8-phenyl-2,3,4,5-tetrahydro-1H-benzo[d]azepine;
or diastereomer or enantiomer thereof, or pharmaceutically acceptable salt, solvate,
or hydrate thereof. These compounds are disclosed in US20070275949.

The 5-HT$_{2C}$ receptor agonists include, for example, a compound selected from a group consisting of:

8-Benzyloxy-1-methyl-2,3,4,5-tetrahydro-1H-benzo[d]azepine;
7-Benzyloxy-1-methyl-2,3,4,5-tetrahydro-1H-benzo[d]azepine;
1-Methyl-8-phenyl-2,3,4,5-tetrahydro-1H-benzo[d]azepine;
7-Methoxy-1-methyl-8-phenyl-2,3,4,5-tetrahydro-1H-benzo[d]azepine;
8-(2-Fluoro-phenyl)-1-methyl-2,3,4,5-tetrahydro-1H-benzo[d]azepine;
8-(3-Fluoro-phenyl)-1-methyl-2,3,4,5-tetrahydro-1H-benzo[d]azepine;
8-(4-Fluoro-phenyl)-1-methyl-2,3,4,5-tetrahydro-1H-benzo[d]azepine;
8-(2,6-Difluoro-phenyl)-1-methyl-2,3,4,5-tetrahydro-1H-benzo[d]azepine;
8-(2,3-Difluoro-phenyl)-1-methyl-2,3,4,5-tetrahydro-1H-benzo[d]azepine;
8-(2,5-Difluoro-phenyl)-1-methyl-2,3,4,5-tetrahydro-1H-benzo[d]azepine;
1-Methyl-8-pyridin-3-yl-2,3,4,5-tetrahydro-1H-benzo[d]azepine;
1-Methyl-8-pyridin-2-yl-2,3,4,5-tetrahydro-1H-benzo[d]azepine;
or diastereomer or enantiomer thereof, or pharmaceutically acceptable salt, solvate,
or hydrate thereof. These compounds are disclosed in US20070275949.

The 5-HT$_{2C}$ receptor agonists include, for example, a compound selected from a group consisting of:

1-methyl-8-(2-phenoxy-tethoxy)-2,3,4,5-tetrahydro-1H-benzo[d]azepine;
(4-fluoro-benzyl)-(5-methyl-2,3,4,5-tetrahydro-1H-benzo[d]azepin-7-yl)-amine;
biphenyl-4-ylmethyl-(5-methyl-2,3,4,5-tetrahydro-1H-benzo[d]azepin-7-yl)-amine;
5-methyl-2,3,4,5-tetrahydro-1H-benzo[d]azepine-7-carboxylic acid phenylamide;
5-methyl-2,3,4,5-tetrahydro-1H-benzo[d]azepine-7-carboxylic acid benzylamide;
5-methyl-2,3,4,5-tetrahydro-1H-benzo[d]azepine-7-carboxylic acid phenethylamide;
5-methyl-2,3,4,5-tetrahydro-1H-benzo[d]azepine-7-carboxylic acid phenpropylamide;
5-methyl-2,3,4,5-tetrahydro-1H-benzo[d]azepine-7-carboxylic acid 4-phenylbenzylamide;
[2-(3,4-dimethoxy-phenyl)-ethyl]-(5-methyl-2,3,4,5-tetrahydro-1H-benzo[d]-azepin-7-yl)-amine;
8-benzyl-1-methyl-2,3,4,5-tetrahydro-1H-benzo[d]azepine;
indan-1'-yl-(5-methyl-2,3,4,5-tetrahydro-1H-benzo[d]azepin-7-yl)-amine;
7-benzyl-8-chloro-1-methyl-2,3,4, 5-tetrahydro-1H-benzo[d]azepine;
8-benzyl-7-methoxy-1-methyl-2,3,4,5-tetrahydro-1H-benzo[d]azepine; and
6-Benzyl-1-methyl-2,3,4,5-tetrahydro-1H-benzo[d]azepin-7-ol;
or diastereomer or enantiomer thereof, or pharmaceutically acceptable salt, solvate,
or hydrate thereof. These compounds are disclosed in US20080009478.

The 5-HT$_{2C}$ receptor agonists include, for example, a compound selected from a group consisting of:

8-(3-Methoxy-benzyl)-1-methyl-2,3,4,5-tetrahydro-1H-benzo[d]azepine;
8-Benzyl-1-methyl-2,3,4,5-tetrahydro-1H-benzo[d]azepine;
8-Benzyl-7-methoxy-1-methyl-2,3,4,5-tetrahydro-1H-benzo[d]azepine;
8-Benzyl-1-methyl-2,3,4,5-tetrahydro-1H-benzo[d]azepin-7-ol;
1-Methyl-8-phenethyl-2,3,4,5-tetrahydro-1H-benzo[d]azepine;
8-(2-Fluoro-benzyl)-1-methyl-2,3,4,5-tetrahydro-1H-benzo[d]azepine;
8-(3-Fluoro-benzyl)-1-methyl-2,3,4,5-tetrahydro-1H-benzo[d]azepine;
8-(4-Fluoro-benzyl)-1-methyl-2,3,4,5-tetrahydro-1H-benzo[d]azepine;

1-Methyl-8-(3-trifluoromethyl-benzyl)-2,3,4,5-tetrahydro-1H-benzo[d]azepine;
8-(2,6-Difluoro-benzyl)-1-methyl-2,3,4,5-tetrahydro-1H-benzo[d]azepine;
8-(2,4-difluoro-benzyl)-1-methyl-2,3,4,5-tetrahydro-1H-benzo[d]azepine;
8-(2,5-Difluoro-benzyl)-1-methyl-2,3,4,5-tetrahydro-1H-benzo[d]azepine;
8-(3,5-difluoro-benzyl)-1-methyl-2,3,4,5-tetrahydro-1H-benzo[d]azepine;
8-(3,4-Difluoro-benzyl)-1-methyl-2,3,4,5-tetrahydro-1H-benzo[d]azepine;
8-(2-Methoxy-benzyl)-1-methyl-2,3,4,5-tetrahydro-1H-benzo[d]azepine;
8-(4-Methoxy-benzyl)-1-methyl-2,3,4,5-tetrahydro-1H-benzo[d]azepine;
1-Methyl-8-(1-phenyl-ethyl)-2,3,4,5-tetrahydro-1H-benzo[d]azepine;
(8-Methoxy-5-methyl-2,3,4,5-tetrahydro-1H-benzo[d]azepin-7-yl)-phenyl-methanone;
(5-Methyl-2,3,4,5-tetrahydro-1H-benzo[d]azepin-7-yl)-phenyl-methanone;
6-Benzyl-1-methyl-2,3,4,5-tetrahydro-1H-benzo[d]azepin-7-ol;
8-Benzyl-7-fluoro-1-methyl-2,3,4,5-tetrahydro-1H-benzo[d]azepine;
8-(3-Fluoro-benzyl)-1-methyl-2,3,4,5-tetrahydro-1H-benzo[d]azepin-7-ol; and
7-(3-Fluoro-benzyloxy)-1-methyl-2,3,4,5-tetrahydro-1H-benzo[d]azepine;
or diastereomer or enantiomer thereof, or pharmaceutically acceptable salt, solvate,
or hydrate thereof. These compounds are disclosed in US20080009478.

The 5-HT$_{2C}$ receptor agonists include, for example, a compound selected from a group consisting of:

1-(3-fluoro-5-thiophene-3-yl-phenyl)-piperazine;
1-(3-fluoro-5-furan-3-yl-phenyl)-piperazine;
1-(2-fluoro-5-thiophene-3-yl-phenyl)-piperazine;
1-(2-fluoro-5-pyridine-3-yl-phenyl)-piperazine;
1-(2-fluoro-5-furan-3-yl-phenyl)-piperazine;
1-(2-fluoro-5-thiophene-2-yl-phenyl)-piperazine;
1-(4-fluoro-3-pyridine-3-yl-phenyl)-piperazine;
1-(5-fluoro-biphenyl-3-yl)-piperazine;
1-(5,2'-difluoro-biphenyl-3-yl)-piperazine;
1-(5,3'-difluoro-biphenyl-3-yl)-piperazine;
1-(5,4' -difluoro-biphenyl-3-yl)-piperazine;
1-(4-fluoro-biphenyl-3-yl)-piperazine;
1-(6-fluoro-biphenyl-3-yl)-piperazine;
1-(2-fluoro-biphenyl-3-yl)-piperazine;
1-(2,2'-difluoro-biphenyl-3-yl)-piperazine;
1-(2,3'-difluoro-biphenyl-3 -yl)-piperazine;
1-(2,4'-difluoro-biphenyl-3-yl)-piperazine;
1-(2-chloro-biphenyl-3-yl)-piperazine;
1-(5 -fluoro-2' -methyl-biphenyl-3 -yl)-piperazine;
1-(5-fluoro-3'-methyl-biphenyl-3-yl)-piperazine;
1-(5 -fluoro-4' -methyl-biphenyl-3 -yl)-piperazine;
1-(5 -fluoro-2' -methoxy-biphenyl-3 -yl)-piperazine;
1-(5-fluoro-3'-methoxy-biphenyl-3-yl)-piperazine;
1-(5 -fluoro-4' -methoxy-biphenyl-3 -yl)-piperazine;
1-(5-fluoro-2'-trifluoromethyl-biphenyl-3-yl)-piperazine;
1-(5-fluoro-3'-trifluoromethyl-biphenyl-3-yl)-piperazine; and
1-(5-fluoro-4'-trifluoromethyl-biphenyl-3-yl)-piperazine
or diastereomer or enantiomer thereof, or pharmaceutically acceptable salt, solvate,
or hydrate thereof. These compounds are disclosed in US20080119477.

The 5-HT$_{2C}$ receptor agonists include, for example, a compound selected from a group consisting of:

1-(3-fluoro-5-thiophene-3-yl-phenyl)-2-methyl-piperazine;
1-(3-fluoro-5-pyridine-3-yl-phenyl)-2-methyl-piperazine;
1-(3-fluoro-5-furan-3-yl-phenyl)-2-methyl-piperazine;
1-(4-fluoro-3-pyridine-3-yl-phenyl)-2-methyl-piperazine;
1-(5 -fluoro-biphenyl-3 -yl-)-2-methyl-piperazine;
1-(5,2'-difluoro-biphenyl-3-yl)-2-methyl-piperazine;

1-(6-fluoro-biphenyl-3-yl)-2-methyl-piperazine;
2-methyl-1-(5-phenyl-pyridine-3-yl)-piperazine;
2-methyl-1-(6-phenyl-pyridine-2-yl)-piperazine; and
1-(2-fluoro-biphenyl-3 -yl)-2-methyl-piperazine;
or diastereomer or enantiomer thereof, or pharmaceutically acceptable salt, solvate,
or hydrate thereof. These compounds are disclosed in US20080255137.

The 5-HT$_{2C}$ receptor agonists include, for example, a compound selected from a group consisting of:

1-(5,3'-difluoro-biphenyl-3-yl)-2-methyl-piperazine;
1-(5,4' -difluoro-biphenyl-3-yl)-2-methyl-piperazine;
2-methyl-1-(5,2',6'-trifluorobiphenyl-3-yl)-piperazine;
1-(4,3'-difluoro-biphenyl-3-yl)-2-methyl-piperazine;
1-(4,4'-difluoro-biphenyl-3-yl)-2-methyl-piperazine; and
1-biphenyl-3 -yl-2-methyl-piperazine;
or diastereomer or enantiomer thereof, or pharmaceutically acceptable salt, solvate,
or hydrate thereof. These compounds are disclosed in US20080255137.

The 5-HT$_{2C}$ receptor agonists include, for example, a compound selected from a group consisting of:

N-(2,2-difluoro-ethyl)-7-methyl-1,2,3,4,6,7-hexahydro-[1,4]diazepino[6,7,1-hi]indole-8-carboxamide;
(S)-N-(2,2-difluoro-ethyl)-7-methyl-1,2,3,4,6,7-hexahydro-[1,4]diazepino[6,7,1-hi]indole-8-carboxamide;
(R)-N-(2,2-difluoro-ethyl)-7-methyl-1,2,3,4,6,7-hexahydro-[1,4]diazepino[6,7,1-hi]indole-8-carboxamide;
N-(2,2-difluoro-ethyl)-7,7-dimethyl-1,2,3,4,6,7-hexahydro-[1,4]diazepino[6,7,1-hi]indole-8-carboxamide;
N-(2,2-difluoro-ethyl)-2,3,4,6-tetrahydro-1H-spiro[[1,4]diazepino[6,7,1-hi]indole-7,1'-cyclobutane]-8-carboxamide
(S)-N-(2,2-difluoro-ethyl)-7-ethyl-1,2,3,4,6,7-hexahydro-[1,4]diazepino[6,7,1-hi]indole-8-carboxamide;
(R)-N-(2,2-difluoro-ethyl)-7-ethyl-1,2,3,4,6,7-hexahydro-[1,4]diazepino[6,7,1-hi]indole-8-carboxamide;
(S)-N-(2,2-difluoro-ethyl)-7-(2,2,2-trifluoroethyl)-1,2,3,4,6,7-hexahydro-[1,4]diazepino[6,7,1-hi]indole-8-carboxamide; and
(R)-N-(2,2-difluoro-ethyl)-7-(2,2,2-trifluoroethyl)-1,2,3,4,6,7-hexahydro-[1,4]diazepino[6,7,1-hi]indole-8-carboxamide;
or diastereomer or enantiomer thereof, or pharmaceutically acceptable salt, solvate,
or hydrate thereof. These compounds are disclosed in US20180186797.

The 5-HT$_{2C}$ receptor agonists include, for example, a compound selected from a group consisting of:

8'-ethyl-2',3',4',4a', 5'-pentahydro-1'H-dispiro[cyclopropane-1,6'-cyclopropane-7',1"-naphtho[1,8-cd]-azepine];
6',6'-dimethyl-2',3',4',4a',5',6'-hexahydro-1'H-spiro[cyclopropane-1,7'-naphtho[1,8-cd]azepine];
(S)-6',6'-dimethyl-2',3',4',4a',5',6'-hexahydro-1'H-spiro[cyclopropane-1,7'-naphtho[1,8-cd]azepine];
(R)-6',6'-dimethyl-2',3',4',4a',5',6'-hexahydro-1'H-spiro[cyclopropane-1,7'-naphtho[1,8-cd]azepine];
8'-fluoro-2',3',4',4a',5'-pentahydro-1'H-dispiro[cyclopropane-1,6'-cyclopropane-7',1"-naphtho[1,8-cd]-azepine];
8-bromo-1,2,3,4,4a,5,6,7-octahydronaphtho[1,8-cd]azepine;
1,2,3,4,4a,5,6,7-octahydronaphtho[1,8-cd]azepine;
2',3',4',4a',5'-pentahydro-1'H-dispiro[cyclobutane-1,6'-cyclopropane-7',1"-naphtho[1,8-cd]-azepine];
8-bromo-7,7-dimethyl-1,2,3,4,4a,5,6,7-octahydronaphtho[1,8-cd]azepine;
(S)-7,7- dimethyl-1,2,3,4,4a,5,6,7-octahydronaphtho[1,8-cd]azepine; 8-chloro-1,2,3,4,4a,5,6,7-octahydronaphtho[1,8-cd]azepine;
(R)-7',7'-dimethyl-2',3',4',4a',5',7'-hexahydro-1'H-spiro[cyclopropane-1,6'-naphtho[1,8-cd]azepine];
(R)-2',3',4',4a',5'-pentahydro-1'H-dispiro[cyclopropane-1,6'-cyclopropane-7',1"-naphtho[1,8-cd]-azepine];
(S)-2',3',4',4a',5'-pentahydro-1'H-dispiro[cyclopropane-1,6'-cyclopropane-7',1"-naphtho[1,8-cd]-azepine];
(S)-7',7'- dimethyl-2',3',4',4a',5',7'-hexahydro-1'H-spiro[cyclopropane-1,6'-naphtho[1,8-cd]azepine];
8'-methyl-2',3',4',4a',5'-pentahydro-1'H-dispiro[cyclopropane-1,6'-cyclopropane-7',1"-naphtho[1,8-cd]-azepine;
2',3',4',4a',5',6'-hexahydro-1'H-spiro[cyclohexane-1,7'-naphtho[1,8-cd]azepine]; (7aR)-5,6,7,7a,8,8a,9,10,11,11a-decahydro-4H-cyclopenta[5,6]naphtho[1,8-cd]azepine;
8'-fluoro-6',6'-dimethyl-2',3',4',4a',5',6'-hexahydro-1'H-spiro[cyclopropane-1,7'-naphtho[1,8-cd]azepine];
7-cyclopropyl-1,2,3,4,4a,5,6,7-octahydronaphtho[1,8-cd]azepine; 7',7'-dimethyl-2',3',4',4a',5',7'-hexahydro-1'H-spiro[cyclopropane-1,6'-naphtho[1,8-cd]azepine];
(R)-7,7-dimethyl-1,2,3,4,4a,5,6,7-octahydronaphtho[1,8-cd]azepine; (S)-2',3',4',4a',5',6'-hexahydro-1'H-spiro[cyclopropane-1,7'-naphtho[1,8-cd]azepine];

(S)-2',3',4',4a',5',6'-hexahydro-1'H-spiro[cyclobutane-1,7'-naphtho[1,8-cd]azepine];

(R)-2',3',4',4a',5',6'-hexahydro-1'H-spiro[cyclobutane-1,7'-naphtho[1,8-cd]azepine];

8-methoxy-1,2,3,4,4a,5,6,7-octahydronaphtho[1,8-cd]azepine;        8-cyclopropyl-1,2,3,4,4a,5,6,7-octahydronaphtho[1,8-cd]azepine; 2',3',4',4a',5',6'-hexahydro-1'H-spiro[cyclopropane-1,7'-naphtho[1,8-cd]azepine]; 2',3',4',4a',5'-pentahydro-1'H-dispiro[cyclopentane-1,6'-cyclopropane-7',1"-naphtho[1,8-cd]-azepine];

2',3',4',4a',5'-pentahydro-1'H-dispiro[cyclopropane-1,6'-cyclopropane-7',1"-naphtho[1,8-cd]-azepine];

2',3',4',4a',5',6'-hexahydro-1'H-spiro[cyclobutane-1,7'-naphtho[1,8-cd]azepine];    (7aS)-5,6,7,7a,8,8a,9,10,11,11a-decahydro-4H-cyclopenta[5,6]naphtho[1,8-cd]azepine;

(R)-8'-fluoro-2',3',4',4a',5'-pentahydro-1'H-dispiro[cyclopropane-1,6'-cyclopropane-7',1"-naphtho[1,8-cd]-azepine];

(S)-8'-fluoro-2',3',4',4a',5'-pentahydro-1'H-dispiro[cyclopropane-1,6'-cyclopropane-7',1''-naphtho[1,8-cd]-azepine];

7,7-dimethyl-1,2,3,4,4a,5,6,7-octahydronaphtho[1,8-cd]azepine;    (R)-2',3',4',4a',5',6'-hexahydro-1'H-spiro[cyclopropane-1,7'-naphtho[1,8-cd]azepine];

2',3',4',4a',5',6'-hexahydro-1'H-spiro[cyclopentane-1,7'-naphtho[1,8-cd]azepine]; 8-fluoro-1,2,3,4,4a,5,6,7-octahydronaphtho[1,8-cd]azepine; 1,1-dimethyl-3,3a,4,5,6,7-hexahydro-1H-isochromeno[5,4-cd]azepine; and 5,6,7,7a,8,8a,9,10,11,11a-decahydro-4H-cyclopenta[5,6]naphtho[1,8-cd]azepine;

or diastereomer or enantiomer thereof, or pharmaceutically acceptable salt, solvate, or hydrate thereof. These compounds are disclosed in US20180214455.

The 5-HT$_{2C}$ receptor agonists include, for example, a compound selected from a group consisting of:

N-(2-methoxyethyl)-1,2,3,4,6,7-hexahydro-[1,4]diazepino[6,7,1-hi]indole-8-carboxamide;

N- methyl- 1,2,3,4,6,7 -hexahydro- [1,4] diazepino [6,7,1 -hi] indole- 8 - carboxamide ;

N-(pyridin-3-yl)-1,2,3,4,6,7-hexahydro-[1,4]diazepino[6,7,1- hi]indole-8-carboxamide;

N-(3-methoxypropyl)-1,2,3,4,6,7-hexahydro-[1,4]diazepino[6,7,1- hi]indole-8-carboxamide;

N-propyl-1,2,3,4,6,7-hexahydro-[1,4]diazepino[6,7,1- hi]indole-8-carboxamide;

N-isopropyl-1,2,3,4,6,7-hexahydro-[1,4]diazepino[6,7,1-hi]indole-8-carboxamide;

N-cyclopropyl-1,2,3,4,6,7-hexahydro-[1,4]diazepino[6,7,1- hi]indole-8-carboxamide;

N-(2-ethoxyethyl)-1,2,3,4,6,7-hexahydro-[1,4]diazepino[6,7,1-hi]indole-8-carboxamide;

methyl 2-(1,2,3,4,6,7-hexahydro-[1,4]diazepino[6,7,1-hi]indole-8-carboxamido)acetate;

N-ethyl-1,2,3,4,6,7-hexahydro-[1,4]diazepino[6,7,1-hi]indole-8-carboxamide;

N-(pyridin-2-ylmethyl)-1,2,3,4,6,7-hexahydro-[1,4]diazepino[6,7,1-hi]indole-8-carboxamide;

N-((6-(trifluoromethyl)pyridin-3-yl)methyl)-1,2,3,4,6,7-hexahydro-[1,4]diazepino[6,7,1- hi]indole-8 -carboxamide;

N-(2-(methylthio)ethyl)-1,2,3,4,6,7-hexahydro-[1,4]diazepino[6,7,1-hi]indole-8-carboxamide;

N-(2-(methylsulfonyl)ethyl)-1,2,3,4,6,7-hexahydro-[1,4]diazepino[6,7,1-hi]indole-8-carboxamide;

N-(2-chloroethyl)-1,2,3,4,6,7-hexahydro-[1,4]diazepino[6,7,1-hi]indole-8-carboxamide;

N-(cyclopropylmethyl)-1,2,3,4,6,7-hexahydro-[1,4]diazepino[6,7,1-hi]indole-8-carboxamide;

N-(2-isopropoxyethyl)-1,2,3,4,6,7-hexahydro-[1,4]diazepino[6,7,1-hi]indole-8-carboxamide;

N-(2-(ethylthio)ethyl)-1,2,3,4,6,7-hexahydro-[1,4]diazepino[6,7,1-hi]indole-8-carboxamide;

N-(1-methoxypropan-2-yl)-1,2,3,4,6,7-hexahydro-[1,4]diazepino[6,7,1-hi]indole-8-carboxamide;

N-(1-ethoxypropan-2-yl)-1,2,3,4,6,7-hexahydro-[1,4]diazepino[6,7,1-hi]indole-8-carboxamide;

N-(2-methoxypropyl)-1,2,3,4,6,7-hexahydro-[1,4]diazepino[6,7,1-hi]indole-8-carboxamide;

N-(2-ethoxypropyl)-1,2,3,4,6,7-hexahydro-[1,4]diazepino[6,7,1-hi]indole-8-carboxamide;

N-(2-propoxyethyl)-1,2,3,4,6,7-hexahydro-[1,4]diazepino[6,7,1-hi]indole-8-carboxamide;

N-(2-phenoxyethyl)-1,2,3,4,6,7-hexahydro-[1,4]diazepino[6,7,1-hi]indole-8-carboxamide;

N-(2-methoxyethyl)-1,2,3,4,6,7-hexahydro-[1,4]diazepino[6,7,1-hi]indole-8-carbothioamide;

N-methyl-1,2,3,4,6,7-hexahydro-[1,4] diazepino [6,7,1-hi] indole- 8 - carbothioamide ;

N-(2-fluoroethyl)-1,2,3,4,6,7-hexahydro-[1,4]diazepino[6,7,1-hi]indole-8-carboxamide;

N-(3-fluoropropyl)-1,2,3,4,6,7-hexahydro-[1,4]diazepino[6,7,1-hi]indole-8-carboxamide; and

N-butyl-1,2,3,4,6,7-hexahydro-[1,4]diazepino[6,7,1-hi]indole-8-carboxamide; or diastereomer or enantiomer thereof, or pharmaceutically acceptable salt, solvate, or hydrate thereof. These compounds are disclosed in WO2015066344.

The 5-HT$_{2C}$ receptor agonists include, for example, a compound selected from a group consisting of:

N-(2-methoxyethyl)-7,7-dimethyl-1,2,3,4,6,7-hexahydro-[1,4]diazepino[6,7,1-hi]indole- 8-carboxamide;

N-(2-ethoxyethyl)-7-methyl-1,2,3,4,6,7-hexahydro-[1,4]diazepino[6,7,1-hi]indole-8-carboxamide;

N-(2-ethoxyethyl)-7-ethyl-1,2,3,4,6,7-hexahydro-[1,4]diazepino[6,7,1-hi]indole-8-carboxamide;

N,7,7-trimethyl-1,2,3,4,6,7-hexahydro-[1,4]diazepino[6,7,1-hi]indole-8-carboxamide;
-methyl-7-propyl-1,2,3,4,6,7-hexahydro-[1,4]diazepino[6,7,1- hi]indole-8-carboxamide;
(R)-N-methyl-7-propyl-1,2,3,4,6,7-hexahydro-[1,4]diazepino[6,7,1-hi]indole-8-carboxamide;
7-ethyl-N-methyl-1,2,3,4,6,7-hexahydro-[1,4]diazepino[6,7,1-hi]indole-8-carboxamide;
(S)-7-ethyl-N-methyl-1,2,3,4,6,7-hexahydro-[1,4]diazepino[6 ,7,1-hi] indole-8-carboxamide;
(R)-7-ethyl-N-methyl-1,2,3,4,6,7-hexahydro-[1,4]diazepino[6,7,1-hi]indole-8-carboxamide;
(S)-N-methyl-7-propyl-1,2,3,4,6,7-hexahydro-[1,4]diazepino[6,7,1-hi]indole-8-carboxamide;
N,7-dimethyl-1,2,3,4,6,7-hexahydro-[1,4]diazepino[6,7,1-hi]indole-8-carboxamide;
6-ethyl-N-methyl-1,2,3,4,6,7-hexahydro- [1,4] diazepino [6 ,7,1-hi] indole- 8-carboxamide;
N-methyl-7-(2,2,2-trifluoroethyl)-1,2,3,4,6,7-hexahydro-[1,4]diazepino[6,7,1-hi]indole-8- carboxamide;
(S)-N-methyl-7-(2,2,2-trifluoroethyl)-1,2,3,4,6,7-hexahydro-[1,4]diazepino[6,7,1-hi] indole-8 -carboxamide;
(R)-N-methyl-7-(2,2,2-trifluoroethyl)-1,2,3,4,6,7-hexahydro-[1,4]diazepino[6,7,1-hi] indole-8 -carboxamide;
N-methyl-7-(trifluoromethyl)-1,2,3,4,6,7-hexahydro-[1,4]diazepino[6,7,1-hi]indole-8- carboxamide;
N,6-dimethyl-1,2,3,4,6,7-hexahydro-[1,4] diazepino [6,7,1-hi] indole- 8 - carboxamide;
N,6,6-trimethyl-1,2,3,4,6,7-hexahydro-[1,4]diazepino[6,7,1-hi]indole-8-carboxamide;
7-(methoxymethyl)-N-methyl-1,2,3,4,6,7-hexahydro-[1,4]diazepino[6,7, - hi]indole-8- carboxamide;
N,4-dimethyl-1,2,3,4,6,7-hexahydro-[1,4] diazepino [6,7,1-hi] indole- 8 - carboxamide ;
N,4,4-trimethyl-1,2,3,4,6,7-hexahydro-[1,4]diazepino[6,7,1-hi] indole-8 - carboxamide ;
N,3-dimethyl-1,2,3,4,6,7-hexahydro-[1,4] diazepino [6,7,1-hi] indole- 8 - carboxamide; and
N,1-dimethyl-1,2,3,4,6,7-hexahydro-[1,4]diazepino[6,7,1-hi] indole- 8-carboxamide;
or diastereomer or enantiomer thereof, or pharmaceutically acceptable salt, solvate, or hydrate thereof. These compounds are disclosed in WO2015066344.

The 5-HT$_{2C}$ receptor agonists include, for example, a compound selected from a group consisting of:

N,4-dimethyl-1,2,3,4,6,7-hexahydro- [1,4] diazepino [6,7,1-hi] indole-8-carboxamide;
N-methyl-6-propyl-1,2,3,4,6,7-hexahydro-[1,4]diazepino[6,7, 1- hi]indole-8-carboxamide;
4-ethyl-N-methyl-1,2,3,4,6,7-hexahydro-[1,4]diazepino[6,7,1-hi]indole-8-carboxamide;
N-(2-ethoxyethyl)-7-(2,2,2-trifluoroethyl)-1,2,3,4,6,7-hexahydro-[1,4]diazepino[6,7,1-hi]indole-8-carboxamide;
N-butyl-7-(2,2,2-trifluoroethyl)-1,2,3,4,6,7-hexahydro-[1,4]diazepino[6,7,1-hi]indole-8- carboxamide;
N-propyl-7-(2,2,2-trifluoroethyl)-1,2,3,4,6, 7-hexahydro-[1,4]diazepino[6,7,1-hi]indole-8 carboxamide;
N-(2-methoxyethyl)-7-(2,2,2-trifluoroethyl)-1,2,3,4,6,7-hexahydro-[1,4]diazepino[6,7,1- hi]indole-8-carboxamide;
N-(2-isopropoxyethyl)-7-(2,2,2-trifluoroethyl)-1,2,3,4,6,7 -hexahydro-[1,4]diazepino[6,7,1-hi]indole-8-carboxamide;
N-ethyl-7-(2,2,2-trifluoroethyl)-1,2,3,4,6,7-hexahydro-[1,4]diazepino[6,7,1-hi]indole-8- carboxamide;
N-(2-fluoroethyl)-7-(2,2,2-trifluoroethyl)-1,2,3,4,6,7-hexahydro-[1,4]diazepino[6,7,1-hi]indole-8-carboxamide;
N-(2,2-difluoroethyl)-7-(2,2,2-trifluoroethyl)-1,2,3,4,6,7-hexahydro-[1,4]diazepino[6,7,1-hi]indole-8-carboxamide;
(R)-N-propyl-7-(2,2,2-trifluoroethyl)-1,2,3,4,6,7-hexahydro-[1,4]diazepino[6,7,1-hi]indole-8-carboxamide;
(S)-N-propyl-7-(2,2,2-trifluoroethyl)-1,2,3,4,6,7-hexahydro-[1,4]diazepino[6,7,1-hi]indole-8-carboxamide;
N,7-bis(2,2,2-trifluoroethyl)-1,2,3,4,6,7-hexahydro-[1,4]diazepino[6,7,1-hi]indole-8-carboxamide;
N-(2,2,3,3,3-pentafluoropropyl)-7-(2,2,2-trifluoroethyl)-1,2,3,4,6,7-hexahydro-[1,4]diazepino[6,7,1-hi']indole-8-carboxamide;
7-ethyl-N-(2-fluoroethyl)-1,2,3,4,6,7-hexahydro-[1,4]diazepino[6,7,1-hi]indole-8-carboxamide; and
N-(2,2-difluoroethyl)-7-ethyl-1,2,3,4,6,7-hexahydro-[1,4]diazepino[6,7,1-hi]indole-8- carboxamide;
or diastereomer or enantiomer thereof, or pharmaceutically acceptable salt, solvate, or hydrate thereof. These compounds are disclosed in WO2015066344.

The 5-HT$_{2C}$ receptor agonists include, for example, a compound selected from a group consisting of:

8-methoxy-1,2,3,4,6,7-hexahydro-[1,4]diazepino[6,7,1-hi]indole;
8-bromo-1,2,3,4,6, 7-hexahydro-[1,4]diazepino[6,7,1-hi]indole;
8 -chloro-1,2,3,4,6,7 -hexahydro-[1,4]diazepino [6,7,1-hi]indole ;
8-(trifluoromethyl)-1,2,3,4,6,7-hexahydro-[1,4]diazepino[6,7,1-hi] indole;
8-phenyl-1,2,3,4,6,7-hexahydro-[1,4]diazepino[6,7,1-hi]indole;
8-ethyl-1,2,3,4,6,7-hexahydro-[1,4]diazepino[6,7,1-hi'] indole;
8-methyl-1,2,3,4,6,7-hexahydro-[1,4]diazepino[6,7, 1-hi]indole;
7- methyl-1,2,3,4,6,7-hexahydro-[1,4]diazepino[6,7,1-hi]indol-9-ol;
8- benzyl-1,2,3,4,6,7-hexahydro-[1,4]diazepino[6,7,1-hi'] indole;
8-phenethyl-1,2,3,4,6,7-hexahydro-[1,4]diazepino[6,7,1-hi] indole;

8-propyl-1,2,3,4,6, 7-hexahydro-[1,4]diazepino[6,7, 1-hi]indole;

8-bromo-7,7-dimethyl-1,2,3,4,6,7-hexahydro-[1,4] diazepino[6,7,1-hi] indole;

8 -isobutyl-1,2,3,4,6,7-hexahydro- [1,4] diazepino [6 ,7,1-hi] indole ;

8-iodo-1,2,3,4,6,7-hexahydro-[1,4]diazepino[6,7,1-hi'] indole;

7,7- dimethyl-1,2,3,4,6,7-hexahydro-[1,4]diazepino[6,7,1-hi]indole;

7- methyl-1,2,3,4,6,7-hexahydro-[1,4]diazepino[6,7,1-hi]indole;

8- chloro-7,7-dimethyl-1,2,3,4,6,7-hexahydro-[1,4]diazepino[6,7,1-hi]indole;

7,8- dimethyl-1,2,3,4,6,7-hexahydro-[1,4]diazepino[6,7,1-hi]indole;

8 -fluoro-1,2,3,4,6,7-hexahydro-[1,4] diazepino [6,7,1-hi]indole ;

(S)-7,8-dimethyl-1,2,3,4,6,7-hexahydro-[1,4]diazepino[6,7,1-hi] indole;

(R)-7,8-dimethyl-1,2,3,4,6,7-hexahydro-[1,4]diazepino[6,7,1-hi] indole;

7,7,8 -trimethyl-1 ,2 ,3 ,4 ,6 ,7-hexahydro- [1,4] diazepino [6,7,1-hi] indole ;

8-ethyl-7,7-dimethyl-1,2,3,4,6,7-hexahydro-[1,4]diazepino[6,7,1-hi]indole;

9-bromo-7-methyl-1,2,3,4,6,7-hexahydro-[1,4]diazepino[6,7,1-hi]indole;

9-chloro-7-methyl-1,2,3,4,6,7-hexahydro-[1,4]diazepino[6,7,1-hi]indole;

9-fluoro-8-methyl-1,2,3,4,6,7-hexahydro-[1,4]diazepino[6,7,1-hi]indole;

9-bromo-7,7-dimethyl-1,2,3,4,6,7-hexahydro-[1,4]diazepino[6,7,1-hi] indole;

9-chloro-7,7-dimethyl-1,2,3,4,6,7-hexahydro-[1,4]diazepino[6,7,1-hi]indole;

8-fluoro-7-methyl-1,2,3,4,6,7-hexahydro-[1,4]diazepino[6,7,1-hi]indole;

2,2',3,3',4,5',6,6'-octahydro-1H-spiro[[1,4] diazepino [6,7,1-hi] indole-7,4'-pyran];

8 -bromo-7-methyl- 1,2,3,4,6,7-hexahydro- [1,4] diazepino [6,7,1-hi] indole ;

(1,2,3,4,6,7-hexahydro- [1,4] diazepino [6,7,1-hi] indol-8-yl)methanol;

(1 ,2 ,3 ,4 ,6 ,7-hexahydro- [1,4] diazepino [6,7,1-hi] indol-8-yl)methanamine ;

8-(piperidin-1-ylmethyl)-1,2,3,4,6,7-hexahydro-[1,4]diazepino[6,7,1-hi] indole;

8 -cyclopropyl-7,7-dimethyl-1,2,3,4,6,7-hexahydro- [1,4] diazepino [6 ,7,1-hi] indole;

8 -cyclopropyl-1,2,3,4,6,7 -hexahydro- [1,4] diazepino [6,7,1-hi] indole ;

8-fluoro-7,7-dimethyl-1,2,3,4,6,7-hexahydro-[1,4]diazepino[6,7,1-hi]indole;

8-chloro-7-methyl-1,2,3,4,6,7-hexahydro-[1,4]diazepino[6,7,1-hi]indole;

8-ethyl-7-methyl-1,2,3,4,6,7-hexahydro-[1,4]diazepino[6,7,1-z]indole;

8- cyclopropyl-7-methyl-1,2,3,4,6,7-hexahydro-[1,4]diazepino[6,7,1-hi]indole;

(7-methyl-1,2,3,4,6,7-hexahydro-[1,4]diazepino[6,7,1-hi]indol-8-yl)methanol;

7,7-dimethyl-1,2,3,4,6,7-hexahydro-[1,4]diazepino[6,7,1-hi]indol-8-amine;

ethyl (1,2,3,4,6,7-hexahydro-[1,4]diazepino[6,7,1-hi]indol-8-yl)carbamate;

N-(1,2,3,4,6,7-hexahydro-[1,4]diazepino[6,7,1-hi]indol-8-yl)butyramide;

9- bromo-8-chloro-7-methyl-1,2,3,4,6,7-hexahydro-[1,4]diazepino[6,7,1-hi]indole;

8,9-dichloro-7-methyl-1,2,3,4,6, 7-hexahydro-[1,4]diazepino[6,7, 1-hi]indole;

8- bromo-9-chloro-7-methyl-1,2,3,4,6,7-hexahydro-[1,4]diazepino[6,7,1-hi] indole;

8-chloro-7,9-dimethyl-1,2,3,4,6,7-hexahydro-[1,4]diazepino[6,7,1-hi]indole;

9- chloro-7,8-dimethyl-1,2,3,4,6,7-hexahydro-[1,4]diazepino[6,7,1-hi]indole;

2,3,4,6-tetrahydro-1H-spiro[[1,4]diazepino[6,7,1-hi]indole-7,1'-cyclobutane];

7,7-diethyl-1,2,3,4,6,7-hexahydro-[1,4]diazepino[6,7,1-hi]indole;

methyl 3-(1,2,3,4,6,7-hexahydro-[1,4]diazepino[6,7,1-hi]indol-8-yl)propanoate;

8-(2-ethoxyethyl)-1,2,3,4,6,7-hexahydro-[1,4]diazepino[6,7,1-hi]indole;

7,7,9-trimethyl-1,2,3,4,6,7-hexahydro-[1,4]diazepino[6,7,1-hi]indole;

9-cyclopropyl-7 ,7 -dimethyl-1,2,3,4,6,7-hexahydro- [1,4] diazepino [6 ,7,1-hi] indole;

6-methyl-1,2,3,4,6,7-hexahydro-[1,4]diazepino[6,7,1-hi]indole;

7,9-dimethyl-1,2,3,4,6,7-hexahydro-[1,4]diazepino[6,7,1-hi]indole;

(6R,7R)-6,7-dimethyl-1,2,3,4,6,7-hexahydro-[1,4]diazepino[6,7,1-hi]indole;

(6S,7R)-6,7-dimethyl-1,2,3,4,6,7-hexahydro-[1,4]diazepino[6,7,1-hi]indole;

8 -bromo-6-methyl- 1,2,3,4,6,7-hexahydro- [1,4]diazepino[6,7,1-hi] indole ;

2,3,4,6-tetrahydro-1H-spiro[[1,4]diazepino[6,7,1-hi]indole-7,1'-cyclopropane];

6,8-dimethyl-1,2,3,4,6,7-hexahydro-[1,4]diazepino[6,7,1-hi]indole;

7- (2,2,2-trilluoroethyl)-1,2,3,4,6,7-hexahydro-[1,4]diazepino[6,7,1-hi]indole;

(R)-3,7,7-trimethyl-1,2,3,4,6,7-hexahydro-[1,4]diazepino[6,7,1-hi]indole;

(7,7-dimethyl-1,2,3,4,6,7-hexahydro-[1,4]diazepino[6,7,1-hi]indol-8-yl)methanol;

(S)-3,7,7-trimethyl-1,2,3,4,6,7-hexahydro-[1,4]diazepino[6,7,1-hi]indole;

7,7-dimethyl-2,4,6,7-tetrahydro-1H-spiro[[1,4]diazepino[6,7,1-hi]indole-3,1',-cyclopropane] ;

4,7,7-trimethyl-1,2,3,4,6,7-hexahydro-[1,4]diazepino [6,7,1-hi] indole ;

4-ethyl-7,7-dimethyl-1,2,3,4,6,7-hexahydro-[1,4]diazepino[6,7,1-hi]indole;
4-methyl-2,3,4,6-tetrahydro-1H-spiro[[1,4]diazepino[6,7,1-hi]indole-7,1'-cyclobutane];
4-cyclopropyl-7,7-dimethyl-1,2,3,4,6,7-hexahydro-[1,4] diazepino [6,7,1-hi] indole;
9-methoxy-7-methyl-1,2,3,4,6,7-hexahydro- [1,4] diazepino [6,7,1-hi] indole ;
8- fluoro-4,7,7-trimethyl-1,2,3,4,6,7-hexahydro-[1,4]diazepino[6,7,1-hi]indole;
1,2,3,4,6,7-hexahydro-[1,4]diazepino[6,7,1-hi]indole-8-carbonitrile;
N-((1,2,3,4,6,7-hexahydro-[1,4]diazepino[6,7,1-hi]indol-8-yl)methyl)-2-methoxyethanamine;
1-(1,2,3,4,6,7-hexahydro-[1,4]diazepino[6,7,1-hi]indol-8-yl)-N,N-dimethylmethanamine;
8-(pyrrolidin-1-ylmethyl)-1,2,3,4,6,7-hexahydro-[1,4]diazepino[6,7,1-hi]indole;
N-((1,2,3,4,6,7-hexahydro-[1,4]diazepino[6,7,1-hi]indol-8-yl)methyl)-3-methoxypropan-1-amine;
1-(1,2,3,4,6,7-hexahydro-[1,4]diazepino[6,7,1-hi]indol-8-yl)-N-methylmethanamine;
8- ((4-methoxypiperidin-1-yl)methyl)-1,2,3,4,6,7-hexahydro-[1,4]diazepino[6,7,1-hi]indole;
N-((1,2,3,4,6,7-hexahydro-[1,4]diazepino[6,7,1-hi]indol-8-yl)methyl)pyridin-4-amine;
N-(7,7-dimethyl-1,2,3,4,6,7-hexahydro-[1,4]diazepino[6,7,1-hi]indol-8-yl)acetamide;
N-(1,2,3,4,6,7-hexahydro-[1,4]diazepino[6,7,1-hi]indol-8-yl)acetamide;
1,2,3,4,6,7-hexahydro-[1,4]diazepino[6,7,1-hi]indol-8-amine;
N-(1,2,3,4,6,7-hexahydro-[1,4]diazepino[6,7,1-hi]mdol-8-yl)propionamide;
ethyl (7,7-dimethyl-1,2,3,4,6,7-hexahydro-[1,4]diazepino[6,7,1-hi]indol-8-yl)carbamate;
9-fluoro-7,7-dimethyl-1,2,3,4,6,7-hexahydro-[1,4]diazepino[6,7,1-hi]indole;
7,7-dimethyl-9-propyl-1,2,3,4,6,7-hexahydro-[1,4]diazepino[6,7,1-hi]indole; and
2,3,4,6-tetrahydro-1H-spiro[[1,4]diazepino[6,7,1-hi]indole-7,1'-cyclopentane];
or diastereomer or enantiomer thereof, or pharmaceutically acceptable salt, solvate,
or hydrate thereof. These compounds are disclosed in WO2015066344.

The 5-HT$_{2C}$ receptor agonists include, for example, a compound selected from a group consisting of:

8-bromo-1,2,3,4,6,7-hexahydro-[1,4]diazepino[6,7,1-hi]indole;
8-chloro-1,2,3,4,6,7-hexahydro-[1,4]diazepino[6,7,1-hi]indole ;
8-(trifluoromethyl)-1,2,3,4,6,7-hexahydro-[1,4]diazepino[6,7,1-hi]indole;
8-ethyl-1,2,3,4,6,7-hexahydro-[1,4]diazepino[6,7,1-hi]indole;
8-methyl-1,2,3,4,6,7-hexahydro-[1,4]diazepino[6,7,1-hi]indole;
7- methyl-1,2,3,4,6,7-hexahydro-[1,4]diazepino[6,7,1-hi]indol-9-ol;
8- propyl-1,2,3,4,6,7-hexahydro-[1,4]diazepino[6,7,1-hi]indole;
8-bromo-7,7-dimethyl-1,2,3,4,6,7-hexahydro-[1,4]diazepino[6,7,1-hi]indole;
8 -isobutyl-1,2,3,4,6,7-hexahydro- [1,4] diazepino [6 ,7,1-hi]indole ;
8-iodo-1,2,3,4,6,7-hexahydro-[1,4]diazepino[6,7,1-hi']indole;
7,7- dimethyl-1,2,3,4,6,7-hexahydro-[1,4]diazepino[6,7,1-hi]indole;
7-methyl-1,2,3,4,6,7-hexahydro-[1,4]diazepino[6,7,1-hi]indole;
8- chloro-7,7-dimethyl-1,2,3,4,6,7-hexahydro-[1,4]diazepino[6,7,1-hi]indole;
7,8- dimethyl-1,2,3,4,6,7-hexahydro-[1,4]diazepino[6,7,1-hi]indole;
(S)-7,8-dimethyl-1,2,3,4,6,7-hexahydro-[1,4]diazepino[6,7,1-hi]indole;
(R)-7,8-dimethyl-1,2,3,4,6, 7-hexahydro-[1,4]diazepino[6,7, 1-hi]indole;
7,7,8-trimethyl-1,2,3,4,6,7-hexahydro-[1,4]diazepino[6,7,1-hi]indole;
8-ethyl-7,7-dimethyl-1,2,3,4,6,7-hexahydro-[1,4]diazepino[6,7,1-hi]indole;
8-fluoro-7-methyl-1,2,3,4,6,7-hexahydro-[1,4]diazepino[6,7,1-hi] indole;
8-bromo-7-methyl- 1,2,3,4,6,7-hexahydro- [1,4] diazepino [6,7,1-hi] indole ;
8-cyclopropyl-7 ,7-dimethyl-1,2,3,4,6,7-hexahydro- [1,4]diazepino[6 ,7,1-hi] indole ;
8 -cyclopropyl-1,2,3,4,6,7-hexahydro- [1,4]diazepino[6,7,1-hi] indole ;
8-fluoro-7,7-dimethyl-1,2,3,4,6,7-hexahydro-[1,4]diazepino[6,7,1-hi] indole;
8-chloro-7-methyl-1,2,3,4,6,7-hexahydro-[1,4]diazepino[6,7,1-hi] indole;
8-ethyl-7-methyl-1,2,3,4,6,7-hexahydro-[1,4] diazepino[6,7,1-hi]indole ;
8-cyclopropyl-7-methyl-1,2,3,4,6,7-hexahydro-[1,4]diazepino[6,7,1-hi]indole;
9-bromo-8-chloro-7-methyl-1,2,3,4,6,7-hexahydro-[1,4]diazepino[6,7,1-hi] indole;
8, 9-dichloro-7-methyl-1,2,3,4,6,7-hexahydro-[1,4]diazepino[6,7,1-hi]indole;
8-bromo-9-chloro-7-methyl-1,2,3,4,6,7-hexahydro-[1,4]diazepino[6,7,1-hi] indole;
8-chloro-7,9-dimethyl-1,2,3,4,6,7-hexahydro-[1,4]diazepino[6,7,1-hi]indole;
9-chloro-7,8-dimethyl-1,2,3,4,6,7-hexahydro-[1,4]diazepino[6,7,1-hi]indole;
2,3,4,6-tetrahydro-1H-spiro[[1,4]diazepino[6,7,1-hi]indole-7,1'-cyclobutane];

7,7-diethyl-1,2,3,4,6,7-hexahydro-[1,4]diazepino[6,7,1-hi]indole;
2,3,4,6-tetrahydro-1H-spiro[[1,4]diazepino[6,7,1-hi]indole-7,1'-cyclopropane];
7-(2,2,2-trifluoroethyl)-1,2,3,4,6,7-hexahydro-[1,4]diazepino[6,7,1-hi]indole; and
2,3,4,6-tetrahydro-1H-spiro[[1,4]diazepino[6,7,1-hi]indole-7,1'-cyclopentane];
or diastereomer or enantiomer thereof, or pharmaceutically acceptable salt, solvate,
or hydrate thereof. These compounds are disclosed in WO2015066344.

The 5-HT$_{2C}$ receptor agonists include, for example, a compound selected from a group consisting of:

8-bromo-2,3,4,6-tetrahydro-1H-spiro[[1,4]diazepino[6,7,1-hi]indole-7,1'-cyclobutane] ;
8-(1H-1,2,4-triazol-1-yl)-1,2,3,4,6,7-hexahydro-[1,4]diazepino[6,7,1-hi]indole;
8-chloro-2,3,4,6-tetrahydro-1H-spiro[[1,4]diazepino[6,7,1-hi]indole-7,1'-cyclobutane] ;
8-methyl-2,3,4,6-tetrahydro-1H-spiro[[1,4]diazepino[6,7,1-hi]indole-7,1'-cyclobutane];
8-(1H-pyrazol-1-yl)-1,2,3,4,6,7-hexahydro-[1,4]diazepino[6,7,1-hi]indole;
8-(1H-imidazol-1-yl)-1,2,3,4,6,7-hexahydro-[1,4]diazepino[6,7,1-hi]indole;
8-(1H-pyrrol-2-yl)-1,2,3,4,6,7-hexahydro-[1,4]diazepino[6,7,1-hi]indole;
8-bromo-7-(2,2,2-trifluoroethyl)-1,2,3,4,6,7-hexahydro-[14]diazepino[6,7,1-hi] indole;
8-(thiophen-3-yl)-1,2,3,4,6,7-hexahydro-[1,4]diazepino[6,7,1-hi]indole;
8-(1H-pyrazol-3-yl)-1,2,3,4,6,7-hexahydro-[1,4]diazepino[6,7,1-hi]indole ;
8-(1H-pyrazol-5-yl)-1,2,3,4,6,7-hexahydro-[1,4]diazepino[6,7,1-hi]indole;
8-(methoxymethyl)-1,2,3,4,6,7-hexahydro-[1,4]diazepino[6,7,1-hi]indole;
8-(isopropoxymethyl)-1,2,3,4,6,7-hexahydro-[1,4]diazepino[6,7,1-hi']indole;
8-(3,3,3-trifluoropropyl)-1,2,3,4,6,7-hexahydro-[1,4]diazepino[6,7,1-hi]indole;
8-(furan-2-yl)-1,2,3,4,6,7-hexahydro-[1,4]diazepino[6,7,1-hi]indole; and
8-(1H-pyrazol-4-yl)-1,2,3,4,6,7-hexahydro-[1,4]diazepino[6,7,1-hi]indole;
or diastereomer or enantiomer thereof, or pharmaceutically acceptable salt, solvate,
or hydrate thereof. These compounds are disclosed in WO2015066344.

The 5-HT$_{2C}$ receptor agonists include, for example, a compound selected from a group consisting of:

(R)-4-(benzo[d][1,3]dioxol-5-yl)-6,6a,7,8,9,10-hexahydro-5H-pyrazino[1,2-a] [1,8]naphthyridine;
(R)-N-methyl-6,6a,7,8,9,10-hexahydro-5H-pyrazino[1,2-a][1,8]naphthyridine-4-carboxamide;
(R)-3-propyl-6,6a,7,8,9,10-hexahydro-5H-pyrazino[1,2-a][1,8]naphthyridine;        (R)-2-chloro-N-(6,6a,7,8,9,10-hexahydro-5H-pyrazino[1,2-a][1,8]naphthyridin-4-yl)benzamide;
(R)-4-(3-(trifluoromethoxy)phenyl)-6,6a,7,8,9,10-hexahydro-5H-pyrazino[1,2-a][1,8]naphthyridine;
(R)-4-benzyl-6,6a,7,8,9,10-hexahydro-5H-pyrazino[1,2-a][1,8]naphthyridine;        (R)-4-(2-methoxyethyl)-6,6a,7,8,9,10-hexahydro-5H-pyrazino[1,2-a][1,8]naphthyridine;
(R)-4-pentyl-6,6a,7,8,9,10-hexahydro-5H-pyrazino[1,2-a][1,8]naphthyridine;        (6aR)-4-(pentan-2-yl)-6,6a,7,8,9,10-hexahydro-5H-pyrazino[1,2-a][1,8]naphthyridine;
(R)-4-ethyl-6,6a,7,8,9,10-hexahydro-5H-pyrazino[1,2-a][1,8]naphthyridine;
(R)-4-isopropyl-6,6a,7,8,9,10-hexahydro-5H-pyrazino[1,2-a][1,8]naphthyridine;
(R)-4-butyl-6,6a,7,8,9,10-hexahydro-5H-pyrazino[1,2-a][1,8]naphthyridine;
(R)-4-propyl-6,6a,7,8,9,10-hexahydro-5H-pyrazino[1,2-a][1,8]naphthyridine;
(R)-4-isobutyl-6,6a,7,8,9,10-hexahydro-5H-pyrazino[1,2-a][1,8]naphthyridine;
(R)-4-(3-fluorobenzyl)-6,6a,7,8,9,10-hexahydro-5H-pyrazino[1,2-a][1,8]naphthyridine;
(R)-4-(2-fluorobenzyl)-6,6a,7,8,9,10-hexahydro-5H-pyrazino[1,2-a][1,8]naphthyridine;
(R)-4-methyl-6,6a,7,8,9,10-hexahydro-5H-pyrazino[1,2-a][1,8]naphthyridine;
(R)-4-isopentyl-6,6a,7,8,9,10-hexahydro-5H-pyrazino[1,2-a][1,8]naphthyridine;
(R)-4-(methoxymethyl)-6,6a,7,8,9,10-hexahydro-5H-pyrazino[1,2-a][1,8]naphthyridine;
(R)-4-(cyclohexylmethyl)-6,6a,7,8,9,10-hexahydro-5H-pyrazino[1,2-a][1,8]naphthyridine;
(R)-4-neopentyl-6,6a,7,8,9,10-hexahydro-5H-pyrazino[1,2-a][1,8]naphthyridine;
cyclobutyl((R)-6,6a,7,8,9,10-hexahydro-5H-pyrazino[1,2-a][1,8]naphthyridin-4-yl)methanol;
(R)-ethyl(6,6a,7,8,9,10-hexahydro-5H-pyrazino[1,2-a][1,8]naphthyridin-4-yl)carbamate;
(R)-N-(6,6a,7,8,9,10-hexahydro-5H-pyrazino[1,2-a][1,8]naphthyridin-4-yl)-2-phenylacetamide;
(R)-N-(6,6a,7,8,9,10-hexahydro-5H-pyrazino[1,2-a][1,8]naphthyridin-4-yl)butyramide;
(R)-4-(thiophen-2-yl)-6,6a,7,8,9,10-hexahydro-5H-pyrazino[1,2-a][1,8]naphthyridine;        (R)-4-cyclohexyl-6,6a,7,8,9,10-hexahydro-5H-pyrazino[1,2-a][1,8]naphthyridine;
(R)-4-(4-fluorobenzyl)-6,6a,7,8,9,10-hexahydro-5H-pyrazino[1,2-a][1,8]naphthyridine;

(R)-4-ethyl-3-propyl-6,6a,7,8,9,10-hexahydro-5H-pyrazino[1,2-a][1,8]naphthyridine;
(R)-3-benzyl-4-ethyl-6,6a,7,8,9,10-hexahydro-5H-pyrazino[1,2-a][1,8]naphthyridine;
(R)-N-(6,6a,7,8,9,10-hexahydro-5H-pyrazino[1,2-a][1,8]naphthyridin-4-yl)pyrrolidine-1-carboxamide;
(6aR)-4-((tetrahydro-2H-pyran-2-yl)methyl)-6,6a,7,8,9,10-hexahydro-5H-pyrazino[1,2-a][1,8]naphthyridine;
(R)-4-(((tetrahydro-2H-pyran-4-yl)methoxy)methyl)-6,6a,7,8,9,10-hexahydro-5H-pyrazino[1,2-a][1,8]naphthyridine;
(R)-(2-(trifluoromethyl)phenyl)-6,6a,7,8,9,10-hexahydro-5H-pyrazino[1,2-a][1,8]naphthyridine;
(R)-4-(4-methoxyphenyl)-6,6a,7,8,9,10-hexahydro-5H-pyrazino[1,2-a][1,8]naphthyridine;
4-bromo-6,6a,7,8,9,10-hexahydro-5H-pyrazino[1,2-a][1,8]naphthyridine;
4-(cyclobutylmethyl)-6,6a,7,8,9,10-hexahydro-5H-pyrazino[1,2-a][1,8]naphthyridine;
(R)-2,3-difluoro-N-(6,6a,7,8,9,10-hexahydro-5H-pyrazino[1,2-a][1,8]naphthyridin-4-yl)benzamide;
(R)-N-(6,6a,7,8,9,10-hexahydro-5H-pyrazino[1,2-a][1,8]naphthyridin-4-yl)benzamide;
(R)-N-(2,2-difluoroethyl)-6,6a,7,8,9,10-hexahydro-5H-pyrazino[1,2-a][1,8]naphthyridine-4-carboxamide;
(R)-N-(6,6a,7,8,9,10-hexahydro-5H-pyrazino[1,2-a][1,8]naphthyridin-4-yl)cyclopropanecarboxamide;
(R)-3-(6,6a,7,8,9,10-hexahydro-5H-pyrazino[1,2-a][1,8]naphthyridin-4-yl)propanenitrile;
(R)-4-(pyridin-2-ylmethyl)-6,6a,7,8,9,10-hexahydro-5H-pyrazino[1,2-a][1,8]naphthyridine;
(R,E)-4-(but-2-en-1-yl)-6,6a,7,8,9,10-hexahydro-5H-pyrazino[1,2-a][1,8]naphthyridine;
(R)-4-(isopropoxymethyl)-6,6a,7,8,9,10-hexahydro-5H-pyrazino[1,2-a][1,8]naphthyridine;
(R)-4-(5-chloropyridin-2-yl)-6,6a,7,8,9,10-hexahydro-5H-pyrazino[1,2-a][1,8]naphthyridine;
(R)-4-cyclopentyl-6,6a,7,8,9,10-hexahydro-5H-pyrazino[1,2-a][1,8]naphthyridine;
(R)-6,6a,7,8,9,10-hexahydro-5H-pyrazino[1,2-a][1,8]naphthyridine;
(R)-4-cyclobutyl-6,6a,7,8,9,10-hexahydro-5H-pyrazino[1,2-a][1,8]naphthyridine;
R)-4-chloro-6,6a,7,8,9,10-hexahydro-5H-pyrazino[1,2-a][1,8]naphthyridine;
(R)-4-cyclopropyl-6,6a,7,8,9,10-hexahydro-5H-pyrazino[1,2-a][1,8]naphthyridine;
(R)-4-(3,3,3-trifluoropropyl)-6,6a,7,8,9,10-hexahydro-5H-pyrazino[1,2-a][1,8]naphthyridine;
(R)-6,6a,7,8,9,10-hexahydro-5H-pyrazino[1,2-a][1,7]naphthyridine;
(R)-7-(cyclobutylmethyl)-2,3,4,4a,5,6-hexahydro-1H-pyrazino[1,2-a][1,6]naphthyridine;
(R)-2,3,4,4a,5,6-hexahydro-1H-pyrazino[1,2-a][1,6]naphthyridine;
(R)-4-bromo-5,6,6a,7,8,9,10,11-octahydro-[1,4]diazepino[1,2-a][1,8]naphthyridine;
(R)-4-(3,3,3-trifluoropropyl)-5,6,6a,7,8,9,10,11-octahydro-[1,4]diazepino[1,2-a][1,8]naphthyridine;
5-methyl-6,6a,7,8,9,10-hexahydro-5H-pyrazino[1,2-a][1,8]naphthyridine;
(R)-4-chloro-2-(methylthio)-5,6,6a,7,8,9,10,11-octahydropyrimido[5',4':5,6]pyrido[1,2-a][1,4]diazepine;
(R)-4-chloro-5,6,6a,7,8,9,10,11-octahydropyrimido[5',4':5,6]pyrido[1,2-a][1,4]diazepine;
(R)-4-phenethyl-6,6a,7,8,9,10-hexahydro-5H-pyrazino[1,2-a][1,8]naphthyridine;
(R)-3-(4-ethyl-6,6a,7,8,9,10-hexahydro-5H-pyrazino[1,2-a][1,8]naphthyridin-3-yl)propanenitrile;
(R)-4-ethyl-3-(isopropoxymethyl)-6,6a,7,8,9,10-hexahydro-5H-pyrazino[1,2-a][1,8]naphthyridine;
(R)-3-(cyclohexylmethyl)-4-ethyl-6,6a,7,8,9,10-hexahydro-5H-pyrazino[1,2-a][1,8]naphthyridine;
(6aR)-4-ethyl-3-((tetrahydro-2H-pyran-2-yl)methyl)-6,6a,7,8,9,10-hexahydro-5H-pyrazino[1,2-a][1,8]naphthyridine;
(R)-3-cyclobutyl-4-ethyl-6,6a,7,8,9,10-hexahydro-5H-pyrazino[1,2-a][1,8]naphthyridine;
(R)-3-chloro-4-(3,3,3-trifluoropropyl)-6,6a,7,8,9,10-hexahydro-5H-pyrazino[1,2-a][1,8]naphthyridine;
(R)-8-methyl-4-(3,3,3-trifluoropropyl)-6,6a,7,8,9,10-hexahydro-5H-pyrazino[1,2-a][1,8]naphthyridine;
(R)-4-chloro-2-(methylthio)-6,6a,7,8,9,10-hexahydro-5H-pyrazino[1',2':1,6]pyrido[2,3-d]pyrimidine;
(R)-4-(cyclopentylmethyl)-6,6a,7,8,9,10-hexahydro-5H-pyrazino[1,2-a][1,8]naphthyridine;
(R)-3-(cyclopentylmethyl)-4-ethyl-6,6a,7,8,9,10-hexahydro-5H-pyrazino[1,2-a][1,8]naphthyridine;
(R)-4-bromo-6,6a,7,8,9,10-hexahydro-5H-pyrazino[1,2-a][1,7]naphthyridine;
(R)-4-propyl-6,6a,7,8,9,10-hexahydro-5H-pyrazino[1,2-a][1,7]naphthyridine;
(R)-4-(cyclohexylmethyl)-6,6a,7,8,9,10-hexahydro-5H-pyrazino[1,2-a][1,7]naphthyridine; and
(R)-4-benzyl-6,6a,7,8,9,10-hexahydro-5H-pyrazino[1,2-a][1,7]naphthyridine;
(R)-4-(cyclobutylmethyl)-6,6a,7,8,9,10-hexahydro-5H-pyrazino[1,2-a][1,8]naphthyridine;

or diastereomer or enantiomer thereof, or pharmaceutically acceptable salt, solvate, or hydrate thereof. These compounds are disclosed in WO2018035477.

The 5-HT$_{2C}$ receptor agonists include, for example, a compound selected from a group consisting of:

8-chloro-1-methyl-2,3,4,5-tetrahydro-1H-3-benzazepine;
8-bromo-1-methyl-2,3,4,5-tetrahydro-1H-3-benzazepine;
8-iodo-1-methyl-2,3,4,5-tetrahydro-1H-3-benzazepine;

8-trifluoromethyl-1-methyl-2,3,4,5-tetrahydro-1H-3-benzazepine;
8-trifluoromethyl-1-ethyl-2,3,4,5-tetrahydro-1H-3-benzazepine;
8-chloro-1-ethyl-2,3,4,5-tetrahydro-1H-3-benzazepine;
8-bromo-1-ethyl-2,3,4,5-tetrahydro-1H-3-benzazepine;
8-iodo-1-ethyl-2,3,4,5-tetrahydro-1H-3-benzazepine;
7,8-dichloro-1-methyl-2,3,4,5-tetrahydro-1H-3-benzazepine;
7,8-dichloro-1-ethyl-2,3,4,5-tetrahydro-1H-3-benzazepine;
8-chloro-7-fluoro-1-methyl-2,3,4,5-tetrahydro-1H-3-benzazepine;
8-chloro-7-methoxy-1-methyl-2,3,4,5-tetrahydro-1H-3-benzazepine; and
8-chloro-7-fluoro-1-ethyl-2,3,4,5-tetrahydro-1H-3-benzazepine;
or diastereomer or enantiomer thereof, or pharmaceutically acceptable salt, solvate,
or hydrate thereof. These compounds are disclosed in US8153621.

The 5-HT$_{2C}$ receptor agonist may be Compound A or salt thereof (e.g., hydroxy chloride salt), or Compound B or salt thereof (e.g., hydroxy chloride salt), which can be used in the present invention.

The 5-HT$_{2C}$ receptor agonist may be a non-solvate or may be a solvate. The solvate may be a solvate of ethanol or water. The solvate which incorporate water as a solvent is a hydrate. The hydrate encompasses stoichiometric hydrates as well as hydrates containing various amounts of water.

Examples of the salt of the 5-HT$_{2C}$ receptor agonist (Compound A, Compound B, etc.) include salts with inorganic bases, ammonium salts, salts with organic bases, salts with inorganic acids, salts with organic acids, and salts with basic or acidic amino acids.

Preferred examples of the salts with inorganic bases include: alkali metal salts such as sodium salt and potassium salt; alkaline earth metal salts such as calcium salt, magnesium salt, and barium salt; and aluminum salts.

Preferred examples of the salts with organic bases include salts with trimethylamine, triethylamine, pyridine, picoline, ethanolamine, diethanolamine, triethanolamine, dicyclohexylamine, and N,N'-dibenzylethylenediamine.

Preferred examples of the salts with inorganic acids include salts with hydrochloric acid, hydrobromic acid, nitric acid, sulfuric acid, and phosphoric acid.

Preferred examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.

Preferred examples of the salts with basic amino acids include salts with arginine, lysine, and ornithine.

Preferred examples of the salts with acidic amino acids include salts with aspartic acid and glutamic acid.

Among these salts, a pharmaceutically acceptable salt is preferred.

In a particular embodiment of the present invention, the pharmaceutically acceptable salt of Compound A is hydrochloride salt. In a particular embodiment of the present invention, the pharmaceutically acceptable salt of Compound B is hydrochloride salt.

The present inventors have found that Compound A or a salt thereof exerts a greater body weight lowering effect than placebo by 3.8% and 2.6% at an average plasma concentration in a day of 203 ng/mL and 116 ng/mL, respectively, based on its free form in human subjects. In addition, at least the greater body weight lowering effect than placebo by 3.8% is recognized necessary for launching as an anti-obesity drug (Non Patent Literature 2).

According to the present invention, Compound A, or a salt thereof, has urethra-closing function-enhancing effects (treatment effect on stress urinary incontinence) at greater than 2 ng/mL based on its free form in human subjects.

Thus, in the present invention, Compound A, or a salt thereof, is useful in treating stress urinary incontinence by administration to a human subject in need thereof, resulting in a plasma concentration range from greater than 2 ng/mL to less than 203 ng/mL, based on its free form with showing no substantial body weight lowering effect.

In one embodiment of the invention, Compound A, or a salt thereof, when administered to a patient suffering from stress urinary incontinence, results in a plasma concentration range from about 45 ng/mL to less than 203 ng/mL, based on its free form, and elicits no body weight lowering effect. In another embodiment of the invention, Compound A, or a salt thereof, when administered to a patient suffering from stress urinary incontinence, results in a plasma concentration range from about 45 ng/mL to 116 ng/mL, based on its free form, and elicits no body weight lowering effect.

In another embodiment of the invention, Compound A, or a salt thereof, when administered to a patient suffering from stress urinary incontinence, results in a plasma concentration range from greater than 2 ng/mL to about 116 ng/mL, based on its free form eliciting no body weight lowering effect. In another embodiment of the invention, Compound A, or a salt thereof, when administered to a patient suffering from stress urinary incontinence, results in a plasma concentration range from greater than 2 ng/mL to about 45 ng/mL, based on its free form, eliciting no body weight lowering effect, while reducing the frequency of other adverse effects.

Compound B or a salt thereof is known to exert a greater body weight lowering effect than placebo by 3.8% and 2.6% at an average plasma concentration in a day of 43 ng/mL and 24 ng/mL, based on its free form in human subjects (Non Patent Literature 2), which corresponds to b.i.d. administration of 10 mg and q.d. administration of 10 mg, respectively (Non Patent Literatures 2 to 4).

According to the present invention, Compound B or a salt thereof is analyzed to have urethra-closing function-enhancing effects (treatment effect on stress urinary incontinence) at greater than 0.32 ng/mL based on its free form in human subjects.

Thus, in the present invention, Compound B or a salt thereof may treat stress urinary incontinence by administration resulting in its plasma concentration range from greater than 0.32 ng/mL to less than 43 ng/mL based on its free form to a human subject with showing no body weight lowering effect.

In another embodiment of the invention, Compound B or a salt thereof, when administered to a patient suffering from stress urinary incontinence, results in a plasma concentration range from about 7.1 ng/mL to less than 43 ng/mL, based on its free form, eliciting no body weight lowering effect. In another embodiment of the invention, preferably Compound B or a salt thereof, when administered to a patient suffering from stress urinary incontinence, results in a plasma concentration range from about 7.1 ng/mL to 24 ng/mL, based on its free form, eliciting no body weight lowering effect.

In another embodiment of the invention, Compound B or a salt thereof, when administered to a patient suffering from stress urinary incontinence, results in a plasma concentration range from greater than 0.32 ng/mL to about 24 ng/mL, based on its free form, eliciting no body weight lowering effect. In another embodiment of the invention, , Compound B or a salt thereof, when administered to a patient suffering from stress urinary incontinence results in a plasma concentration range from greater than 0.32 ng/mL to about 7.1 ng/mL, based on its free form, eliciting no body weight lowering effect, while reducing the frequency of other adverse effects.

The daily dosage of Compound A or a salt thereof may vary depending on the administration route or the dosage form. In addition, the preferred duration to keep the effective plasma concentration for treating SUI in a patient (also referred to as "effective duration") may depend on the patient's demand. For example, in case where patients want to avoid SUI only during a three-hour trip, a medicament with at least 3-hour effective duration may be sufficient to meet the demand. In case of full-time employment, at least 8 to 12 hour-effectiveness may be preferred. Therefore, a preferred effective duration may depend on patient's demand, and a medicament with variety of effective duration, for example, from 1 hour to 24 hours maybe useful in the treatment of SUI. Therefore, the administration of Compound A or a salt thereof which provides the effective plasma concentration for an effective duration from 1 hour to 24 hours, may be used for the treatment of SUI. Furthermore, the medicament comprising the effective amount of Compound A or a salt thereof which provide the effective plasma concentration for the duration from 1 hour to 24 hours also may be used for the treatment of SUI. For this purpose, the daily dosage of Compound A or a salt thereof may be administered in one portion or two divided or three divided portions per day. A plasma concentration of Compound A as a free base form may range from greater than 2 ng/mL to less than 203 ng/mL, for example, from 45 ng/mL to less than 203 ng/mL, from 45 ng/mL to 116 ng/mL, from greater than 2 ng/mL to 116 ng/mL, from greater than 2 ng/mL to 45 ng/mL according to patient's need. In some embodiments, the onset of action of Compound A is about 1 hour after administration and lasts up to 24 hours after administration. In some embodiments, the plasma concentration of Compound A when attained lasts up to 3 hours, or up to 6 hours, or up to 8 hours, or up to 16 hours, or up to 24 hours.

The daily dosage of Compound B or a salt thereof may vary depending on the administration route or the dosage form. In addition, as explained above, the duration required to keep the effective plasma concentration for treating SUI in a patient may depend on the patient's demand, and, for example, the medicament providing variety of effective duration from 1 hour to 24 hours may be preferred. Therefore, the administration of Compound B or a salt thereof, which provide the effective plasma concentration for the duration from 1 hour to 24 hours, may be used for the treatment of SUI. Furthermore, the medicament comprising Compound B or a salt thereof which provides the effective plasma concentration for the duration from 1 hour to 24 hours also may be used for the treatment of SUI. For this purpose, the daily dosage of Compound B or a salt thereof may be administered in one portion or two divided or three divided portions per day. A plasma concentration of Compound B as a free base form may ranges from greater than 0.32 ng/mL to less than 43 ng/mL, for example, from greater than 7.1 ng/mL to less than 43 ng/mL, from 7.1 ng/mL to 24 ng/mL, from greater than 0.32 ng/mL to 24 ng/mL, from greater than 0.32 ng/mL to 7.1 ng/mL for 1 hour to 24 hours according to patient's demand. In some embodiments, the onset of action of Compound B is about 1 hour after administration and lasts up to 24 hours after administration. In some embodiments, the plasma concentration of Compound B when attained lasts up to 3 hours, or up to 6 hours, or up to 8 hours, or up to 16 hours, or up to 24 hours. In some embodiments, Compound B or a salt thereof may be administered in a form of immediate release formulation. In some embodiments, Compound B or a salt thereof may be administered in a form of sustained release formulation.

In order to cause a weight lowering effect larger than that shown in a placebo group by 3.8%, an immediate release formulation containing 10 mg of Compound B is required to be continuously administered twice daily (Non Patent Literature 2). Further, an average blood concentration comparable to that achieved when an immediate release formulation containing 10 mg of Compound B is administered twice daily can be obtained by continuously administering a sustained release formulation containing 20 mg of Compound B once daily (Non Patent Literature 3). Further, an immediate release formulation containing 10 mg of Compound B to be administered once daily has not been approved as an anti-obesity drug (Non Patent Literature 2). From these results, it can be considered that 10 mg or less of Compound B as a total daily dose would show no weight lowering effect.

The duration during which urethra-closing function-enhancing effects are achieved will vary depend on a demand of a patient having stress urinary incontinence. For example, the effects may be required only during a one-hour trip. Therefore, a dose of Compound B or a salt thereof which will achieve a blood concentration more than 0.32 ng/mL as a free form for a required duration can be used in a patient having stress urinary incontinence. Examples of the relationship between a dose of Compound B and a salt thereof and the blood level of Compound B as a free form are as follows. The maximum blood concentration and a half-life in blood after a single dose of 10 mg of Compound B' are 46.0 ng/mL and 11.1 hours, respectively (Non Patent Literature 4). Therefore, a single dose of an immediate release formulation containing 0.1 mg or more of Compound B or a salt thereof may achieve a blood concentration more than 0.32 ng/mL as a free form. Further, the blood concentration causing a sufficient urethra-closing function-enhancing effect was calculated as 7.1 ng/mL of Compound B (see Example 10). Therefore, a single dose of an immediate release formulation containing 1.5 mg or more of Compound B or a salt thereof may achieve a blood concentration more than 7.1 ng/mL as a free form. The maximum blood concentration after an immediate release formulation comprising 10 mg of Compound B' is administered once daily is 40 ng/mL (Blossom trial). Therefore, administering once daily an immediate release formulation containing 0.1 mg or more of Compound B or a salt thereof may achieve a blood concentration more than 0.32 ng/mL as a free form. Further, administering once daily an immediate release formulation containing 1.8 mg or more of Compound B or a salt thereof may achieve a blood concentration more than 7.1 ng/mL as a free form. The maximum blood concentration after an immediate release formulation comprising 10 mg of Compound B' is administered twice daily is 56 ng/mL (Blossom trial). Therefore, administering twice daily an immediate release formulation containing 0.1 mg or more of Compound B or a salt thereof may achieve a blood concentration more than 0.32 ng/mL as a free form. Further, administering twice daily an immediate release formulation containing 1.3 mg or more of Compound B or a salt thereof may achieve a blood concentration more than 7.1 ng/mL as a free form. The blood concentration may be 39 ng/mL after a single administration of a sustained release formulation containing 20 mg of Compound B or a salt thereof. Therefore, administering the single dose of a sustained release formulation containing 0.2 mg or more of Compound B or a salt thereof may achieve a blood concentration more than 0.32 ng/mL as a free form. Further, administering the single dose of a sustained release formulation containing 3.6 mg or more of Compound B or a salt thereof may achieve a blood concentration more than 7.1 ng/mL as a free form. The blood concentration after a sustained release formulation containing 20 mg of Compound B or a salt thereof once daily is 74 ng/mL. Therefore, administering once daily a sustained release formulation containing 0.1 mg or more of Compound B or a salt thereof may achieve a blood concentration more than 0.32 ng/mL as a free form. Further, administering once daily a sustained release formulation containing 1.9 mg or more of Compound B or a salt thereof may achieve a blood concentration more than 7.1 ng/mL as a free form.

From the above reasons, it is preferable to administer a total daily dose ranging between 0.1 mg and 10 mg of Compound B in order to obtain urethra-closing function-enhancing effects without showing weight lowering effect by administering Compound B or a salt thereof in an immediate release formulation or a sustained release formulation. For example, in an embodiment, a dose ranging, for example, but not limited to, between 0.1 mg and 1 mg, 1 mg and 2 mg, 2 mg and 3 mg, 3 mg and 4 mg, 4 mg and 5 mg, 5 mg and 6 mg, 6 mg and 7 mg, 7 mg and 8 mg, 8mg and 9 mg, or 9 mg and 10 mg, but not limited to the above can be administered. Further, considering stronger efficacy and reduced adverse effects, the lower limit of a total daily dose may be 0.15 mg, 0.2 mg, 0.25 mg, 0.3 mg, 0.35 mg, 0.4 mg, 0.45 mg, 0.5 mg, 0.55 mg, 0.6 mg. 0.65 mg, 0.7 mg, 0.75 mg, 0.8 mg, 0.85 mg, 0.9 mg, 0.95 mg, 1 mg, 2 mg, 3 mg, 4 mg, or 5mg, and then the upper limit of a total daily dose may be 9 mg, 8 mg, 7 mg, 6 mg, 5 mg, 4 mg, 3 mg, 2 mg, or 1 mg. therefore, in a preferred embodiment, 0.2 mg to 8 mg, 0.3 mg to 7 mg, 0.4 mg to 6 mg, or 0.5 mg to 5 mg as a total daily dose may be administered. In an embodiment, the total daily dose can be administered at a single dose or separately in a multiple dose (for example, twice, three times, four times or more). As for a multiple dose per day of a sustained release formulation containing Compound B, those skilled in the art can easily determine the number of dose and the interval between the doses. For example, as a dose causing a strong drug efficacy with fewer adverse effects, 1.5 mg to 10 mg is preferably administered when an immediate release formulation is administered as a single dose, 1.8 mg to 10 mg is preferably administered when an immediate release formulation is administered once daily, 1.3 mg to 10 mg is preferably administered when an immediate release formulation is administered twice daily, 3.6 mg to 10 mg is preferably administered when a sustained release formulation is administered as a single dose, 1.9 mg to 10 mg is preferably administered when a sustained release formulation is administered once daily.

In an alternative aspect of the present invention, the 5-HT$_{2C}$ receptor agonist such as Compound A and the salt thereof, and Compound B and the salt thereof may be used in the treatment of a disease such as incontinence of feces, prolapse of various organs, or dribbling after urination.

In some embodiments, Compound A' or Compound B' may be administered in combination with exercises to increase the strength of the pelvic floor muscles for the treatment of stress urinary incontinence.

In some embodiments, Compound A' or Compound B' may be administered in combination with exercises to increase the strength of the pelvic floor muscles for the treatment of a disease such as incontinence of feces, prolapse of various organs, or dribbling after urination.

The dosage of the 5-HT$_{2C}$ receptor agonist such as Compound A or Compound B may also differ depending on the type of the compound or the pharmaceutically acceptable salt thereof, the administration route, symptoms, the age of a patient, etc. In the case of, for example, oral administration to an adult subject, Compound A, a salt thereof, Compound B or a salt thereof may treat a disease such as incontinence of feces, prolapse of various organs, or dribbling after urination using the same plasma concentration and/or dosage as the aforementioned plasma concentration and/or dosage for preventing or treating stress urinary incontinence.

The medicament of the present invention may contain a pharmaceutically acceptable carrier in addition to the 5-HT$_{2C}$ receptor agonist.

Any of various organic or inorganic carrier materials routinely used as pharmaceutical materials may be used as the pharmaceutically acceptable carrier. Examples thereof include: excipients, lubricants, binding agents, and disintegrants for solid preparations; and solvents, solubilizing agents, suspending agents, isotonic agents, buffering agents, and soothing agents for liquid preparations. If necessary, pharmaceutical additives such as preservatives, antioxidants, stabilizers, colorants, and sweeteners may also be used.

In the case of administering the medicament of the present invention to an obese subject (i.e., a subject having a body mass index (BMI) $\geq$ 25) having stress urinary incontinence, the medicament of the present invention may be used in combination with an additional anti-obesity drug.

Specifically, in the case of administering the medicament of the present invention to an obese subject (i.e., a subject having a body mass index (BMI) $\geq$ 25) having stress urinary incontinence, the stress urinary incontinence may be treated by the administration of the medicament of the present invention at a dosage lower than the minimum dosage of the agonist as an anti-obesity drug to the subject or at a dosage that shows no body weight lowering effect to the subject, while the obesity may be treated by the administration of the additional anti-obesity drug to the subject. In this way, both obesity and stress urinary incontinence may be treated using the optimum medicament at the appropriate dosage for each of the diseases. For the combined use, the medicament of the present invention and the additional anti-obesity drug may be administered as a mixture, or the medicament of the present invention and the additional anti-obesity drug provided in separate forms may be administered either concurrently or separately.

In the present specification, the term "additional anti-obesity drug" means an anti-obesity drug having no or substantially no ability to activate 5-HT$_{2C}$ receptors.

Thus, a medicament of the present invention may contain an additional anti-obesity drug in addition to the 5-HT$_{2C}$ receptor agonist. In a certain aspect of the present invention, the present invention provides a medicament for use in treating stress urinary incontinence in an obese subject (i.e., a subject having a body mass index (BMI) $\geq$ 25) having stress urinary incontinence, comprising a 5-HT$_{2C}$ receptor agonist and an additional anti-obesity drug, wherein the 5-HT$_{2C}$ receptor agonist is administered at a dosage lower than the minimum dosage of the agonist as an anti-obesity drug to the subject or at a dosage that shows no anti-obesity effect to the subject.

The present invention also may provide a medicament for use in a treatment of stress urinary incontinence in an obese subject (i.e., a subject having a body mass index (BMI) $\geq$ 25) having stress urinary incontinence, comprising a 5-HT$_{2C}$ receptor agonist, wherein the 5-HT$_{2C}$ receptor agonist is administered at a dosage lower than the minimum dosage of the agonist as an anti-obesity drug to the subject or at a dosage that shows no anti-obesity effect to the subject, and administered in combination with an additional anti-obesity drug.

The present invention may further provide a combination medicament for use in treating stress urinary incontinence and obesity in an obese subject (i.e., a subject having a body mass index (BMI) $\geq$ 25) having stress urinary incontinence, the combination medicament comprising a combination of a 5-HT$_{2C}$ receptor agonist and an additional anti-obesity drug, wherein the 5-HT$_{2C}$ receptor agonist is administered at a dosage lower than the minimum dosage of the agonist as an anti-obesity drug to the subject or at a dosage that shows no anti-obesity effect to the subject.

Examples of the additional anti-obesity drug used in the present invention include, but are not particularly limited to, anti-central obesity drugs (e.g., dexfenfluramine, fenfluramine, phentermine, sibutramine, amfepramone, dexamphetamine, mazindol, phenylpropanolamine, and clobenzorex), pancreatic lipase inhibitors (e.g., orlistat), β3 agonists (e.g., CL-316243, SR-58611-A, UL-TG-307, AJ-9677, and AZ40140), peptidic appetite suppressants (e.g., leptin and CNTF (ciliary neurotrophic factor)), and cholecystokinin agonists (e.g., lintitript and FPL-15849).

In an alternative aspect, the medicament of the present invention may be administered in combination with an additional medicament for use in treating stress urinary incontinence.

The present invention provides a medicament for use in treating stress urinary incontinence, comprising a 5-HT$_{2C}$ receptor agonist, wherein the 5-HT$_{2C}$ receptor agonist is administered at a dosage lower than the minimum dosage of the agonist as an anti-obesity drug to a subject or at a dosage that shows no anti-obesity effect to a subject, and administered in combination with an additional medicament for use in treating stress urinary incontinence.

The present invention also provides a combination medicament for use in treating stress urinary incontinence, the combination medicament comprising a combination of a 5-HT$_{2C}$ receptor agonist and an additional medicament for use in treating stress urinary incontinence, wherein the 5-HT$_{2C}$ receptor agonist is administered at a dosage lower than the minimum dosage of the agonist as an anti-obesity drug to a subject or at a dosage that shows no anti-obesity effect to a subject.

In the present specification, the term "additional medicament for use in treating stress urinary incontinence" means a medicament for use in treating stress urinary incontinence having no or substantially no ability to activate 5-HT$_{2C}$ receptors.

The additional medicament for use in treating stress urinary incontinence may be, for example, a therapeutic drug that does not activate 5-HT$_{2C}$ receptors in the spinal cord. Examples of the additional medicament for use in treating stress urinary incontinence include, but are not particularly limited to, adrenaline $\alpha$1 receptor agonists (e.g., ephedrine hydrochloride and midodrine hydrochloride), adrenaline $\beta$2 receptor agonists (e.g., clenbuterol hydrochloride), noradrenaline uptake inhibitory substances, tricyclic antidepressants (e.g., imipramine hydrochloride), anticholinergic agents or smooth muscle relaxants (e.g., oxybutynin hydrochloride and propiverine hydrochloride), and female hormones (e.g., conjugated estrogen (Premarin) and estriol). The medicament of the present invention may be administered in combination with any of these drugs.

In a further alternative aspect, the medicament of the present invention may also be administered in combination with a medicament for use in treating incontinence of feces, for example, adrenaline $\alpha$1 receptor agonists (e.g., phenylephrine and the salt thereof), high-absorbent polymers (e.g., polycarbophil calcium), opioid receptor agonists (e.g., loperamide hydrochloride, trimebutine maleate), 5-HT$_3$ antagonists (e.g., ramosetron hydrochloride). The combination use of the present medicament with a medicament for use in treating incontinence of feces may be useful in a subject concomitantly having stress urinary incontinence and incontinence of feces.

In a further alternative aspect, the medicament of the present invention may also be administered in combination with a medicament for use in treating overactive bladder, for example, anticholinergic agents (e.g., solifenacin, imidafenacin, oxybutynin chloride, oxybutynin hydrochloride, darifenacin hydrochloride, tolterodine tartrate, fesoterodine fumarate, propiverine hydrochloride, trospium chloride, and afacifenacin fumarate), $\beta_3$ receptor stimulants (e.g., mirabegron, solabegron hydrochloride, ritobegron, vibegron, AK134 and TAC-301 [TRK-380]), and other drugs (flavoxate hydrochloride, *Clostridium botulinum* toxin A, ajulemic acid, and XED-D0501).

In addition, the 5-HT$_{2C}$ receptor agonist in the medicament of the present invention may be administered in combination with, for example, any of the following drugs (1) to (10) (hereinafter, the drugs mentioned above and mentioned below that may be used in combination therewith are collectively referred to as a "concomitant drug").

(1) Therapeutic agents for diabetes mellitus

Insulin preparations [e.g., animal insulin preparations extracted from the bovine or swine pancreas; human insulin preparations synthesized by genetically engineering using *Escherichia coli* or yeast; zinc insulin; protamine zinc insulin; and fragments or derivatives of insulin (e.g., INS-1)], insulin sensitizers (e.g., pioglitazone hydrochloride, troglitazone, rosiglitazone or its maleate, JTT-501, MCC-555, YM-440, GI-262570, KRP-297, FK-614, and CS-011), $\alpha$-glucosidase inhibitors (e.g., voglibose, acarbose, miglitol, and emiglitate), biguanides (e.g., phenformin, metformin, and buformin), sulfonylureas (e.g., tolbutamide, glibenclamide, gliclazide, chlorpropamide, tolazamide, acetohexamide, glyclopyramide, and glimepiride) and other insulin secretagogues (e.g., repaglinide, senaglinide, mitiglinide or its calcium salt hydrate, GLP-1, and nateglinide), dipeptidyl peptidase-IV inhibitors (e.g., NVP-DPP-278, PT-100, and P32/98), $\beta$3 agonists (e.g., CL-316243, SR-58611-A, UL-TG-307, AJ-9677, and AZ40140), amylin agonists (e.g., pramlintide), phosphotyrosine phosphatase inhibitors (e.g., vanadic acid), gluconeogenesis inhibitors (e.g., glycogen phosphorylase inhibitors, glucose-6-phosphatase inhibitors, and glucagon antagonists), SGLT (sodium-glucose cotransporter) inhibitors (e.g., T-1095), etc.

(2) Therapeutic agents for diabetic complications

Aldose reductase inhibitors (e.g., tolrestat, epalrestat, zenarestat, zopolrestat, fidarestat (SNK-860), minalrestat (ARI-509), and CT-112), neurotrophic factors (e.g., NGF and NT-3), AGE inhibitors (e.g., ALT-945, pimagedine, pyratoxatin, N-phenacylthiazolium bromide (ALT-766), and EXO-226), active oxygen scavengers (e.g., thioctic acid), cerebral vasodilators (e.g., tiapride), etc.

(3) Antihyperlipidemic agents

Statin compounds as cholesterol synthesis inhibitors (e.g., pravastatin, simvastatin, lovastatin, atorvastatin, fluvastatin, and cerivastatin, or their salts (e.g., sodium salt)), squalene synthase inhibitors or fibrate compounds having a triglyceride lowering effect (e.g., bezafibrate, clofibrate, simfibrate, and clinofibrate), etc.

(4) Antihypertensive agents

Angiotensin converting enzyme inhibitors (e.g., captopril, enalapril, and delapril), angiotensin II antagonists (e.g., losartan, candesartan, and cilexetil), calcium antagonists (e.g., manidipine, nifedipine, amlodipine, efonidipine, and nicardipine), clonidine, etc.

(5) Diuretics

Xanthine derivatives (e.g., theobromine sodium salicylate and theobromine calcium salicylate), thiazide preparations (e.g., ethiazide, cyclopenthiazide, trichlormethiazide, hydrochlorothiazide, hydroflumethiazide, benzylhydrochlorothiazide, penflutizide, polythiazide, and methyclothiazide), antialdosterone preparations (e.g., spironolactone and triamterene), carbonic anhydrase inhibitors (e.g., acetazolamide), chlorobenzenesulfonamide preparations (e.g., chlorthalidone, mefruside, and indapamide), azosemide, isosorbide, ethacrynic acid, piretanide, bumetanide, furosemide, etc.

(6) Chemotherapeutics

Alkylating agents (e.g., cyclophosphamide and ifosfamide), antimetabolites (e.g., methotrexate and 5-fluorouracil), anticancer antibiotics (e.g., mitomycin and adriamycin), plant-derived anticancer agents (e.g., vincristine, vindesine, and Taxol), cisplatin, carboplatin, etoposide, etc., among others, a 5-fluorouracil derivative Furtulon or Neofurtulon, etc.

(7) Immunotherapeutics

Microbial or bacterial components (e.g., muramyl dipeptide derivatives and Picibanil), polysaccharides having immunoenhancing activity (e.g., lentinan, sizofiran, and Krestin), cytokines obtained by genetic engineering approaches (e.g., interferon and interleukin (IL)), and colony-stimulating factors (e.g., granulocyte colony-stimulating factor and erythropoietin), among others, IL-1, IL-2, IL-12, etc.

(8) Drugs confirmed to have a cachexia ameliorating effect either in animal models or clinically

Progesterone derivatives (e.g., megestrol acetate) [Journal of Clinical Oncology, Vol. 12, p. 213-225, 1994], metoclopramide drugs (Id.), tetrahydrocannabinol drugs (Id.), agents improving fat metabolism (e.g., eicosapentaenoic acid) [British Journal of Cancer, Vol. 68, p. 314-318, 1993], growth hormone, IGF-1, antibodies against a cachexia-inducing factor TNF-$\alpha$, LIF, IL-6, or oncostatin M, etc.

(9) Antiphlogistics

Steroids (e.g., dexamethasone), sodium hyaluronate, cyclooxygenase inhibitors (e.g., indomethacin, ketoprofen, loxoprofen, meloxicam, ampiroxicam, celecoxib, and rofecoxib), etc.

(10) Therapeutic drug for constipation

For example, a medicament according to the present invention can be administered in case of incontinence of feces which will be a problem when a therapeutic drug for constipation exerts the improvement of constipation. In particular, the therapeutic drugs for constipation include, for example, enterokinesis-improving agent (cholinesterase inhibitor (neostigmine, physostigmine and the like), 5-HT$_4$ agonist (prucalopride, mosapride, and the like), ghrelin agonist (capromorelin and the like), motilin receptor agonist (camicinal, erythromycin, and the like), opioid antagonist (naltrexone, naloxegol, and the like), gastrointestinal secretion promoting agent (guanylate cyclase C agonist (linaclotide and the like), chloride channel 2 opener (lubiprostone and the like), sodium hydrogen antiporter 3 inhibitors (tenapanor and the like), anti-constipation medicine (sennoside, magnesium oxide, magnesium hydroxide, bisacodyl, polycarbophil calcium, sugar laxatives (lactulose, and the like), laxoberon, crude drugs having anti-constipation effect (psyllium, and the like), and the like)

(11) Others

Glycation inhibitors (e.g., ALT-711), nerve regeneration promoting agents (e.g., Y-128, VX853, and prosaptide), central nervous system agents (e.g., antidepressants such as desipramine, amitriptyline, imipramine, fluoxetine, paroxetine, and doxepin), antiepileptic agents (e.g., lamotrigine and carbamazepine), anti-arrhythmic agents (e.g., mexiletine), acetylcholine receptor ligands (e.g., ABT-594), endothelin receptor antagonists (e.g., ABT-627), monoamine uptake inhibitors (e.g., tramadol), indoleamine uptake inhibitors (e.g., fluoxetine and paroxetine), narcotic analgesics (e.g., morphine), GABA receptor agonists (e.g., gabapentin), GABA uptake inhibitors (e.g., tiagabine), $\alpha_2$ receptor agonists (e.g., clonidine), local analgesics (e.g., capsaicin), protein kinase C inhibitors (e.g., LY-333531), anti-anxiety agents (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate and sumatriptan), serotonin receptor antagonists (e.g., cyproheptadine hydrochloride and ondansetron), serotonin uptake inhibitors (e.g., fluvoxamine maleate, fluoxetine, and paroxetine), sleep inducing agents (e.g., triazolam and zolpidem), anticholinergic agents (e.g., atropine, scopolamine, homatropine, tropicamide, cyclopentolate, butylscopolamine bromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, and chloride trospium, or their salts thereof (e.g., atropine sulfate, scopolamine hydrobromide, homatropine hydrobromide, cyclopentolate hydrochloride, flavoxate hydrochloride, pirenzepine hydrochloride, trihexyphenidyl hydrochloride, oxybutynin chloride, and tolterodine tartrate)), acetylcholine esterase inhibitors (e.g., distigmine), $\alpha_1$) receptor blockers (e.g., tamsulosin), muscle relaxants (e.g., baclofen), $K^+$ channel openers (e.g., nicorandil), calcium channel blockers (e.g., nifedipine), preventive or therapeutic drugs for Alzheimer's disease (e.g., donepezil, rivastigmine, and galanthamine), therapeutic drugs for Parkinson's disease (e.g., L-dopa), preventive or therapeutic drugs for multiple sclerosis (e.g., interferon β-1a), histamine $H_1$ receptor inhibitors (e.g., promethazine hydrochloride), proton pump inhibitors (e.g., lansoprazole and omeprazole), antithrombotic agents (e.g., aspirin and cilostazol), NK-2 receptor antagonists, therapeutic drugs for HIV infection (saquinavir, zidovudine, lamivudine, and nevirapine), therapeutic drugs for chronic obstructive pulmonary disease (salmeterol, tiotropium bromide, and cilomilast), etc.

The medicament in which the 5-HT$_{2C}$ receptor agonist of the present invention and the concomitant drug may be used as a mixture or in combination includes all of a medicament obtained as a single formulation containing the 5-HT$_{2C}$ receptor agonist and the concomitant drug, and separate formulations of the 5-HT$_{2C}$ receptor agonist and the concomitant drug. Hereinafter, these formulations are collectively referred to as the combined use agents of the present invention.

The combined use agents of the present invention may be prepared by separately or simultaneously formulating the 5-HT$_{2C}$ receptor agonist and the concomitant drug, either directly or after mixing with a pharmaceutically acceptable carrier, etc., in the same way as in the aforementioned medicament comprising the 5-HT$_{2C}$ receptor agonist of the present invention. The daily dose of the combined use agents of the present invention differs depending on severity, the age, sex, or body weight of a recipient subject, difference in sensitivity, the time of administration, dosing intervals, the properties, prescription, or type of the medicament, the type of the active ingredient, etc., and is not particularly limited.

For the administration of the combined use agents of the present invention, the 5-HT$_{2C}$ receptor agonist and the concomitant drug may be administered at the same time. Alternatively, the concomitant drug may be first administered, followed by the administration of the 5-HT$_{2C}$ receptor agonist, or the 5-HT$_{2C}$ receptor agonist may be first administered, followed by the administration of the concomitant drug. In the case of administering the agonist and the concomitant drug in the separate manner, the time interval of the administrations differs depending on the active ingredient to be administered, the dosage form, and the administration method. Examples of the method for first administering the concomitant drug may include a method which involves administering the concomitant drug and administering the 5-HT$_{2C}$ receptor agonist within 1 minute to 3 days, preferably within 10 minutes to 1 day, more preferably within 15 minutes to 1 hour thereafter. Examples of the method for first administering the 5-HT$_{2C}$ receptor agonist may include a method which involves administering the 5-HT$_{2C}$ receptor agonist and administering the concomitant drug within 1 minute to 1 day, preferably within 10 minutes to 6 hours, more preferably within 15 minutes to 1 hour thereafter.

The respective amounts of the 5-HT$_{2C}$ receptor agonist and the concomitant drug in the combined use agents of the present invention containing the 5-HT$_{2C}$ receptor agonist and the concomitant drug in a single formulation differ depending on the form of the formulation and may usually be approximately 0.01 to 90% by weight, preferably approximately 0.1 to 50% by weight, more preferably approximately 0.5 to 20% by weight, with respect to the total weight of the preparation.

The content of the carrier in the combined use agents may usually be approximately 0 to 99.8% by weight, preferably approximately 10 to 99.8% by weight, more preferably approximately 10 to 90% by weight, with respect to the total weight of the preparation.

When the combined use agents of the present invention comprise separate medicaments respectively containing the 5-HT$_{2C}$ receptor agonist and the concomitant drug, the medicament containing the concomitant drug may be produced in the same way as in the medicament comprising the 5-HT$_{2C}$ receptor agonist of the present invention.

The medicament of the present invention may be any of solid formulations including powders, granules, tablets, and capsules, and liquid formulations including syrups and emulsions.

The medicament of the present invention may be produced by a routine method, for example, mixing, kneading, granulation, tableting, coating, sterilization, and/or emulsification, according to the form of the formulation. For such pharmaceutical production, see, for example, each section of "General Rules for Preparations" in the Japanese Pharmacopoeia. The medicament of the present invention may be prepared into a sustained-release agent containing the active ingredient and a biodegradable polymer compound. The sustained-release agent can be prepared according to a method described in Japanese Patent Laid-Open Publication No. H09-263545.

In a further aspect, the present invention provides a method for treating stress urinary incontinence in a subject in need thereof, comprising administering a 5-HT$_{2C}$ receptor agonist at a dosage lower than the minimum dosage of the agonist as an anti-obesity drug to the subject. The present invention also provides a method for treating stress urinary incontinence in a subject in need thereof, comprising administering a 5-HT$_{2C}$ receptor agonist at a dosage that shows no body weight lowering effect to the subject.

In another aspect, the present invention provides a method for treating stress urinary incontinence and obesity in an obese subject (i.e., a subject having a body mass index (BMI) $\geq$ 25) having stress urinary incontinence, comprising administering a 5-HT$_{2C}$ receptor agonist at a dosage lower than the minimum dosage of the agonist as an anti-obesity drug or at a dosage that shows no body weight lowering effect to the subject, and administering an additional anti-obesity drug to the subject.

In the case of administering the medicament of the present invention to an obese subject (i.e., a subject having a body mass index (BMI) $\geq$ 25) having incontinence of feces, the medicament of the present invention may be used in combination with an additional anti-obesity drug.

Specifically, in the case of administering the medicament of the present invention to an obese subject (i.e., a subject having a body mass index (BMI) $\geq$ 25) having incontinence of feces, the incontinence of feces may be treated by the administration of the medicament of the present invention at a dosage lower than the minimum dosage of the agonist as an anti-obesity drug to the subject or at a dosage that shows no body weight lowering effect to the subject, while the obesity may be treated by the administration of the additional anti-obesity drug to the subject. In this way, both obesity and incontinence of feces may be treated using the optimum medicament at the appropriate dosage for each of the diseases. For the combined use, the medicament of the present invention and the additional anti-obesity drug may be administered as a mixture, or the medicament of the present invention and the additional anti-obesity drug provided in separate forms may be administered either concurrently or separately.

In the present specification, the term "additional anti-obesity drug" means an anti-obesity drug having no or substantially no ability to activate 5-HT$_{2C}$ receptors.

Thus, a medicament of the present invention may contain an additional anti-obesity drug in addition to the 5-HT$_{2C}$ receptor agonist. In a certain aspect of the present invention, the present invention provides a medicament for use in treating incontinence of feces in an obese subject (i.e., a subject having a body mass index (BMI) $\geq$ 25) having incontinence of feces, comprising a 5-HT$_{2C}$ receptor agonist and an additional anti-obesity drug, wherein the 5-HT$_{2C}$ receptor agonist is administered at a dosage lower than the minimum dosage of the agonist as an anti-obesity drug to the subject or at a dosage that shows no anti-obesity effect to the subject.

The present invention also may provide a medicament for use in a treatment of incontinence of feces in an obese subject (i.e., a subject having a body mass index (BMI) $\geq$ 25) having incontinence of feces, comprising a 5-HT$_{2C}$ receptor agonist, wherein the 5-HT$_{2C}$ receptor agonist is administered at a dosage lower than the minimum dosage of the agonist as an anti-obesity drug to the subject or at a dosage that shows no anti-obesity effect to the subject, and administered in combination with an additional anti-obesity drug.

The present invention may further provide a combination medicament for use in treating incontinence of feces and obesity in an obese subject (i.e., a subject having a body mass index (BMI) $\geq$ 25) having incontinence of feces, the combination medicament comprising a combination of a 5-HT$_{2C}$ receptor agonist and an additional anti-obesity drug, wherein the 5-HT$_{2C}$ receptor agonist is administered at a dosage lower than the minimum dosage of the agonist as an anti-obesity drug to the subject or at a dosage that shows no anti-obesity effect to the subject.

Examples of the additional anti-obesity drug used in the present invention include, but are not particularly limited to, anti-central obesity drugs (e.g., dexfenfluramine, fenfluramine, phentermine, sibutramine, amfepramone, dexamphetamine, mazindol, phenylpropanolamine, and clobenzorex), pancreatic lipase inhibitors (e.g., orlistat), $\beta$3 agonists (e.g., CL-316243, SR-58611-A, UL-TG-307, AJ-9677, and AZ40140), peptidic appetite suppressants (e.g., leptin and CNTF (ciliary neurotrophic factor)), and cholecystokinin agonists (e.g., lintitript and FPL-15849).

In an alternative aspect, the medicament of the present invention may be administered in combination with an additional medicament for use in treating incontinence of feces.

The present invention provides a medicament for use in treating incontinence of feces, comprising a 5-HT$_{2C}$ receptor agonist, wherein the 5-HT$_{2C}$ receptor agonist is administered at a dosage lower than the minimum dosage of the agonist as an anti-obesity drug to a subject or at a dosage that shows no anti-obesity effect to a subject, and administered in combination with an additional medicament for use in treating incontinence of feces.

The present invention also provides a combination medicament for use in treating incontinence of feces, the combination medicament comprising a combination of a 5-HT$_{2C}$ receptor agonist and an additional medicament for use in treating incontinence of feces, wherein the 5-HT$_{2C}$ receptor agonist is administered at a dosage lower than the minimum dosage of the agonist as an anti-obesity drug to a subject or at a dosage that shows no anti-obesity effect to a subject.

In the present specification, the term "additional medicament for use in treating incontinence of feces" means a medicament for use in treating incontinence of feces having no or substantially no ability to activate 5-HT$_{2C}$ receptors.

The additional medicament for use in treating incontinence of feces may be, for example, a therapeutic drug that does not activate 5-HT$_{2C}$ receptors in the spinal cord. Examples of the additional medicament for use in treating incontinence of feces include drugs for treatment of diarrheal symptoms in patients with irritable bowel syndrome. Other examples of the additional medicament for use in treating incontinence of feces include, but are not particularly limited to, adrenaline $\alpha$1 receptor agonists (e.g., phenylephrine and the salt thereof), highly water-absorptive polymers (e.g., polycarbophil calcium), opioid receptor agonists (e.g., loperamide hydrochloride, trimebutine maleate), 5-HT$_3$receptor antagonists (e.g., ramosetron hydrochloride). The medicament of the present invention may be administered in combination with any of these drugs.

In a further alternative aspect, the medicament of the present invention may also be administered in combination with a medicament for use in treating stress urinary incontinence, for example, adrenaline $\alpha$1 receptor agonists (e.g., ephedrine hydrochloride and midodrine hydrochloride), adrenaline $\beta$2 receptor agonists (e.g., clenbuterol hydrochloride), noradrenaline uptake inhibitory substances, tricyclic antidepressants (e.g., imipramine hydrochloride), anticholinergic agents or smooth muscle relaxants (e.g., oxybutynin hydrochloride and propiverine hydrochloride), and female hormones (e.g., conjugated estrogen (Premarin) and estriol). The combination use of the present medicament with a medicament for use in treating stress urinary incontinence may be useful in a subject concomitantly having stress urinary incontinence and incontinence of feces.

In a further alternative aspect, the medicament of the present invention may also be administered in combination with a medicament for use in treating overactive bladder, for example, anticholinergic agents (e.g., solifenacin, imidafenacin, oxybutynin chloride, oxybutynin hydrochloride, darifenacin hydrochloride, tolterodine tartrate, fesoterodine fumarate, propiverine hydrochloride, trospium chloride, and afacifenacin fumarate), $\beta_3$ receptor stimulants (e.g., mirabegron, solabegron hydrochloride, ritobegron, vibegron, AK134 and TAC-301 [TRK-380]), and other drugs (flavoxate hydrochloride, *Clostridium botulinum* toxin A, ajulemic acid, and XED-D0501).

In addition, the 5-HT$_{2C}$ receptor agonist in the medicament of the present invention may be administered in combination with, for example, any of the following drugs (1) to (10) (hereinafter, the drugs mentioned above and mentioned below that may be used in combination therewith are collectively referred to as a "concomitant drug").

(1) Therapeutic agents for diabetes mellitus

Insulin preparations [e.g., animal insulin preparations extracted from the bovine or swine pancreas; human insulin preparations synthesized by genetically engineering using *Escherichia coli* or yeast; zinc insulin; protamine zinc insulin; and fragments or derivatives of insulin (e.g., INS-1)], insulin sensitizers (e.g., pioglitazone hydrochloride, troglitazone, rosiglitazone or its maleate, JTT-501, MCC-555, YM-440, GI-262570, KRP-297, FK-614, and CS-011), $\alpha$-glucosidase inhibitors (e.g., voglibose, acarbose, miglitol, and emiglitate), biguanides (e.g., phenformin, metformin, and buformin), sulfonylureas (e.g., tolbutamide, glibenclamide, gliclazide, chlorpropamide, tolazamide, acetohexamide, glyclopyramide, and glimepiride) and other insulin secretagogues (e.g., repaglinide, senaglinide, mitiglinide or its calcium salt hydrate, GLP-1, and nateglinide), dipeptidyl peptidase-IV inhibitors (e.g., NVP-DPP-278, PT-100, and P32/98), $\beta$3 agonists (e.g., CL-316243, SR-58611-A, UL-TG-307, AJ-9677, and AZ40140), amylin agonists (e.g., pramlintide), phosphotyrosine phosphatase inhibitors (e.g., vanadic acid), gluconeogenesis inhibitors (e.g., glycogen phosphorylase inhibitors, glucose-6-phosphatase inhibitors, and glucagon antagonists), SGLT (sodium-glucose cotransporter) inhibitors (e.g., T-1095), etc.

(2) Therapeutic agents for diabetic complications

Aldose reductase inhibitors (e.g., tolrestat, epalrestat, zenarestat, zopolrestat, fidarestat (SNK-860), minalrestat (ARI-509), and CT-112), neurotrophic factors (e.g., NGF and NT-3), AGE inhibitors (e.g., ALT-945, pimagedine, pyratoxatin, N-phenacylthiazolium bromide (ALT-766), and EXO-226), active oxygen scavengers (e.g., thioctic acid), cerebral vasodilators (e.g., tiapride), etc.

(3) Antihyperlipidemic agents

Statin compounds as cholesterol synthesis inhibitors (e.g., pravastatin, simvastatin, lovastatin, atorvastatin, fluvastatin, and cerivastatin, or their salts (e.g., sodium salt)), squalene synthase inhibitors or fibrate compounds having a triglyceride lowering effect (e.g., bezafibrate, clofibrate, simfibrate, and clinofibrate), etc.

(4) Antihypertensive agents

Angiotensin converting enzyme inhibitors (e.g., captopril, enalapril, and delapril), angiotensin II antagonists (e.g., losartan, candesartan, and cilexetil), calcium antagonists (e.g., manidipine, nifedipine, amlodipine, efonidipine, and nicardipine), clonidine, etc.

(5) Diuretics

Xanthine derivatives (e.g., theobromine sodium salicylate and theobromine calcium salicylate), thiazide preparations (e.g., ethiazide, cyclopenthiazide, trichlormethiazide, hydrochlorothiazide, hydroflumethiazide, benzylhydrochlorothiazide, penflutizide, polythiazide, and methyclothiazide), antialdosterone preparations (e.g., spironolactone and triamterene), carbonic anhydrase inhibitors (e.g., acetazolamide), chlorobenzenesulfonamide preparations (e.g., chlorthalidone, mefruside, and indapamide), azosemide, isosorbide, ethacrynic acid, piretanide, bumetanide, furosemide, etc.

(6) Chemotherapeutics

Alkylating agents (e.g., cyclophosphamide and ifosfamide), antimetabolites (e.g., methotrexate and 5-fluorouracil), anticancer antibiotics (e.g., mitomycin and adriamycin), plant-derived anticancer agents (e.g., vincristine, vindesine, and Taxol), cisplatin, carboplatin, etoposide, etc., among others, a 5-fluorouracil derivative Furtulon or Neofurtulon, etc.

(7) Immunotherapeutics

Microbial or bacterial components (e.g., muramyl dipeptide derivatives and Picibanil), polysaccharides having immunoenhancing activity (e.g., lentinan, sizofiran, and Krestin), cytokines obtained by genetic engineering approaches (e.g., interferon and interleukin (IL)), and colony-stimulating factors (e.g., granulocyte colony-stimulating factor and erythropoietin), among others, IL-1, IL-2, IL-12, etc.

(8) Drugs confirmed to have a cachexia ameliorating effect either in animal models or clinically

Progesterone derivatives (e.g., megestrol acetate) [Journal of Clinical Oncology, Vol. 12, p. 213-225, 1994], metoclopramide drugs (Id.), tetrahydrocannabinol drugs (Id.), agents improving fat metabolism (e.g., eicosapentaenoic acid) [British Journal of Cancer, Vol. 68, p. 314-318, 1993], growth hormone, IGF-1, antibodies against a cachexia-inducing factor TNF-$\alpha$, LIF, IL-6, or oncostatin M, etc.

(9) Antiphlogistics

Steroids (e.g., dexamethasone), sodium hyaluronate, cyclooxygenase inhibitors (e.g., indomethacin, ketoprofen, loxoprofen, meloxicam, ampiroxicam, celecoxib, and rofecoxib), etc.

(10) Therapeutic drug for constipation

Enterokinesis-improving agent (cholinesterase inhibitor (neostigmine, physostigmine and the like), 5-HT$_4$ agonist (prucalopride, mosapride, and the like), ghrelin agonist (capromorelin and the like), motilin receptor agonist (camicinal, erythromycin, and the like), opioid antagonist (naltrexone, naloxegol, and the like), gastrointestinal water secretion promoting agent (guanylate cyclase C agonist (linaclotide and the like), chloride channel 2 opener (lubiprostone and the like), sodium hydrogen antiporter 3 inhibitors (tenapanor and the like), anti-constipation medicine (sennoside, magnesium oxide, magnesium hydroxide, bisacodyl, polycarbophil calcium, sugar laxatives (lactulose, and the like), laxoberon, crude drugs having anti-constipation effect (psyllium, and the like), and the like).

Combination use with a therapeutic drug for constipation may be useful in treating continence of feces and the like that can be caused by administration of the therapeutic drug for constipation in order to improve constipation. For example, the present medicament, and a combination (or combination use) of the present medicament and a therapeutic drug for constipation can be used to treat neuropathic constipation in a subject having neuropathic constipation.

(11) Others

Glycation inhibitors (e.g., ALT-711), nerve regeneration promoting agents (e.g., Y-128, VX853, and prosaptide), central nervous system agents (e.g., antidepressants such as desipramine, amitriptyline, imipramine, fluoxetine, paroxetine, and doxepin), antiepileptic agents (e.g., lamotrigine and carbamazepine), anti-arrhythmic agents (e.g., mexiletine), acetylcholine receptor ligands (e.g., ABT-594), endothelin receptor antagonists (e.g., ABT-627), monoamine uptake inhibitors (e.g., tramadol), indoleamine uptake inhibitors (e.g., fluoxetine and paroxetine), narcotic analgesics (e.g., morphine), GABA receptor agonists (e.g., gabapentin), GABA uptake inhibitors (e.g., tiagabine), $\alpha_2$ receptor agonists (e.g., clonidine), local analgesics (e.g., capsaicin), protein kinase C inhibitors (e.g., LY-333531), anti-anxiety agents (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate and sumatriptan), serotonin receptor antagonists (e.g., cyproheptadine hydrochloride and ondansetron), serotonin uptake inhibitors (e.g., fluvoxamine maleate, fluoxetine, and paroxetine), sleep inducing agents (e.g., triazolam and zolpidem), anticholinergic agents (e.g., atropine, scopolamine, homatropine, tropicamide, cyclopentolate, butylscopamine bromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, and chloride trospium, or their salts thereof (e.g., atropine sulfate, scopolamine hydrobromide, homatropine hydrobromide, cyclopentolate hydrochloride, flavoxate hydrochloride, pirenzepine hydrochloride, trihexyphenidyl hydrochloride, oxybutynin chloride, and tolterodine tartrate)), acetylcholine esterase inhibitors (e.g., distigmine), $\alpha_1$ receptor blockers (e.g., tamsulosin), muscle relaxants (e.g., baclofen), $K^+$ channel openers (e.g., nicorandil), calcium channel blockers (e.g., nifedipine), preventive or therapeutic drugs for Alzheimer's disease (e.g., donepezil, rivastigmine, and galanthamine), therapeutic drugs for Parkinson's disease (e.g., L-dopa), preventive or therapeutic drugs for multiple sclerosis (e.g., interferon β-1a), histamine $H_1$ receptor inhibitors (e.g., promethazine hydrochloride), proton pump inhibitors (e.g., lansoprazole and omeprazole), antithrombotic agents (e.g., aspirin and cilostazol), NK-2 receptor antagonists, therapeutic drugs for HIV infection (saquinavir, zidovudine, lamivudine, and nevirapine), therapeutic drugs for chronic obstructive pulmonary disease (salmeterol, tiotropium bromide, and cilomilast), etc.

The medicament in which the 5-HT$_{2C}$ receptor agonist of the present invention and the concomitant drug may be used as a mixture or in combination includes all of a medicament obtained as a single formulation containing the 5-HT$_{2C}$ receptor agonist and the concomitant drug, and separate formulations of the 5-HT$_{2C}$ receptor agonist and the concomitant drug. Hereinafter, these formulations are collectively referred to as the combined use agents of the present invention.

The combined use agents of the present invention may be prepared by separately or simultaneously formulating the 5-HT$_{2C}$ receptor agonist and the concomitant drug, either directly or after mixing with a pharmaceutically acceptable carrier, etc., in the same way as in the aforementioned medicament comprising the 5-HT$_{2C}$ receptor agonist of the present invention. The daily dose of the combined use agents of the present invention differs depending on severity, the age, sex, or body weight of a recipient subject, difference in sensitivity, the time of administration, dosing intervals, the properties, prescription, or type of the medicament, the type of the active ingredient, etc., and is not particularly limited.

For the administration of the combined use agents of the present invention, the 5-HT$_{2C}$ receptor agonist and the concomitant drug may be administered at the same time. Alternatively, the concomitant drug may be first administered, followed by the administration of the 5-HT$_{2C}$ receptor agonist, or the 5-HT$_{2C}$ receptor agonist may be first administered, followed by the administration of the concomitant drug. In the case of administering the agonist and the concomitant drug in the separate manner, the time interval of the administrations differs depending on the active ingredient to be administered, the dosage form, and the administration method. Examples of the method for first administering the concomitant drug may include a method which involves administering the concomitant drug and administering the 5-HT$_{2C}$ receptor agonist within 1 minute to 3 days, preferably within 10 minutes to 1 day, more preferably within 15 minutes to 1 hour thereafter. Examples of the method for first administering the 5-HT$_{2C}$ receptor agonist may include a method which involves administering the 5-HT$_{2C}$ receptor agonist and administering the concomitant drug within 1 minute to 1 day, preferably within 10 minutes to 6 hours, more preferably within 15 minutes to 1 hour thereafter.

The respective amounts of the 5-HT$_{2C}$ receptor agonist and the concomitant drug in the combined use agents of the present invention containing the 5-HT$_{2C}$ receptor agonist and the concomitant drug in a single formulation differ depending on the form of the formulation and may usually be approximately 0.01 to 90% by weight, preferably approximately 0.1 to 50% by weight, more preferably approximately 0.5 to 20% by weight, with respect to the total weight of the preparation.

The content of the carrier in the combined use agents may usually be approximately 0 to 99.8% by weight, preferably approximately 10 to 99.8% by weight, more preferably approximately 10 to 90% by weight, with respect to the total weight of the preparation.

When the combined use agents of the present invention comprise separate medicaments respectively containing the 5-HT$_{2C}$ receptor agonist and the concomitant drug, the medicament containing the concomitant drug may be produced in the same way as in the medicament comprising the 5-HT$_{2C}$ receptor agonist of the present invention.

The medicament of the present invention may be any of solid formulations including powders, granules, tablets, and capsules, and liquid formulations including syrups and emulsions.

The medicament of the present invention may be produced by a routine method, for example, mixing, kneading, granulation, tableting, coating, sterilization, and/or emulsification, according to the form of the formulation. For such pharmaceutical production, see, for example, each section of "General Rules for Preparations" in the Japanese Pharmacopoeia. The medicament of the present invention may be prepared into a sustained-release agent containing the active ingredient and a biodegradable polymer compound. The sustained-release agent can be prepared according to a method described in Japanese Patent Laid-Open Publication No. H09-263545.

The medicament of the present invention may comprise, for example, one or more ingredients selected from microcrystalline cellulose, mannitol, and magnesium stearate.

The medicament of the present invention may comprise, for example, (hydroxypropyl)methyl cellulose.

In an embodiment, the medicament of the present invention may comprise (hydroxypropyl)methyl cellulose and one or more ingredients selected from: microcrystalline cellulose, mannitol, and magnesium stearate.

The medicament of the present invention may comprise a film coating. For example, the medicament of the present invention may comprise a water-soluble film coating. For example, the medicament of the present invention may comprise a film coating comprising ethyl cellulose. For example, the medicament of the present invention may comprise a film coating comprising ethyl cellulose and (hydroxypropyl)methyl cellulose. In this specific embodiment, in the medicament of the present invention, the ratio of the ethyl cellulose to the (hydroxypropyl)methyl cellulose is about 75:25; about 80:20; or about 85: 15.

In an embodiment, the medicament of the present invention may comprise (*R*)-8-chloro-1-methyl-2,3,4,5-tetrahydro-1H-3-benzazepine or a salt thereof and one or more ingredients selected from microcrystalline cellulose, mannitol, and magnesium stearate. In this specific embodiment, the medicament of the present invention may comprise a film coating or a water-soluble film coating.

In this specific embodiment, the medicament of the present invention may comprise a film coating or a water-soluble film coating.

In an embodiment, the medicament of the present invention may comprise (*R*)-8-chloro-1-methyl-2,3,4,5-tetrahydro-1H-3-benzazepine or a salt thereof; one or more ingredients selected from microcrystalline cellulose, mannitol, and magnesium stearate; and (hydroxypropyl)methyl cellulose. In this specific embodiment, the medicament of the present invention may comprise a film coating or a water-soluble film coating.

In an embodiment, the medicament of the present invention may comprise (*R*)-8-chloro-1-methyl-2,3,4,5-tetrahydro-1H-3-benzazepine or a salt thereof; mannitol; (hydroxypropyl)methyl cellulose; microcrystalline cellulose; and magnesium stearate, and preferably comprise a water-soluble film coating.

In an embodiment, the medicament of the present invention may comprise (*R*)-8-chloro-1-methyl-2,3,4,5-tetrahydro-1H-3-benzazepine or a salt thereof; mannitol; (hydroxypropyl)methyl cellulose; microcrystalline cellulose; and magnesium stearate, and preferably may further comprise a film coating preferably comprise ethyl cellulose and (hydroxypropyl)methyl cellulose, wherein the ratio of the ethyl cellulose to the (hydroxypropyl)methyl cellulose may optionally be the ratio as described above.

In an embodiment, the medicament of the present invention may comprise a core tablet and a film coating, wherein the core tablet comprises: (*R*)-8-chloro-1-methyl-2,3,4,5-tetrahydro-1H-3-benzazepine hydrochloride salt hemihydrate, Form III; mannitol; (hydroxypropyl)methyl cellulose; microcrystalline cellulose; and magnesium sterate; and the film coating may further comprise a water-soluble film coating.

In an embodiment, the medicament of the present invention may comprise a core tablet and a film coating, wherein the weight to weight ratio of the core tablet to the coating is about 20: 1 ; and wherein the core tablet comprises: about 7% (R)-8-chloro-1-methyl-2,3,4,5-tetrahydro-1H-3-benzazepine hydrochloride salt hemihydrate, Form III; about 22.5% mannitol; about 50% (hydroxypropyl)methyl cellulose; about 20% microcrystalline cellulose; and about 0.5% magnesium sterate, wherein the film coating may further comprise ethyl cellulose and (hydroxypropyl)methyl cellulose.

In an embodiment, the medicament of the present invention may comprise a core tablet and a film coating, wherein the core tablet comprises: (*R*)-8-chloro-1-methyl-2,3,4,5-tetrahydro-1H-3-benzazepine hydrochloride salt hemihydrate, Form III; mannitol; (hydroxypropyl)methyl cellulose; microcrystalline cellulose; and magnesium sterate; and the film coating may further comprise ethyl cellulose and (hydroxypropyl)methyl cellulose.

In an embodiment, the medicament of the present invention may comprise a core tablet and a film coating, wherein the weight to weight ratio of the core tablet to the coating is about 20:1; and wherein the core tablet comprises about 7% (R)-8-chloro-1-methyl-2,3,4,5-tetrahydro-1H-3-benzazepine hydrochloride salt hemihydrate, Form III; about 22.5% mannitol; about 50% (hydroxypropyl)methyl cellulose; about 20% microcrystalline cellulose; and about 0.5% magnesium sterate; and the film coating may comprise about 85% ethyl cellulose and about 15% (hydroxypropyl)methyl cellulose; or about 75% ethyl cellulose and about 25% (hydroxypropyl)methyl cellulose.

In an embodiment, the medicament of the present invention may be a releasing dosage whose T80% is at least 3 hours; at least 6 hours; at least 9 hours; or at least 12 hours. The term "T80%" refers to the time needed to achieve 80% cumulative release of an active pharmaceutical ingredient. Those skilled in the art will control the releasing property.

In an embodiment, the medicament of the present invention may be a solid dosage form (for example, a tablet). The medicament of the present invention may be a core tablet.

The core tablet of the present invention comprises a core, an outer shell, a hydrophilic gel-forming polymer in the core and the outer shell, and the active ingredient in the core and/or the outer shell, which may control the release behavior of the active ingredient by adjusting the content of the active ingredient in the core and/or the outer shell, thereby releasing the active ingredient in a sustained manner.

In the core table of the present invention, the release rate of the active ingredient is, for example, 0 to 30% after 2 hours, 15 to 55% after 6 hours, 35 to 80% after 9 hours, and preferably, 5 to 25% after 2 hours, 20 to 50% after 6 hours, 40 to 75% after 9 hours, and more preferably, 20 to 35% after 2 hours, 50 to 70% after 6 hours, 70 to 95% after 9 hours when the rate is measured by a paddle method according to the Japanese Pharmacopoeia 16th edition (the second solution in the releasing test in the Japanese Pharmacopoeia).

In another embodiment, the rate is usually 10 to 45 % after 2 hours, 40 to 80% after 6 hours, 60 to 100% after 9 hours, preferably 15 to 40% after 2 hours, 45 to 75% after 6 hours, 65 to 95% after 9 hours, more preferably 20 to 35% after 2 hours, 50 to 70% after 6 hours, 70 to 95% after 9 hours.

The solid formulation of the present invention may comprise a hydrophilic gel-forming polymer.

When the solid formulation of the present invention comprises a hydrophilic gel-forming polymer, the viscosity of the hydrophilic gel-forming polymer (at 25 °C in 1% aqueous solution) is, for example, 100 cP or more, preferably 5500 cP or more, more preferably 7500 cP or more. In the present invention, the viscosity of the hydrophilic gel-forming polymer (at 25 °C in 1% aqueous solution) is usually 500000 cp or less.

Hydrophilic gel-forming polymers which can be used in the present invention include, for example, but not limited to, polyethyleneoxide (for example, Polyox™ WSR303 (The Dow Chemical Company), PEO-20NF (Sumitomo Seika Chemicals Company Limited), hydroxypropylmethylcellulose (for example, METOLOSE 90SH-100000SR (Shin-Etsu Chemical Co., Ltd.), carboxymethylcellulose, hydroxypropylcellulose, hydroxyehylcellulose, carboxyvinylpolymer (for example, Carbopol971PNF (The Lubrizol Corporation)), methylcellulose, sodium carboxymethylcellulose, and preferably, polyethyleneoxide, hydroxypropylmethylcellulose, carboxyvinylpolymer, and particularly preferably, polyethyleneoxide.

In the solid formulation of the present invention, as a hydrophilic gel-forming polymer, a hydrophilic gel-forming polymer with a high viscosity (for example, polyethyleneoxide) is preferably used in that this can control the release of *N*-methyl-*N*-(1-methylethyl)-6,7,8,9-tetrahydropyrazino[2,3-*f*][1,4]oxazepine-3-amine or a salt thereof, which has a high solubility.

In the solid formulation of the present invention, the amount of a hydrophilic gel-forming polymer is usually 15 to 95%, preferably 25 to 85%, more preferably 35 to 75% by weight to the weight of uncoated formulation (uncoated tablet).

An uncoated formulation (an uncoated tablet) as used herein refers to a formulation (a tablet) before the coating in case where the solid formulation has been coated as mentioned below, and to a solid formulation (a tablet) itself in case where the solid formulation has not been coated.

The present medicament in a form of solid formulation includes, for example, (A), (B), and (C) below:

(A) a solid formulation (a tablet) with a single layer comprising *N*-(1-methylethyl)-6,7,8,9-tetrahydropyrazino[2,3-*f*][1,4]oxazepine-3-amine or a salt thereof and a hydrophilic gel-forming polymer (hereinafter also referred to as the present single layer tablet);

(B)

(1) a solid formulation (for example, a tablet) comprising a core comprising N-(1-methylethyl)-6,7,8,9-tetrahydropyrazino[2,3*f*][1,4]oxazepine-3-amine or a salt thereof and a first hydrophilic gel-forming polymer; and
(2) a solid formulation (for example, a tablet) comprising an outer shell comprising *N*-(1-methylethyl)-6,7,8,9-tetrahydropyrazino[2,3-*f*][1,4]oxazepine-3-amine or a salt thereof and a second hydrophilic gel-forming polymer.

(C) (1) a solid formulation (for example, a tablet) comprising a core comprising N-(1-methylethyl)-6,7,8,9-tetrahydropyrazino[2,3f][1,4]oxazepine-3-amine or a salt thereof and a first hydrophilic gel-forming polymer, and (2) an outer shell comprising a second hydrophilic gel-forming polymer, without comprising *N*-(1-methylethyl)-6,7,8,9-tetrahydropyrazino[2,3-*f*][1,4]oxazepine-3-amine or a salt thereof (hereinafter (B) and (C) above are also referred to as the present core tablet).

In the present single layer tablet, the amounts of *N*-(1-methylethyl)-6,7,8,9-tetrahydropyrazino[2,3-*f*][1,4]oxazepine-3-amine or a salt thereof and the hydrophilic gel-forming polymer are as described above.

The present single layer tablet may have a film coating as explained below.

The amount of the hydrophilic gel-forming polymer indicates the amount in the uncoated tablet before the film coating in case where the present single layer tablet has been film coated.

In the present invention, an outer shell of a hydrophilic gel-forming polymer without comprising *N*-(1-methylethyl)-6,7,8,9-tetrahydropyrazino[2,3-*f*][1,4]oxazepine-3-amine or a salt thereof can be formed outside the single layer tablet. The embodiment will be explained as a core tablet whose outer shell does not comprise *N*-(1-methylethyl)-6,7,8,9-tetrahydropyrazino[2,3f][1,4]oxazepine-3-amine or a salt thereof.

In the present core tablet, the core may be a tablet (a core which is a tablet is described as an inner core tablet in the specification).

In the present core tablet, the amount of N-(1-methylethyl)-6,7,8,9-tetrahydropyrazino[2,3-f][1,4]oxazepine-3-amine or a salt thereof in the core is usually 0.5 to 100 mg, preferably 2.5 to 800 mg, more preferably 5 to 500 mg as a free form.

In the present core tablet, the amount of N-(1-methylethyl)-6,7,8,9-tetrahydropyrazino[2,3-f][1,4]oxazepine-3-amine or a salt thereof in the outer shell is usually 0 to 500 mg, preferably 0 to 400 mg, more preferably 0 to 250 mg as a free form.

The present core tablet may comprise N-(1-methylethyl)-6,7,8,9-tetrahydropyrazino[2,3-f][1,4]oxazepine-3-amine or a salt thereof in both of the core and the outer shell, or only in the core.

For example, the present solid formulation encompasses a core tablet comprising N-(1-methylethyl)-6,7,8,9-tetrahydropyrazino[2,3f][1,4]oxazepine-3-amine or a salt thereof in the core without comprising N-(1-methylethyl)-6,7,8,9-tetrahydropyrazino[2,3-f][1,4]oxazepine-3-amine or a salt thereof in the outer shell.

In view of the expectation of more delayed, longer release of the active ingredient, in the present core tablet, the amount (weight) of N(1-methylethyl)-6,7,8,9-tetrahydropyrazino[2,3-f][1,4]oxazepine-3-amine or a salt thereof in the core is preferably equal to or more than that in the outer shell.

In the present core tablet, a first and second hydrophilic gel-forming polymers include the hydrophilic gel-forming polymers as described in the present solid formulation.

The first hydrophilic gel-forming polymer may be the same as or different from the second gel-forming polymer.

In the present core tablet, both of the first and second hydrophilic gel-forming polymers are preferably polyethyleneoxide.

In the present core tablet, the amount of the first hydrophilic gel-forming polymer in the core is usually 0.01 to 90%, preferably 5 to 70%, more preferably 10 to 50% by weight to the weight of the core.

In the present core tablet, the amount of the second hydrophilic gel-forming polymer in the outer shell is usually 20 to 100%, preferably 30 to 95%, more preferably 40 to 90% by weight to the weight of the outer shell.

The present core tablet may have been film coated as described below.

The aforementioned amount of the hydrophilic gel-forming polymer refers to that in the formulation before film coating (the uncoated tablet) in case where the core tablet has been film coated.

In the present core tablet, the weight ratio of the core and the outer shell is usually 1:1.5 to 3.5, preferably 1:1.5 to 3.0, more preferably 1:2 to 2.5, still more preferably about 1:2.

The present solid formulation (the single layer tablet, the core tablet) may further comprise additives commonly used in the field of pharmaceutical formulation.

The additives include, for example, excipients (eg, mannitol, spray-dried mannitol, starch, lactose, white sugar, calcium carbonate, calcium phosphate, crystalline cellulose,binders (eg, starch, gum arabic, alginic acid, gelatin, polyvinyl pyrrolidone, hydroxypropyl cellulose (provided that those having a low viscosity not functioning as a hydrophilic gel-forming polymer, such as HPC-SL, HPC-L (Nippon Soda Co., LTD.))), and lubricants (eg, stearic acid, magnesium stearate, calcium stearate, talc).

In the present invention, additives other than a hydrophilic gel-forming polymer may be minimized by using a hydrophilic gel-forming polymer.

In the present solid formulation, the total amount of the additives other than a hydrophilic gel-forming polymer are usually 5 to 95%, preferably 15 to 75%, more preferably 25 to 65% by weight to the weight of the uncoated tablet.

The present solid formulation (a single layer tablet, a core tablet) can be manufactured by a conventional method in the field of pharmaceutical formulation, using ingredients as described above.

The present single layer tablet can be manufactured, for example, by mixing N-(1-methylethyl)-6,7,8,9-tetrahydropyrazino[2,3-f][1,4]oxazepine-3-amine or a salt thereof, a hydrophilic gel-forming polymer (eg, polyethyleneoxide, hydroxypropylmethylcellulose, carboxyvinylpolymer), optional additives (eg, an excipient (eg, mannitol), a lubricant (eg, magnesium stearate), binder), and being subjected to compressed molding (tablet making).

The present core tablet can be produced, for example, as described below.

The core (the inner core tablet) is manufactured by mixing N(1-methylethyl)-6,7,8,9-tetrahydropyrazino[2,3-f][1,4]oxazepine-3-amine or a salt thereof, a hydrophilic gel-forming polymer (eg, polyethyleneoxide, hydroxypropylmethylcellulose, carboxyvinylpolymer), optional additives (eg, an excipient (eg, mannitol), a lubricant (eg, magnesium stearate), binder), and being subjected to compressed molding.

Separately, N(1-methylethyl)-6,7,8,9-tetrahydropyrazino[2,3-f][1,4]oxazepine-3-amine or a salt thereof, a hydrophilic gel-forming polymer (eg, polyethyleneoxide, hydroxypropylmethylcellulose, carboxyvinylpolymer), optional additives (eg, an excipient (eg, mannitol), a lubricant (eg, magnesium stearate), binder) can be mixed to obtain a powder mixture of the outer shell (i.e., powder mixture of the ingredients forming the outer shell).

The inner core tablet and the powder mixture of the outer shell can be subjected to compressed molding to obtain a core tablet.

The core tablet which does not comprise *N*-(1-methylethyl)-6,7,8,9-tetrahydropyrazino[2,3-*f*][1,4]oxazepine-3-amine or a salt thereof in the outer shell can be manufactured by the above-described method wherein *N*(1-methylethyl)-6,7,8,9-tetrahydropyrazino[2,3-*f*][1,4]oxazepine-3-amine or a salt thereof is not added to the powder mixture of the outer shell.

The mixing can be performed, for example, with a mixing machine such as a V-shaped mixing machine, tumbler mixing machine. The compressed molding can be performed by using a single-punch tableting machine, rotary tableting machine.

The present solid formulation (a single layer tablet, a core tablet) may be coated by a conventional method in the field of pharmaceutical formulation, if needed. For example, film coating base, additive for coating can be used.

Film coating bases include, for example, hydroxypropylmethylcellulose (TC-5E, TC-5R (Shin-Etsu Chemical Co., LTD.)), hydroxypropyl cellulose, polyvinyl pyrrolidone, polyvinyl alcohol, methyl cellulose, hydroxyethyl methyl cellulose.

Additives for coating include, for example, a light shielding agent such as titanium oxide; a fluidizing agent such as talc and sterilized talc; a coloring agent such as iron sesquioxide and yellow ferric oxide; plasticizers such as polyethylene glycols (eg, Macrogol 6000), triethyl citrate, castor oil and polysorbates; and organic acids such as citric acid, tartaric acid, malic acid, ascorbic acid and the like.

The amount of the film coating is usually 1% to 8%, preferably 2% to 6% to the weight of the uncoated formulation (uncoated tablet) in case where the present solid formulation is film coated.

The present solid formulation is usually 100 mg to 1500 mg, preferably 200 mg to 1000 mg in weight.

The present singly layer tablet is usually 6 mm to 20 mm, preferably 8 mm to 15 mm in size.

The present core tablet is usually 4.5 mm to 17 mm, preferably 5 mm to 12 mm in size, and the core (inner core tablet) in the core tablet is usually 7.5 mm to 20 mm, preferably 8 mm to 15 mm in size.

The shape of the present solid formulation is not particularly limited, and may be any of a round shape a caplet shape, a donut shape, an oblong shape, and the like.

(*R*)-8-chloro-1-methyl-2,3,4,5-tetrahydro-1H-3-benzazepine hydrochloride salt hemihydrate, Form III (as described in WO2012/030927), which can be used as an active ingredient of the present medicament.

The present medicament is, for example, an immediate release formulation and, comprises, for example, a part or all of ingredients selected from a group consisting of silicified microcrystalline cellulose, hydroxypropylcellulose F, croscarmellose sodium, polyvinyl alcohol, polyethylene glycol, titanium dioxide, talc, FD & C blue # 2 / indigo carmine aluminum lake, and magnesium steate. The present medicament is, for example, a sustained-release preparation, and comprises, for example, , a part or all of ingredients selected from a group consisting of microcrystalline cellulose, mannitol, hypromellose, ethylcellulose dispersant Type B, silicon dioxide colloid, polyvinyl alcohol, polyethylene glycol, titanium dioxide, talc, FD & C yellow # 6 / sunset yellow FCF aluminum lake, iron oxide yellow, iron oxide red, and magnesium stearate.

In a further aspect, the present invention provides a method for treating incontinence of feces in a subject in need thereof, comprising administering a 5-HT$_{2C}$ receptor agonist at a dosage lower than the minimum dosage of the agonist as an anti-obesity drug to the subject. The present invention also provides a method for treating incontinence of feces in a subject in need thereof, comprising administering a 5-HT$_{2C}$ receptor agonist at a dosage that shows no body weight lowering effect to the subject.

In another aspect, the present invention provides a method for treating incontinence of feces and obesity in an obese subject (i.e., a subject having a body mass index (BMI) ≥ 25) having incontinence of feces, comprising administering a 5-HT$_{2C}$ receptor agonist at a dosage lower than the minimum dosage of the agonist as an anti-obesity drug or at a dosage that shows no body weight lowering effect to the subject, and administering an additional anti-obesity drug to the subject.

[Examples]

Hereinafter, the present invention will be described in more detail with reference to Examples. However, the present invention is not intended to be limited by these Examples, and various changes or modifications can be made without departing from the scope of the present invention.

Compound A' was prepared in the same way as the method described in Example 8 of WO2010/147226. The synthesis of Compound B' was entrusted to KNC Laboratories Co., Ltd., and a synthetic product was obtained. In Examples below, the dose and concentration of each compound were described as values calculated on the basis of a free form of the compound, unless otherwise specified.

**Example 1: *In Vitro* test of agonistic potency on human 5-HT$_{2C}$ receptor**

**Method**

Each test compound was evaluated for agonistic activity for 5-HT$_{2C}$ receptors on the basis of changes in intracellular Ca$^{2+}$ concentration. CHO-K1 cells expressing recombinant human 5-HT$_{2C}$ receptors were cultured in UltraCHO medium containing 1% dialyzed bovine serum, 400 $\mu$g/mL geneticin, 100 IU penicillin, and 100 $\mu$g/mL strepto-mycin. These cells were seeded into black-walled clear-bottom 384-well plates at a density of 1 $\times$ 10$^4$ cells per well, then treated 10 mmol/L sodium butyrate, and cultured at 37°C for approximately 24 hours under 5% CO$_2$ conditions. A solution containing Fluo-4 AM (Ca$^{2+}$ indicator fluorescent dye) was prepared as an intracellular Ca$^{2+}$ indicator and used as Fluo-4 loading solution. After removal of the culture medium, the cells were loaded with 40 $\mu$L/well of the Fluo-4 loading solution. The plates were left standing at room temperature for approximately 60 minutes so that Fluo-4 AM was incorporated into the cells. The test compound was diluted with an assay buffer. The plates containing the intracellular Ca$^{2+}$ indicator-incorporated cells, and the diluted test compound solution were placed in a fluorometric image analysis plate reader equipped with an automatic dispenser to measure the change in intracellular Ca$^{2+}$ concentration. Fluores-cence intensity was monitored at 2-second intervals. 20 $\mu$L of the test compound solution was added after 10-second baseline value recording to measure the fluorescence intensity at 2-second intervals for 30 seconds. In this context, the activity of Compound A', Compound B', or 5-HT (Wako Pure Chemical industries, Ltd) was measured.

**Results**

5-HT, Compound B', and Compound A' exhibited the agonistic activity on human 5-HT$_{2C}$ receptors with EC$_{50}$ values of 0.12 nmol/L, 0.81 nmol/L, and 2.6 nmol/L, respectively.

**Example 2: Effect of Compound A' on urethral resistance during intravesical pressure rise by electrical stimulation of rat abdominal muscle**

In this Example, the effect of Compound A' was evaluated with duloxetine hydrochloride (hereinafter, referred to as "duloxetine") as a benchmark. The duloxetine is known as a serotonin-noradrenaline reuptake inhibitor and is commercially available as a medicament for use in treating stress urinary incontinence in three European countries.

**Method**

Fifty six female rats of Sprague-Dawley strain (hereinafter, referred to as "SD strain") were used. The rats were anesthetized with urethane, and their spinal cord was transected at the T8-T9 (the eighth thoracic cord - the ninth thoracic cord) level for the elimination of reflex voiding. Isoflurane inhalation was added, if necessary. After opening of the abdomen, a catheter (PE-100) was inserted into the bladder. In order to secure reduced urethral resistance, unilateral nerve to iliococcygeus and pubococcygeus muscles was transected. For intraduodenal administration, another catheter (PE-50) was inserted into the duodenum in advance. The abdominal muscle and skin were each closed with sutures. Two (right and left) sites of abdominal skin near the diaphragm were cut to expose abdominal muscle. The bladder was emptied through the catheter, followed by the injection of 0.2 to 0.3 mL of 0.1% Evans blue solution. Change in intravesical pressure was recorded using the bladder catheter via a pressure transducer, an electric signal amplifier, and an analog-to-digital converter. While change in intravesical pressure was recorded, the presence or absence of fluid leakage from the urethral orifice was observed under the electrical stimulation of the exposed abdominal muscle using an electrical stimulator and an isolator. During the electrical stimulation, the stimulus intensity was gradually increased in the range from 1 to 10 V under conditions involving a pulse width of 0.5 ms, 50 Hz, and 1 second to increase the intravesical pressure step by step. The peak of the intravesical pressure during the stimulation of the abdominal muscle was measured, and the lowest pressure at which the fluid leakage was observed among the trials was defined as leak point pressure (hereinafter, referred to as "LPP") for the electrical abdominal muscle stimulation method and used as an index for urethral resistance. Each drug was evaluated for its effect by comparing LPP before drug administration with that after drug administration. The drug or a vehicle was intraduodenally administered to selected rats whose LPP was greater than 20 cm H$_2$O and lower than 70 cm H$_2$O. The drugs were suspended in 0.5% methylcellulose solution (MC). Compound A' at 0.3, 1 and 3 mg/mL/kg, and duloxetine at 5, 10 and 20 mg/mL/kg were administered. LPP was measured again 30 minutes after administration. Regarding change in LPP before and after drug administration, the differences between the mean values of the drug-treated and vehicle groups were analyzed by the one-tailed Williams' test.

### Results

Compound A' and duloxetine were each intraduodenally administered, and LPP was measured before administration and 30 minutes after administration and compared. As a result, 0.5% MC exhibited no effect, whereas both the drugs increased LPP in a dose-dependent manner (see Table 2). These results indicated that Compound A' increases urethral resistance during the compression of the bladder.

[Table 2]

Table 2: Effects of compound A' and duloxetine on urethral resistance during momentary intravesical pressure rise by electrical stimulation of rat abdominal muscles

| Drug | Dose (mg/kg. i.d.) | Pre | LPP ($cmH_2O$) Post | Increase |
|---|---|---|---|---|
| Experiment 1 | | | | |
| Vehicle | - | $46.1 \pm 6.4$ | $45.4 \pm 8.1$ | $-0.7 \pm 3.7$ |
| Compound A' | 0.3 | $47.2 \pm 5.7$ | $55.0 \pm 5.4$ | $7.8 \pm 3.2$ |
| | 1 | $47.3 \pm 5.0$ | $61.5 \pm 4.9$ | $14.2 \pm 2.7$* |
| | 3 | $47.6 \pm 3.1$ | $77.3 \pm 8.1$ | $29.7 \pm 5.5$* |
| | | | | |
| Experiment 2 | | | | |
| Vehicle | - | $35.6 \pm 4.3$ | $33.9 \pm 4.3$ | $-1.6 \pm 1.5$ |
| Duloxetine | 5 | $36.6 \pm 3.0$ | $38.3 \pm 2.7$ | $1.7 \pm 1.4$ |
| | 10 | $35.3 \pm 3.0$ | $39.1 \pm 2.9$ | $3.7 \pm 2.3$ |
| | 20 | $36.9 \pm 2.8$ | $46.6 \pm 5.7$ | $9.7 \pm 3.5$* |

Data are expressed as mean $\pm$ SEM from 6 rats at each dosage for experiment 1 and from 8 rats at each dosage for experiment 2.

The differences of LPP increase in the vehicle- and drug-treated groups were analyzed by the one-tailed Williams' test. *: $p \leq 0.025$ vs. the vehicle-treated group.

### Example 3: Influence of antagonist on urethral resistance increasing effect of Compound A' in rats

In this Example, a 5-$HT_{2C}$ receptor antagonist SB 242084 (Tocris Bioscience, Batch No. 3A/101389) and a 5-$HT_2$ receptor antagonist methiothepin maleate (hereinafter, referred to as "methiothepin"; Tocris Bioscience, Batch No. 6*A/103646) were used to block the urethral resistance increasing effect of Compound A', and also to reveal that Compound A' works in the spinal cord to show urethral resistance increasing effects.

### Method

Ninety female rats of SD strain were used. The rats were anesthetized with urethane, and their spinal cord was transected at the T8-T9 (the eighth thoracic cord - the ninth thoracic cord) level for the elimination of reflex voiding. Isoflurane inhalation was added during the surgery, if necessary. In the experiment with intrathecal administration, the dura mater was exposed by laminectomy and then partially opened by incision, through which a catheter (PE-10) filled with saline was then implanted at the level of the L6-S1 site, followed by the closing of the incision. After opening of the abdomen, a catheter (PE-90) was inserted into the bladder. In order to secure reduced urethral resistance, unilateral nerve to iliococcygeus and pubococcygeus muscles was transected. For intraduodenal administration, another catheter (PE-50) was inserted into the duodenum in advance. The abdominal muscle and skin were each closed with sutures. Two (right and left) sites of abdominal skin near the diaphragm were cut to expose abdominal muscle. The bladder was emptied through the catheter, followed by the injection of 0.2 to 0.3 mL of Evans blue solution. Change in intravesical pressure was recorded using the bladder catheter via a pressure transducer, an electric signal amplifier, and an analog-to-digital converter. While change in intravesical pressure was recorded, the presence or absence of fluid leakage from the urethral orifice was observed under the electrical stimulation of the exposed abdominal muscle using an electrical stimulator and an isolator. During the electrical stimulation, the stimulus intensity was gradually increased in the range from 1 to 10 V under conditions involving a pulse width of 0.5 ms, 50 Hz, and 1 second to increase the intravesical pressure step by step. The peak of the intravesical pressure during the stimulation of the abdominal muscle was measured, and the lowest pressure at which the fluid leakage was observed among the trials was defined as LPP for the electrical abdominal muscle stimulation method. In order to examine whether the urethral resistance increasing effect of Compound A' is based on its agonistic potency on 5-HT2C receptors, SB 242084, methiothepin, or a vehicle was intravenously administered, and Compound A' or a vehicle was intraduodenally administered 5 minutes later. SB 242084 was dissolved in a mixed solution of N,N-dimethylacetamide and polyethylene glycol 400 (1:1) and administered at 0.03 mg/0.5 mL/kg, 0.1 mg /0.5 mL/kg and 0.3 mg/0.5 mL/kg. Methiothepin was dissolved in saline and administered at 0.03 mg /0.5 mL/kg. 0.1 mg /0.5 mL/kg and 0.3 mg/0.5 mL/kg. Compound A' was suspended in 0.5% MC and administered at 3 mg/mL/kg. LPP was measured again 30 minutes after Compound A' administration. In order to examine where Compound A' works in the body to increase urethral resistance, the blocking of the effect of Compound A' was confirmed by blocking the functions of Compound A' by giving methiothepin at a particular site in the body. Particularly, in the experiment with intrathecal administration, methiothepin or a vehicle was intrathecally administered, and Compound A' or a vehicle was intraduodenally administered 5 minutes later. Methiothepin was dissolved in saline and 20 $\mu$L of the obtained solution was administered. Compound A' was suspended in 0.5% MC and administered at 3 mg/mL/kg. LPP was measured again 30 minutes after Compound A' administration.

Regarding change in LPP before and after drug administration, the differences between the mean values of the Compound A'-treated and vehicle-treated groups were analyzed by the Student's t test or the Welch's test. In order to analyze the influence of the antagonists on the effect of Compound A', the differences between the mean values of the SB 242084- or methiothepin- and Compound A'-administered group and the vehicle- and Compound A'- administered group were analyzed by the one-tailed Williams' test.

**Results**

The intravenous administration of the selective 5-HT$_{2C}$ receptor antagonist SB 242084 dose-dependently blocked the LPP increasing effect of Compound A' (see Table 3). The high-dose administration of SB 242084 almost completely blocked the effect of Compound A'. Similar study was conducted using the 5-HT$_2$ receptor antagonist methiothepin. As a result, methiothepin dose-dependently blocked the LPP increasing effect of Compound A', and the effect of Compound A' disappeared by the high-dose administration of methiothepin (see Table 4). Further, the intrathecal administration of methiothepin dose-dependently blocked the LPP increasing effect of Compound A' (see Table 5).

The urethral resistance increasing effect of Compound A' was significantly antagonized by the selective 5-HT$_{2C}$ receptor antagonist SB 242084 and therefore found to be an effect mediated by the stimulation of 5-HT$_{2C}$ receptors. This effect was also significantly antagonized by the intrathecally administered 5-HT$_2$ receptor antagonist methiothepin, suggesting that the urethral resistance increasing effect of Compound A' works in the spinal cord.

[Table 3]

Table 3: Blocking effect of intravenously administered SB 242084 on urethral resistance increasing effect of compound A' during electrical stimulation of rat abdominal muscles

| SB 242084 (mg/kg. i.v.) | Compound A' (mg/kg, i.d.) | Pre | LPP (cmH$_2$O) Post | Increase |
|---|---|---|---|---|
| 0 | 0 | 46.1 ± 5.2 | 48.3 ± 8.0 | 2.3 ± 3.8 |
| 0.3 | 0 | 45.9 ± 5.4 | 49.3 ± 8.7 | 3.4 ± 4.6 |
| 0 | 3 | 46.0 ± 5.1 | 72.4 ± 9.5 | 26.4 ± 5.9## |
| 0.03 | 3 | 44.0 ± 7.4 | 73.9 ± 8.7 | 29.9 ± 8.4 |
| 0.1 | 3 | 45.0 ± 6.7 | 57.6 ± 12.2 | 12.6 ± 6.3 |
| 0.3 | 3 | 44.3 ± 7.0 | 48.0 ± 6.9 | 3.8 ± 4.4 |

Data are expressed as mean ± SEM from 5 rats.
## P ≤ 0.01: compared with the vehicle intravenously and intraduodenally administered group using the Student's *t*-test
\* P ≤ 0.025: compared with the vehicle intravenously and compound A' intraduodenally administered group using the one-tailed Williams' test

[Table 4]

Table 4: Blocking effect of intravenously administered methiothepin on urethral resistance increasing effect of compound A' during electrical stimulation of rat abdominal muscles

| MethiotheDin (mg/kg, i.v.) | Compound A' (mg/kg, i.d.) | Pre | LPP (cmH$_2$O) Post | Increase |
|---|---|---|---|---|
| 0 | 0 | 47.2 $\pm$ 3.6 | 49.3 $\pm$ 2.6 | 2.0 $\pm$ 2.9 |
| 0.3 | 0 | 46.0 $\pm$ 9.6 | 44.0 $\pm$ 7.1 | -2.0 $\pm$ 2.5 |
| 0 | 3 | 45.9 $\pm$ 4.4 | 61.0 $\pm$ 8.3 | 15.1 $\pm$ 4.0# |
| 0.03 | 3 | 44.4 $\pm$ 2.5 | 62.3 $\pm$ 4.4 | 17.9 $\pm$ 4.3 |
| 0.1 | 3 | 50.4 $\pm$ 4.4 | 59.9 $\pm$ 3.7 | 9.5 $\pm$ 3.3 |
| 0.3 | 3 | 42.9 $\pm$ 3.8 | 40.6 $\pm$ 4.9 | -2.2 $\pm$ 2.9* |

Data are expressed as mean $\pm$ SEM from 5 rats.
\# P $\leq$ 0.05: compared with the vehicle intravenously and intraduodenally administered group using the Student's *t*-test
\* P $\leq$ 0.025: compared with the vehicle intravenously and compound A' intraduodenally administered group using the one-tailed Williams' test

[Table 5]

Table 5: Blocking effect of intrathecally administered methiothepin on urethral resistance increasing effect of compound A' during electrical stimulation of rat abdominal muscles

| MethiotheDin ($\mu$g i.t.) | Compound A' (mg/kg, i.d.) | Pre | LPP (cmH$_2$O) Post | Increase |
|---|---|---|---|---|
| 0 | 0 | 43.6 $\pm$ 7.0 | 46.6 $\pm$ 6.9 | 3.0 $\pm$ 2.1 |
| 100 | 0 | 45.8 $\pm$ 4.8 | 40.5 $\pm$ 3.2 | -5.3 $\pm$ 3.0 |
| 0 | 3 | 40.3 $\pm$ 5.6 | 72.1 $\pm$ 5.7 | 31.8 $\pm$ 6.2## |
| 10 | 3 | 34.1 $\pm$ 3.2 | 69.4 $\pm$ 10.4 | 35.3 $\pm$ 9.8 |
| 30 | 3 | 41.0 $\pm$ 3.6 | 52.1 $\pm$ 8.6 | 11.1 $\pm$ 6.5 |
| 100 | 3 | 40.4 $\pm$ 3.0 | 45.9 $\pm$ 5.5 | 5.6 $\pm$ 3.5* |

Data are expressed as mean $\pm$ SEM from 5 rats.
\#\# P $\leq$ 0.01: compared with the vehicle intrathecally and intraduodenally. administered group using the Welch's test
\* P $\leq$ 0.025: compared with the vehicle intrathecally and compound A' intraduodenally administered group using the one-tailed Williams' test

**Example 4: Effect of Compound A' on urethra-closing reflex response in rats**

**Method**

Thirty-two female rats of SD strain were used. The rats were anesthetized with urethane. Isoflurane inhalation was added during the surgery, if necessary. Their spinal cord was transected at the T8-T9 (the eighth thoracic cord - the ninth thoracic cord) level to eliminate the reflex voiding and to inhibit the transition from the urine storage phase to the voiding phase in the voiding cycle. After opening of the abdomen, the bladder neck was ligated with suture, and a catheter (PE-100) was inserted into the bladder. The bladder catheter was connected to a pressure transducer and a saline-containing reservoir via three-way stopcocks. A microtip transducer catheter was inserted from the urethral orifice toward the bladder so that its transducer was inserted into the urethra. Local change in pressure within the urethra (intraurethral pressure) was recorded via an electric signal amplifier and an analog-to-digital converter. The intravesical pressure was increased from 0 cm H$_2$O to 50 cm H$_2$O by elevating the position of the saline-containing reservoir by 50 cm, while change in intraurethral pressure was observed for 30 seconds. Then, the reservoir was brought back to the original position to adjust the intravesical pressure to its baseline. Such change in intraurethral pressure induced by intravesical pressure rise was repeatedly measured, and the mean value from the last 2 trials was used as a Pre value. The vehicle used was 0.5% MC. Compound A' at 3 mg/mL/kg, and duloxetine at 20 mg/mL/kg were intraduodenally administered. Thirty minutes after administration, the change in intraurethral pressure induced by intravesical pressure rise was measured again and used as a Post value. The average value before intravesical pressure rise was subtracted from the average value during intravesical pressure rise, and the subtracted value was used as urethra-closing reflex response. The ratio of the Post value to the Pre value was calculated. The Welch's test was used as a significance test in comparison with the vehicle-administered group.

**Results**

The forced elevation of intravesical pressure in the urethane-anaesthetized spinalized rats increased the responses within the urethra. The vehicle did not change this urethral responses (see Table 6). By contrast, the intraduodenal administration of Compound A' at 3 mg/kg significantly enhanced the urethra-closing reflex responses (see Figure 1 and Table 6). The intraduodenal administration of duloxetine at 20 mg/kg significantly enhanced the urethra-closing reflex responses (see Table 6).

Compound A' significantly enhanced the urethra-closing reflex responses induced by intravesical pressure rise in rats.

[Table 6]

Table 6: Effects of compound A' and duloxetine on urethra-closing reflex induced by intravesical pressure rise by 50 cm $H_2O$ in rats

| Treatment | Dose | Urethra-closing Reflex response | | |
|---|---|---|---|---|
| | | Pre (cmH$_2$O) | Post (cmH$_2$O) | Ratio (Post/Pre) |
| Experiment 1 | | | | |
| Vehicle | - | 3.79 ± 0.98 | 3.69 ± 075 | 1.11 ± 0.13 |
| Comoound A' | 3 mg/kg, i,d. | 3.58 ± 0.42 | 10.40 ± 1.78 | 2,82 ± 0.29*** |
| Experiment 2 | | | | |
| Vehicle | - | 4.88 ± 0.86 | 5.03 ± 1.08 | 1.07 ± 0,11 |
| Duloxetine | 20 mg/kg, i.d. | 4.88 ± 0.86 | 8.66 ± 1.34 | 2.00 ± 0.28* |

Data are expressed as mean ± SEM from 8 rats.
* $P \leq 0.05$, *** $P \leq 0.001$: The ratio of the Post value to the Pre value was compared between the drug- and the vehicle-administered groups using the Welch's test.

**Example 5: Effects of Compound A', Compound B', and duloxetine on rat urethral resistance measured by high speed infusion of saline into the bladder**

**Method**

A hundred and twenty-four female rats of SD strain were used. The rats were anesthetized with isoflurane. Their spinal cord was transected at the T8-T9 (the eighth thoracic cord - the ninth thoracic cord) level. After opening of the bladder dome, two polyethylene tubes (PE-100) were inserted into the bladder. The incised portion of the bladder was closed with sutures, and the other incisions were also closed. The rats were placed in Bollman cages. The catheter for intravesical pressure measurement was connected to a pressure transducer, while the catheter for saline filling was connected to a syringe filled with Evans Blue-dyed saline. Change in intravesical pressure was recorded using the pressure transducer via an electric signal amplifier and an analog-to-digital converter. After recovery of the animals from anesthesia, the intravesical pressure measurement was started, and saline was infused at a speed of 0.1 mL/sec into the bladder using an infusion pump. The infusion was stopped when the leakage of the saline from the urethral orifice was observed. Immediately thereafter, the bladder was emptied by releasing the pressure. In this operation, the peak of recorded intravesical pressure during the saline infusion was used as LPP in this Example. This measurement was repeated, and the average LPP from the last three measurements was used as the LPP value of the trial. The mean from 2 such trials was used as a Pre value. 0.5% MC was used as the vehicle. Compound A' at 1.25, 2.5, 5, 10 and 20 mg/2 mL/kg, Compound B' at 1, 2.5 and 5 mg/2 mL/kg, and duloxetine at 5, 10, 20 and 40 mg/2 mL/kg were orally administered, and LPP was measured in the same way 30 minutes after administration. The difference in LPP from the Pre value (increased amount of LPP) was calculated. Regarding change in LPP before and after drug administration, the differences between the mean values of the drug-treated and vehicle-treated groups were analyzed by the one-tailed Williams' test.

**Results**

The high speed infusion of saline into the bladder increased the intravesical pressure. In this method, the oral administration of Compound A', Compound B', and duloxetine dose-dependently increased the LPP value (see Tables 7 and 8). $LPP_{10}$, the doses at which Compound A' and Compound B' increase LPP by 10 cm $H_2O$, was 1.6 mg/kg and 1.2 mg/kg, respectively.

[Table 7]

Table 7: Effects of compound A' and duloxetine on urethral resistance during infusion of saline at 0.1 mL/sec into rat bladder

| Drug | Dose (mg/kg. p.o.) | Pre | LPP (cmH$_2$O) Post | Increase |
|---|---|---|---|---|
| Experiment 1 | | | | |
| Vehicle | - | 60.7 ± 2.4 | 58.1 ± 3.0 | -2.5 ± 1.9 |
| Compound A' | 1.25 | 57.7 ± 3.6 | 63.2 ± 4.7 | 5.5 ± 3.0 |
| | 2.5 | 59.2 ± 2.6 | 70.2 ± 3.8 | 10.9 ± 2.2* |
| | 5 | 60.6 ± 3.6 | 75.9 ± 3.3 | 15.3 ± 3.7* |
| | 10 | 56.7 ± 2.4 | 78.4 ± 5.6 | 21.7 ± 4.4* |
| | 20 | 56.3 ± 5.1 | 77.7 ± 3.5 | 21.5 ± 3.4* |
| Experiment 2 | | | | |
| Vehicle | - | 617 ± 53 | 59.5 ± 4.9 | -2.2 ± 1.7 |
| Duloxetine | 5 | 59.9 ± 5.1 | 64.1 ± 5.5 | 4.2 ± 1.7 |
| | 10 | 59.8 ± 3.8 | 67.6 ± 3.5 | 7.7 ± 2.2* |
| | 20 | 579 ± 2.7 | 69.8 ± 4.0 | 11.9 ± 3.7* |
| | 40 | 60.2 ± 3.1 | 73.3 ± 5.3 | 13.1 ± 2.9* |

Data are expressed as mean ± SEM from 8 subjects.
*: p ≤ 0.025: The LPP increases in the vehicle- and drug-treated groups were compared using the one-tailed Williams' test.

[Table 8]

Table 8: Effect of Compound B' on urethral resistance during infusion of saline at 0.1 mL/sec into rat bladder

| Drug | Dose (mg/kg. p.o.) | Pre | LPP (cmH$_2$O) Post | Increase |
|---|---|---|---|---|
| Vehicle | - | 61.1 ± 3.8 | 62.4 ± 3.5 | 1.3 ± 0.9 |
| Compound B' | 1 | 67.9 ± 2.5 | 77.0 ± 3.6 | 9.0 ± 1.9* |
| | 2.5 | 68.5 ± 3.8 | 88.1 ± 3.5 | 19.6 ± 1.7* |
| | 5 | 60.2 ± 5.1 | 85.4 ± 4.1 | 25.1 ± 2.7* |

Data are expressed as mean ± SEM from 8 to 10 subjects.
*: p ≤ 0.025: The LPP increase in the vehicle- and drug-treated groups were compared using the one-tailed Williams' test.

**Example 6: Calculation of effective plasma concentrations of Compound A' and Compound B' (method for measuring urethral resistance in rats by high speed infusion of saline into the bladder)**

**Method**

Ten female rats of SD strain were anesthetized with isoflurane. In order to secure the same conditions as those for drug-administered animals, their spinal cord was transected at the T8-T9 (the eighth thoracic cord - the ninth thoracic cord) level. Then, a cannula for blood collection was inserted into the femoral artery. After recovery of the animals from anesthesia, Compound A' at 2.5 mg/2 mL/kg or Compound B' at 4 mg/2 mL/kg was orally administered (N = 5 in each group). These drugs were each dissolved in 0.5% MC. Thirty minutes after oral administration, up to 250 $\mu$L of blood was collected from the femoral artery or the tail vein and treated with heparin. The collected blood was immediately cooled on ice and centrifuged under conditions involving 3,000 rpm and 4°C for 10 minutes to obtain plasma. The plasma samples were stored under conditions of -80°C until drug quantification.

After thawing of the plasma samples, the internal standard compound was added thereto, and the mixture was then centrifuged under conditions of 5,250 rpm. The drug contained in 60 $\mu$L of the supernatant was quantified by LC-MS/MS.

**Results**

The plasma concentrations Compound A and Compound B 30 minutes after oral administration of Compound A' and Compound B' were 94.8 $\pm$ 24.1 ng/mL and 32.9 $\pm$ 18.0 ng/mL (mean $\pm$ S.D.), respectively. The plasma concentrations of Compound A and Compound B at $LPP_{10}$ were calculated by multiplying the obtained data by the ratio between the dosage used in this test and $LPP_{10}$, and consequently were 61 ng/mL and 9.9 ng/mL, respectively.

**Example 7: Effects of continuously subcutaneously administered Compound A' and Compound B' on body weight of obese rats (DIO-F344 rat) and their effective plasma concentrations**

**Method**

Forty male DIO-F344 rats bred with high fat diet (42 kcal % fat) were used. On the day before surgery, the rats were grouped on the basis of their body weight. Compound A' and Compound B' were each dissolved in saline. Osmotic pumps filled with the Compound A' or Compound B' solution were subcutaneously implanted in the rats under isoflurane anesthesia. The body weight just after surgery was used as a Pre value, and the body weight was measured every day until 14 days after surgery. Regarding the averages in the body weight on Day 14, the difference in the mean averages of the drug-treated group and the vehicle-treated group was analyzed by the one-tailed Williams' test.

In the morning and afternoon at Day 14, up to 250 $\mu$L of blood was collected from the tail vein or the vena cava under isoflurane anesthesia and treated with heparin. The collected blood was immediately cooled on ice and centrifuged under conditions involving 3,000 rpm and 4°C for 10 minutes to obtain plasma. The plasma samples were stored under conditions of -80°C. On a later day, after thawing of the plasma samples, the internal standard compound was added thereto, and the mixture was then centrifuged under conditions of 5,250 rpm. The drug contained in 60 $\mu$L of the supernatant was quantified by LC-MS/MS. The average Day 14 plasma concentration from the values of each rat measured in the morning and afternoon was used as a plasma concentration for the animal. The rate of change in body weight was calculated from the values measured just after and 14 days after osmotic pump implantation. The rate (average rate) of change of the vehicle group was subtracted from that of each drug-administered rat to plot the plasma concentration and the change in body weight. The plasma concentration at which the drug lowered the body weight by 3.8% was calculated by linear regression analysis.

**Results**

The continuous administration of the vehicle with the osmotic pump lowered the body weight of the group by 3.3% (see Table 9). The administration of Compound A' at 1.35 and 4.5 mg/animal/day for 2 weeks did not significantly change the body weight of the group compared with the vehicle-treated group. By contrast, the administration of Compound A' at 13.5 mg/animal/day for 2 weeks significantly lowered the body weight of the group (see Table 9).

The administration of Compound B' at 0.45 and 1.35 mg/animal/day for 2 weeks did not significantly change the body weight of the group compared with the vehicle-treated group. By contrast, the administration of Compound B' at 4.5 mg/animal/day for 2 weeks significantly lowered the body weight of the group (see Table 9).

[Table 9]

Table 9: Effects of compound A' and Compound B' subcutaneously administered continuously to male DIO-F344 rats on body weight

| Drug | Dose | Change in body weight (% change) | | | |
| --- | --- | --- | --- | --- | --- |
| | | The number of days lapsed after pump implantation (day) | | | |
| (mg/animal/day) | | 3 | 7 | 10 | 14 |
| Untreated | - | $0.2 \pm 0.3$ | $-0.3 \pm 0.2$ | $-1.1 \pm 0.4$ | $-0.4 \pm 0.7$ |
| Vehicle | - | $-1.0 \pm 0.4$ | $-1.6 \pm 0.5$ | $-3.3 \pm 0.8$ | $-3.3 \pm 1.0$# |
| Compound A' | 1.35 | $-1.5 \pm 0.5$ | $-2.3 \pm 0.8$ | $-4.5 \pm 0.8$ | $-4.7 \pm 0.8$ |
| | 4.5 | $-0.6 \pm 0.2$ | $-2.4 \pm 0.4$ | $-3.9 \pm 0.4$ | $-4.6 \pm 0.6$ |
| | 13.5 | $-2.7 \pm 0.2$ | $-4.8 \pm 0.3$ | $-6.9 \pm 0.5$ | $-7.8 \pm 0.5$* |
| Compound B' | 0.45 | $-0.9 \pm 0.2$ | $-14 \pm 0.5$ | $-3.3 \pm 0.4$ | $-3.7 \pm 0.6$ |
| | 1.35 | $-1.4 \pm 0.3$ | $-2.5 \pm 0.5$ | $-4.6 \pm 0.3$ | $-5.4 \pm 0.2$ |
| | 4.5 | $-3.1 \pm 0.6$ | $-4.8 \pm 0.9$ | $-7.1 \pm 0.7$ | $-8.6 \pm 1.0$* |

Data are expressed as mean $\pm$ SEM from 5 animals at each dosage The drug was administered continuously for 2 weeks using an osmotic pump. Statistical analysis was conducted using data at Day 14

\* $P \leq 0.025$: compared with the vehicle-treated group using the one-tailed Williams' test

\# $P \leq 0.05$: compared with the untreated group using the Student's *t*-test

The plasma concentration and the rate of change in body weight in each rat were plotted for each drug. The plasma concentrations at which Compound A' and Compound B' lowered the body weight by 3.8% were calculated and consequently, were 670 ng/mL and 140 ng/mL, respectively.

## Example 8: Clinical trial with Compound A' in humans

In this Example, MT (threshold inducing urethra-closing response) was measured in 24 healthy Japanese female volunteers by the randomized double-blind single-dose $4 \times 4$ crossover study to confirm the effect of Compound A'.

The test subjects were healthy female volunteers having a body weight of at least 45 kg (average: 50.91 kg, standard deviation: 3.346) and a BMI value of 18.5 kg/m$^2$ to 25.0 kg/m$^2$ (average: 19.57 kg/m$^2$, standard deviation: 0.929).

In the crossover study, the 24 subjects were divided into 4 groups (each group involving 6 persons), and subjected to the series of treatments in the order shown in Table 10 in order to reduce the influence of the difference among subjects and/or the order of the treatments on test results. A washout period of at least 7 days was provided between the treatments.

[Table 10]

Table 10: Order of Treatments

| Administration group | N | Period 1 | Period 2 | Period 3 | Period 4 |
| --- | --- | --- | --- | --- | --- |
| No. 1 | 6 | Treatment A | Treatment B | Treatment C | Treatment D |
| No. 2 | 6 | Treatment B | Treatment D | Treatment A | Treatment C |
| No. 3 | 6 | Treatment C | Treatment A | Treatment D | Treatment B |
| No. 4 | 6 | Treatment D | Treatment C | Treatment B | Treatment A |

In each period, the test subjects received any of Treatments A to D once according to Table 10, and change in the plasma concentration of the administered compound was measured over 24 hours after each Treatment. The details of Treatments A to D were as shown in Table 11.

[Table 11]

Table 11: Contents of Treatments

| Treatment | Compound A' | Duloxetine |
|---|---|---|
| Treatment A | Placebo solution (100 mL) | 40 mg capsule |
| Treatment B | Placebo solution (100 mL) | Placebo capsule |
| Treatment C | 20 mg of Compound A' in solution (100 mL) | Placebo capsule |
| Treatment D | 90 mg of Compound A' in solution (100 mL) | Placebo capsule |

Specifically, 100 mL of the injectable water containing 20 mg or 90 mg of Compound A' based on its free form was supplemented with a sweetener, and this solution was orally administered as Compound A'. As a placebo for Compound A', 100 mL of the injectable water was supplemented with a sweetener, and this solution was orally administered. A capsule containing 40 mg of duloxetine was orally administered as duloxetine together with water. A placebo capsule containing saccharose and spherical starch granules was orally administered as a placebo for duloxetine together with water.

The individual MT test was conducted as described in Boy S. et al., Eur. Urol. 2006, 50 (1): 119-125 and Yono M., LUTS: Lower Urinary Tract Symptoms, John Wiley & Sons Australia, Ltd, Vol. 2, Issue 7, Paper No. 12057, 2014 after emptying of the bladder. MT was measured under transcranial magnetic stimulation (TMS) to confirm the effects of the compounds on urethral functions.

Specifically, during the TMS, the output of a magnetic stimulator (Magstim Rapid Square, manufactured by Magstim Co., Ltd.) was increased by 5% each time using 110 mm double cone coil, and the minimum output at which urethra-closing response occurred abruptly was determined and used as MT (%). The urethra-closing response was detected by measuring contractile responses within the urethra. The contractile response within the urethra was measured using Duet Logic G2 (manufactured by Mediwatch Ltd.) and UniTip (manufactured by Unisensor AG). MT was measured again after administration of each drug, and the rate of change (%) from the baseline was calculated. The rate of change was determined by dividing MT after administration of each drug by MT before administration. Experimental data were interpreted such that decrease in MT (%) means that the test compound decreased the stimulation threshold inducing urethra-closing response and enhanced the responsiveness of the urethra, i.e., enhanced the urethral functions.

Plasma samples were obtained from each subject at indicated time points and plasma concentration of Compound A or duloxetine were measured (see Table 12).

[Table 12]

Table 12: Measurement schedule for each Treatment

| Day | Time point | MT test | Compound A concentration measurement | Duloxetine concentration measurement |
|---|---|---|---|---|
| Day 1 | Predose | × | × | × |
| | Administration | | | |
| | 0.5 hr | × | × | × |
| | 1 hr | | × | |
| | 1.5 hr | | | |
| | 2 hr | | × | |
| | 3 hr | × | × | × |
| | 4 hr | | | |
| | 6 hr | × | × | × |
| | 8 hr | | × | × |
| | 12 hr | | × | × |
| Day 2 | 24 hr | | | × |

After this series of MT measurements, 500 mg of an antibiotic (levofloxacin; CRAVIT) was administered for the purpose of preventing infection in the urethra.

The results of the MT test are summarized in Tables 13 and 14 below.

[Table 13]

Table 13: MT of the urethra-closing response in response to TMS (%)

| Time point | Treatment | N | Mean | S D |
|---|---|---|---|---|
| Predose | Placebo | 23 | 68.0 | 9.97 |
| | 20 mg of Compound A' | 24 | 66.3 | 12.27 |
| | 90 mg of Compound A' | 23 | 70.0 | 10.22 |
| | Duloxetine | 23 | 67.8 | 11.46 |
| 0.5 hr | Placebo | 23 | 66.7 | 11.04 |
| | 20 mg of Compound A' | 24 | 58.1 | 14.05 |
| | 90 mg of Compound A' | 23 | 55.9 | 11.45 |
| | Duloxetine | 23 | 67.4 | 10.86 |
| 2 hr | Placebo | 23 | 66.3 | 10.47 |
| | 20 mg of Compound A' | 24 | 59.0 | 14.29 |
| | 90 mg of Compound A' | 23 | 52.8 | 10.43 |
| | Duloxetine | 23 | 62.0 | 12.77 |
| 6 hr | Placebo | 23 | 70.0 | 10.87 |
| | 20 mg of Compound A' | 24 | 64.2 | 13.81 |
| | 90 mg of Compound A' | 23 | 56.3 | 10.58 |
| | Duloxetine | 22 | 56.4 | 12.17 |

[Table 14]

Table 14: Rate of change from baseline of MT of the urethra-closing response in response to TMS (%)

| Time point | Treatment | N | Mean | S D |
|---|---|---|---|---|
| 0.5 hr | Placebo | 23 | -1.3 | 4.05 |
| | 20 mg of Compound A' | 24 | -8.1 | 6.89 |
| | 90 mg of Compound A' | 23 | -14.1 | 7.78 |
| | Duloxetine | 23 | -0.4 | 3.34 |
| 2 hr | Placebo | 23 | -1.7 | 4.67 |
| | 20 mg of Compound A' | 24 | -7.3 | 7.22 |
| | 90 mg of Compound A' | 23 | -17.2 | 6.37 |
| | Duloxetine | 23 | -5.9 | 5.36 |
| 6 hr | Placebo | 23 | 2.0 | 4.19 |
| | 20 mg of Compound A' | 24 | -2.1 | 7.36 |
| | 90 mg of Compound A' | 23 | -13.7 | 6.07 |
| | Duloxetine | 22 | -11.8 | 7.33 |

The plasma concentration of Compound A after administration of Compound A' is shown in Table 15 below. Time-dependent change in the plasma concentration of Compound A is shown in Figure 2.

[Table 15]

Table 15: Time-dependent change in plasma concentration of compound A

| Compound A' | Statistics | Visit | | | | | | | |
|---|---|---|---|---|---|---|---|---|---|
| | | Predose | 0.5 hr | 1 hr | 2 hr | 3 hr | 6 hr | 8 hr | 12 hr |
| 20 mg of compound A' | | | | | | | | | |
| | N | 23 | 23 | 23 | 23 | 23 | 23 | 23 | 23 |
| | Mean | 0.000 | 29.21 | 25.19 | 20.46 | 14.28 | 3.476 | 1.416 | 0.1327 |
| | SD | 0.0000 | 10.229 | 10.402 | 8.6609 | 6.6516 | 1.9789 | 0.83107 | 0.31394 |
| | Minimum | 0.00 | 9.98 | 10.1 | 7.12 | 4.07 | 1.01 | 0.00 | 0.00 |
| | Q1 | 0.000 | 23.60 | 18.20 | 13.70 | 8.460 | 1.610 | 0.6900 | 0.000 |
| | Median | 0.000 | 28.40 | 24.10 | 19.20 | 13.10 | 3.760 | 1.590 | 0.000 |
| | Q3 | 0.000 | 37.20 | 29.80 | 26.80 | 20.10 | 4.490 | 1.950 | 0.000 |
| | Maximum | 0.00 | 50.4 | 47.0 | 41.4 | 30.9 | 9.38 | 3.54 | 1.19 |
| 90 mg of compound A' | N | 23 | 23 | 23 | 23 | 23 | 23 | 23 | 23 |
| | Mean | 0.000 | 127.3 | 126.0 | 152.8 | 122.4 | 52.81 | 24.69 | 5.612 |
| | SD | 0.0000 | 79.201 | 59.171 | 68.140 | 52.359 | 31.374 | 16.544 | 4.5311 |
| | Minimum | 0.00 | 15.0 | 40.1 | 55.2 | 36.8 | 7.12 | 3.22 | 0.816 |
| | Q1 | 0.000 | 60.70 | 81.50 | 102.0 | 80.50 | 30.70 | 10.60 | 2.060 |
| | Median | 0.000 | 114.0 | 113.0 | 145.0 | 127.0 | 47.30 | 20.30 | 4.450 |
| | Q3 | 0.000 | 198.0 | 162.0 | 195.0 | 148.0 | 73.80 | 35.70 | 8.260 |
| | Maximum | 0.00 | 283 | 265 | 316 | 241 | 139 | 71. 6 | 21. 2 |

Results

The mean threshold inducing urethra-closing response (MT) in response to TMS decreased after a single administration of Compound A' at doses of 20 mg and 90 mg and duloxetine. The maximum change from baseline in MT in response to TMS was -8.1% in the Compound A' 20 mg group at 0.5 hours post dose, -17.2% in the Compound A' 90 mg group at 3 hours post dose, and -11.8% in the duloxetine 40 mg group at 6 hours post dose. The maximum change from baseline in the MT of Compound A' 90 mg was greater than those in the other treatment groups. The results of the ANOVA analyses showed that the changes from baseline in MT in response to TMS were significantly lower in the Compound A' 20 mg and 90 mg groups than in the placebo group across the 3 evaluation time points (0.5, 3, and 6 hours post dose).

**Example 9: Determination of effective plasma concentration of Compound A' for human**

In this Example, the effective plasma concentration of Compound A' for urethral functions in humans was calculated using results obtained in phase I clinical trial as well as the results obtained in Example 8.

**Clinical trial data**

Specifically, 3 mg, 10 mg, 20 mg, 30 mg, 45 mg, or 60 mg of Compound A' based on its free form or a placebo was orally administered to 54 healthy Japanese female volunteers (hereinafter referred to as "Test 1"), and 3 mg, 10 mg, 30 mg, 60 mg, 120 mg or 180 mg of Compound A' based on Compound A' or a placebo was orally administered to 56 healthy American female volunteers (hereinafter referred to as "Test 2"). In this Example, the results of these tests 1 and 2 as well as the results of Example 8, i.e., the results of the $4 \times 4$ crossover study in which 20 mg or 90 mg of Compound A' based on its free form, a placebo, or duloxetine was orally administered to 24 healthy Japanese female volunteers (in this Example, referred to as "Test 3"), were used.

**Analysis method**

**List of Abbreviations and Definitions of Terms**

| | |
|---|---|
| AIC: | Akaike's information criterion |
| AUC: | area under the plasma concentration-time curve |
| AUCinf: | AUC extrapolated to infinity from time 0 |
| AUClast: | AUC until last measurement |
| BID: | twice a day |
| BSV: | between subject variability |
| C: | concentration Compound A in central compartment |
| CL: | clearance |
| Cmax: | maximum plasma concentration |
| CMT: | compartment |
| CSV: | comma delimited |
| CV%: | coefficient of variation (%) |
| CWRES: | conditional weighted residuals |
| Duloxetine: | compound for comparison |
| DV: | dependent variable |
| F1: | 1st relative bioavailability |
| F2: | 2nd relative bioavailability |
| FOCE: | first-order conditional estimation method in NONMEM |
| FOCEI: | FOCE interaction method |
| GOF: | goodness-of-fit |
| h: | hour |
| ID: | unique subject ID |
| IPRED: | individual prediction |
| ka: | absorption rate constant |
| LLCI: | lower limit of confidence interval |
| LLOQ: | lower limit of quantification |
| MT: | threshold inducing urethra-closing response (motor threshold) |
| NONMEM: | non-linear mixed effects modeling program |
| OBJ: | objective function value |
| PD: | pharmacodynamics |
| PK: | pharmacokinetics |

PRED:       population prediction
QA:         quality assurance
QC:         quality control
QD:         once a day
se:         standard error
SUI:        stress urinary incontinence
ULCI:       upper limit of confidence interval
V:          volume of distribution of the central compartment
VPC:        visual predictive check
WSV:        within-subject variability
$\varepsilon$:          random effect for residual variability
$\eta$:          random effect for BSV
$\theta$:          fixed-effects parameter
$\alpha^2$:         variance of $\varepsilon$
$\chi^2$:         chi square distribution
$\omega^2$:         variance of $\eta$

**Modeling approach**

The PK-PD analysis was performed using a stepwise population modeling approach. In a first step, the population PK model for Compound A' was developed using data from Tests 1 to 3 separately. Two separate preliminary models, previously developed by Takeda Pharmaceuticals International AG were considered as starting structures. The objective of this analysis was to develop a single comprehensive population PK model, based on all available data of Tests 1 to 3. The population PK model for Duloxetine was developed using data from Test 3 and the PK model, developed by Skinner et al., was used as starting model.

In a second step, the individual posthoc estimates of the final PK models developed in the current analysis were used as input for the development of the PK-PD model for % threshold inducing urethra-closing response (%MT).

**PK model development**

**Compound A'** For the development of the PK model of Compound A', the two models developed by Takeda Pharmaceuticals International AG, were reevaluated on the separate and combined data from Tests 1 to 3. In short, both models had two compartments with a dose dependent clearance from the central compartment. Furthermore:
The first model, describing the single rising dose (SRD) data from Test 2 had two absorption sites.
The second model was a two compartmental model with a single absorption site to characterize the PK of Compound A' in Test 1.

Different modifications to the structural model (e.g., one and two compartmental structures, with different absorption, clearance and bioavailability models) and stochastic models (i.e. between subject variability (BSV) on different structural parameters) were evaluated. The influence of the covariate *study site* (i.e., Japanese versus United States) was also included in the analysis.

**Duloxetine** For the development of the PK model of Duloxetine, the model published by Skinner et al. was optimized to the Duloxetine data of Test 3. The published model is a one-compartmental model with first order absorption and BSV on central clearance and volume of distribution. Small structural (e.g. adding lag time) and stochastic modifications to the published model were evaluated to describe the PK of Duloxetine in Test 3 adequately.

**PK-PD model development**

For the development of the PK-PD model a stepwise approach was taken. In a first step, the effect of placebo on %MT was investigated, using the data from the placebo groups only. In a second step, the %MT data following administration of Compound A' and Duloxetine were analysed simultaneously to develop PK-PD models to describe the drug effect of Compound A' and Duloxetine on % MT. The identified placebo parameters were fixed in this step, and the PK posthoc estimates of Compound A' and Duloxetine were used as input for the development of the PK-PD model. For the development of the PK-PD model a drop in objective function of 10.83 (1 degree of freedom; theoretically coinciding with p < 0.001) was considered significant.

Simulations were performed to visualize the PK and PD characteristics of Compound A' using the final PK-PD model. The simulations were based on the parameters for the US population while administering the free base of Compound A'. The following simulations were done:

Simulations of time-above-threshold for different doses and dose regimens (deterministic simulations)
Simulations of the effect - time profile for Compound A' (stochastic simulations) Simulations of the concentration - effect curve for Compound A' (stochastic simulations)

**System Qualifications and Estimation methods**

The population models were fitted by means of non-linear mixed-effects as implemented in the NONMEM software package (version 7 level 2; Icon Development Solutions, Ellicott City, Maryland, USA).

Diagnostic graphics, exploratory analyses, and post-processing of NONMEM output were performed using S-Plus (version 8.2 Professional, Insightful Corp., Seattle, USA), R (R version 3.1.2 (2014-10-31)) facilitated via R-Studio and Berkeley-Madonna Compaq Visual Fortran (version 6.6, Compaq Computer Corporation, Houston, Texas, USA) was used as compiler.

A convergence criterion of 3 significant digits in the parameter estimation was used. The obtained minimum value of objective function, defined as minus twice the log-likelihood, was used for model comparisons. The first-order condition estimation approximation (FOCE) was used as estimation method. Furthermore, the INTERACTION option was used in NONMEM, which takes the presence of an interaction between the two levels of random effects into account.

**Statistical considerations**

In general, the principle of parsimony was applied, meaning that the simplest model that describes the data adequately is preferred in the process of model development. The influence of adding an additional model parameter (structural or stochastic) on the model fit was analyzed according to the following statistical considerations:

To compare two nested models a likelihood-ratio test was performed, under the assumption that the difference in minimum value of objective function (OBJ) of two competing models is $\chi^2$ distributed. The degrees of freedom ($q$) are determined by the number of additional parameters in the more complex model. In case of adding one parameter to the model (degrees of freedom of 1) and a significance level of $p < 0.05$, a decrease in OBJ of 3.84 points was considered statistically significant (unless stated otherwise).

In case of a comparison of models, which are structurally different (and thus not nested), the models were compared using the Akaike Information Criterion (AIC), calculated by Equation (I). Model A is considered statistically superior to model B if $\Delta AIC < 0$, and vice versa.

[Equation 1]

$$\text{Equation (I):} \qquad \Delta(AIC) = OBJ_A - OBJ_B + 2\,(p_A - p_B)$$

In Equation (I), pA and pB are the number of model parameters for model A and B respectively.

The standard error of a structural parameter estimate reported by NONMEM should preferably be less than 50% of the estimated parameter value. This would imply that zero is excluded from the 50% confidence interval of the parameter estimate, assuming normality.

The correlation between parameter estimates (structural and stochastic) reported by NONMEM in the correlation matrix of the model output, should lie between -0.95 and 0.95.

The values estimated for $\eta$ should be adequately centered around zero (reported p-value in the out file should be larger than 0.05).

Shrinkage of the random effects ($\eta$-shrinkage and $\varepsilon$-shrinkage) should preferably be below 30%.

**Model evaluation**

Standard goodness of fit (GOF) plots were inspected visually to evaluate the model fit:

Observations *versus* individual predictions: data should be randomly distributed around the identity line.
Observations *versus* population predictions: data should be randomly distributed around the identity line.
(Conditional) weighted residuals *versus* time: (conditional) weighted residuals should be randomly distributed around zero for the complete time course.
(Conditional) weighted residuals *versus* population predictions: (conditional) weighted residuals should be randomly distributed around zero for all population predicted values.

Due to the limited number of subjects in several treatment groups, no robustness analysis, by means of nonparametric bootstrap re-sampling technique, was performed. In order to evaluate whether the developed models adequately described the Compound A' and % MT observations, simulations were performed with the final models and visually compared with the actual observations. In the visual predictive check (VPC), the observation versus time profile was simulated 500 times (unless stated otherwise) by means of Monte Carlo simulations. In a Monte Carlo simulation, random values are drawn from the distributions of the identified random effects. Subsequently, the median and 5 and 95 percentiles (unless stated otherwise) of the simulated dependent variables were calculated for each time after dose (TAD).

**Results**

**PK Model**

**(1) Compound A'** The PK of Compound A' is nonlinear over the investigated dose range, mostly evident in the dual peak in the pharmacokinetic profile. The Japanese studies (Tests 1 and 3) generally showed higher exposures compared to the US study (Test 2). Furthermore, graphical investigation of the raw data suggests that the dual peak already appears at lower doses in the Japanese population as compared to the US study.

The resulting PK model is a one compartmental model. To describe the dual peak, the dose was divided over two dose compartments, each with a separate lag time. The absorption from the second dose compartment into the central compartment is described with sequential lagged zero and first order process. In addition, the 2nd absorption route includes a nonlinear function to describe this relative bioavailability fraction (Equation (II)):

[Equation 2]

$$F2 = F2_{\text{int}} \cdot \exp^{Dose \cdot F2_{\text{exp}}} \qquad (\text{II})$$

The parameters describing the 2nd relative bioavailability fraction vary between the Japanese studies and the US study. Elimination was described with a first order elimination and a nonlinear clearance that helps describing the increased pseudo half-life with higher doses and, in combination with the 2nd relative bioavailability fraction, the dual peak phenomenon (see Figure 3). In addition, the central volume of distribution (V), clearance (CL), absorption rate constant (ka) and 2nd lag time (ALAG2 - lag time2) differed between the Japanese and US study sites.

BSV was identified on the central volume of distribution (V) and the bioavailability fractions F1 and F2. Covariance was optimized between the BSVs on the relative bioavailability fractions F1 and F2. Furthermore, a proportional error model was used to describe the residual error for Tests 1 to 3 separately.

In the PK model the η-shrinkage was below 9% and the η's were adequately centered around zero. The ε-shrinkage was below 19%. The parameters could be estimated with good precision. The final population PK model described the concentration-time profile of Compound A' generally well, as shown in the VPC for all dose groups and populations (see Figure 4 for 20 mg dose in Japanese population, Figure 5 for 90 mg dose in Japanese population, and Figure 6 for Duloxetine).

**(2) Duloxetine** The Duloxetine model is a one compartmental model with linear first order elimination and lagged first order absorption. Parameters for clearance (CL), the volume of distribution (V), the absorption rate constant (ka) were optimized using the data from Test 3.

A lag time was used to describe the delayed absorption. The estimated parameters for CL, ka and V values are higher than reported by Skinner et al. BSV was identified on both the relative bioavailability fraction (F1) and the lag time, while in the model of Skinner et al. BSV was on CL and V. Both proportional and additive errors were used to describe residual variability. In the PK model the η-shrinkage was below 14% and the η's were adequately centered around zero. The ε-shrinkage was below 20%. The parameters could be estimated with adequate precision. The final population PK model described the concentration-time profile of Duloxetine adequately, as shown in the visual predictive check (Figure 6).

**PK-PD (%MT) model**

A PK-PD model was developed in a stepwise approach to characterize the effect of placebo, Compound A' and Duloxetine on %MT. In a first step, a descriptive model was developed to characterize the placebo effect on %MT using the data following placebo treatment only. The resulting model of the placebo effect on %MT is described using Equation (III):

[Equation 3]

$$\%MT = Baseline_{MT} \cdot (1 + (1 - \exp^{time \cdot k_{onset}}) \cdot \exp^{-time \cdot k_{recov}} \tag{III}$$

with $K_{onset}$ and $K_{recov}$ as the first order rate constant to describe the decrease from the baseline and return to the baseline, respectively.

No additional increase in baseline over time was identified. Furthermore, period effects were explored but not included in the model as confidence intervals widely overlapped. Therefore, the baseline is considered to remain constant over time. However, it should be kept in mind that the number of observations per subject is limited and that only observations up to 6h after dose are available. The placebo effect was fixed in the second step, during the analysis of the drug effect of Compound A' and Duloxetine on %MT. The final PK-PD model used the posthoc estimates of the final PK models of Compound A' and Duloxetine to describe their respective pharmacokinetic individual profiles. A log-linear concentration-effect relationship (Equation (IV)) with the drug effect (DEFF) proportional to the placebo effect was used to describe the %MT-time profile following administration of Compound A' and Duloxetine.

[Equation 4]

$$\text{Equation (IV):} \qquad DEFF = SLP \cdot \log(C+1)$$

with SLP as slope and C as the plasma concentration of Compound A' and Duloxetine.

A separate slope was identified for the concentration-effect relationship. The resulting model is described using Equation (V):

[Equation 5]

Equation (V)

$$\%MT = Baseline_{MT} \cdot (1 + (1 - \exp^{time \cdot k_{onset}}) \cdot \exp^{-time \cdot k_{recov}} \cdot (1 + DEFF)$$

BSV was identified on Baseline MT and SLP for Compound A'. In addition, Baseline MT showed inter-occasion variability (IOV) and an additive error model was used to describe the residual error. The parameters could be estimated with adequate precision. The $\eta$ was centered around zero. The $\eta$- shrinkage was generally below 23% for BSV on Baseline MT and SLP, and IOV on baseline for the first and second period of the administration sequence, while 57 and 36% for IOV on baseline for the third and fourth period. The $\varepsilon$-shrinkage was below 14% (Figure 7). The VPCs demonstrate that the final PK-PD model adequately characterizes the placebo-effect and the concentration-time profile of Compound A' and Duloxetine in the different dose groups.

**Simulations**

The PK and PK-PD parameters for Compound A' in the US population and the PK parameters for Duloxetine were considered for the simulations. The free base of Compound A' was used as input. For these simulations, a number of assumptions and limitations should be considered. In brief:

    The PK of Compound A' is assumed to be the same following single dose (in this analysis) and multiple doses
    The PK of Duloxetine is assumed to be the same in US and Japanese subjects
    The PK and PK-PD of Compound A' and Duloxetine can be extrapolated beyond the investigated dose and time-range
    The effect of placebo, Compound A' and Duloxetine on %MT is the same in US and Japanese study populations.

**Dose and Dose regimen simulation**

The deterministic simulations (only profile of typical subject - no BSV) were performed using Berkeley Madonna. In these simulations the effect during 24h of different doses and dosing regimens (single dose (QD) vs. 2 doses with 8h interval (BID)) of Compound A' was compared with the effect on %MT following a single dose of 40 mg Duloxetine. The simulated %MT-time profiles following placebo, Compound A' and Duloxetine can be found in Figures 8 and 9. As a result, following a single dose of 160 mg Compound A' the same induction threshold (MT) as Duloxetine 40 mg will be achieved at 8h. For the BID dosing regimen of Compound A' a dose of 120 and 200 mg will result in the same induction threshold (MT) as a single dose of 40 mg Duloxetine at 16 and 24h, respectively.

From these simulation, it was calculated how long the effect on %MT was larger than the effect following a single dose of 40 mg duloxetine, using different Compound A' dose strengths (20-200 mg). The results are visualized in Figures 10 and 11. The time above the threshold increases with increasing dose for both a QD and BID dosing regimen.

**Effect - time profile**

Stochastic simulations were performed to visualize the *"percentage from MT baseline"* versus time profile of Compound A' in comparison to 40 mg Duloxetine. Doses of 9, 17, 26, 52, 77, 103, 155, 180 and 206 mg Compound A' were included (n=300 per dose group). The variability in PK and PD was taken into account and the simulations were performed using R. The PK parameters for the US study site were combined with the PD parameters for these simulations. At 0, 0.25, 0.5, 1, 1.5, 2, 3, 4, 6, 8, 10, 12, 16 and 24 hours PD was simulated. The percentiles of the effect were then calculated per time point. Figures 12 and 13 present the resulting outcome. The median and the 90% Prediction Interval with (Figure 12) and without (Figure 13) an additional placebo effect are shown for Duloxetine and Compound A'.

The placebo effect has a minor influence on this maximum effect of Duloxetine. For Compound A' there is a larger placebo influence because the time of the respective maximum effects overlap, especially for the lower Compound A' doses. For the higher doses, the second peak in the PK profile becomes more prominent and diminishes the influence of the placebo effect.

**Concentration - effect relationship**

Furthermore, from the stochastic simulations for the effect versus time profiles, a concentration - effect relationship was constructed for Compound A'. The median concentration per time point versus the corresponding low, median and high percentiles of the effect at that particular time point are plotted. Figure 14 presents the relationship on a linear scale. For each dose the maximum concentration and corresponding effect can be observed. The horizontal intermittent line presents the median maximum placebo effect. The horizontal solid line presents the median of the Duloxetine maximum effect, with the intermittent lines representing the 5th and 95th around that median effect. The vertical lines correspond to the concentration of Compound A' at which concentration a similar effect on %MT can be achieved.

The approximate median Compound A' concentration for reaching the corresponding Duloxetine maximum effect and its percentiles are: 15, 45, and 130 ng/mL for the 5th, median and 95th effect, respectively. Further, a plasma concentration of 2 ng/mL would approximate a similar effect as the maximum effect shown in placebo.

**Example 10: Comparison of plasma concentrations between effects of Compound A' and Compound B' on urethra-closing functions and their anti-obesity effect**

Compound A' and Compound B', which are 5-$HT_{2C}$ receptor agonists, exhibited a urethral resistance increasing effect and a body weight lowering effect in rats at a certain plasma concentration. The respective effective plasma concentrations at which both the drugs exerted their effects were elucidated in Examples 6 and 7. For both of the drugs, as demonstrated in Examples, the plasma concentrations exhibiting a urethral resistance increasing effect were evidently lower than those lowering the body weight in rats. The non-clinical study results were analyzed as follows.

As the maximal urethral resistance increasing effects of duloxetine, a launched drug for treatment of SUI, was around 10 $cmH_2O$ elevation in rats (see Examples 2 and 5), it is recognized that the effect increasing urethral resistance by 10 $cmH_2O$ ($LPP_{10}$) in rats reflects a therapeutically sufficient activity. In addition, obese patients treated with the launched dosage of Compound B' (10 mg, twice daily [bid]) had a significantly greater weight loss than that of a placebo group with a difference of 3.8% (Blossom trial). Therefore, the plasma concentration of Compound A and Compound B at $LPP_{10}$ in rats (61 ng/mL and 9.9 ng/mL, respectively; see Example 6) was compared with that showing greater body weight loss in obese rats than placebo treatment by 3.8% (670 ng/mL and 140 ng/mL, respectively; see Example 7). Compound A' and Compound B' unexpectedly showed sufficient urethral resistance increasing effects in rats at 11- and 14- fold lower plasma concentration, respectively, than that showing greater weight loss by 3.8% in the obese rats.

The potency of Compound A' for each of the 3 distinct desirable effects such as *in vitro* agonist activity for human 5-$HT_{2C}$ receptors, urethral resistance increasing effect in rats and body weight lowering effect in obese rats was lower than those of Compound B', when compared as a free base. The ratio of each activity of Compound B' as a free base to Compound A' as a free base are in the same range (3.3-, 6.2- and 4.8-fold for *in vitro* agonist activity, urethral resistance increasing effect and body weight lowering effect, respectively). Therefore, it is natural to interpret that the ratio of potency between the two compounds for urethral resistance increasing and body weight lowing effects should be mainly due to the ratio of potencies of agonist activity for 5-$HT_{2C}$ receptors.

Both compounds increased urethral resistance at plasma concentrations much lower than that showing greater body weight loss than vehicle treatment in rats. As for the body weight lowering effect, 5-HT$_{2C}$ receptors reportedly work in the hypothalamus, whereas, as for the urethral resistance increasing effect, Compound A' was found to work in the spinal cord (see Examples 2 to 4). The difference in site of action between the body weight lowering effect and the urethral resistance increasing effect is thought to be at least partly responsible for the difference in effective plasma concentrations between these distinct effects.

These non-clinical findings demonstrated that the urethral resistance increasing effects of Compound A' and Compound B' were obtained at clearly lower plasma concentrations than that showing the greater body weight loss than vehicle treatment by 3.8%. At the plasma concentrations for SUI treatment, it would not be necessary to select SUI patients based on their BMI, as SUI patients can be treated with 5-HT$_{2C}$ agonists such that the plasma concentrations of the 5-HT$_{2C}$ agonists will not essentially show body weight lowering effects, regardless of whether or not the SUI patients have obesity.

Plasma concentrations of Compound B, at which the therapeutic effects on SUI are obtained were analyzed by 2 ways as described below.

PK/PD analysis (Example 9) for the clinical study investigating effects on urethra-closing function (threshold inducing urethra-closing response; Example 8) revealed that Compound A at 45 ng/mL exerts similar lowering effects on threshold inducing urethra-closing response to the average lowering effect of duloxetine at Cmax. The plasma concentration in humans of 45 ng/mL was close to the plasma concentration elevating urethral resistance by 10 cmH$_2$O in rats (61 ng/mL), indicating good predictability of the rat assay (1.4-fold difference). Furthermore, PK/PD analysis also indicated that more than 2 ng/mL of Compound A would show the effects on urethra-closing function, which would not be obtained by placebo treatment, and the plasma level of 2 ng/mL is 22.5-fold lower than 45 ng/mL, which will exert similar effects on threshold inducing urethra-closing response to the average effect of duloxetine at Cmax.

On the other hand, the plasma concentration of Compound B which increased urethral resistance by 10 cmH$_2$O in rats was 9.9 ng/mL, indicating that Compound B at 7.1 ng/mL (i.e., 1.4-fold lower than 9.9 ng/mL) would show the sufficient urethra-closing effects in humans. In addition, more than 0.32 ng/mL (i.e., 22.5-fold lower than 7.1 ng/mL) of Compound B would be expected to show effects greater than the maximal effect obtained with placebo.

As explained above, around 14- fold lower plasma concentrations of Compound B than that showing minimum effect as an anti-obesity therapy could sufficiently increase urethral resistance in rats. On the other hand, as the average daily plasma concentration of Compound B during steady state at the launched dose is reportedly 43 ng/mL (Blossom trial), plasma concentrations of around 3 ng/mL (14-fold lower than 43 ng/mL) would be estimated to show sufficient effects on urethra-closing function in humans. The values obtained by the 2 analysis methods, 3 ng/mL and 7.1 ng/mL, are roughly in the same range, increasing confidence in the obtained value of 7.1 ng/mL based on urethral function data in rats and humans.

The analysis in Examples 9 and 10 indicates that Compound A at more than 2 ng/mL and Compound B at more than 0.32 ng/mL should show some effects on urethra-closing functions in humans, and Compound A at 45 ng/mL and above and Compound B at 7.1 ng/mL and above would be expected to be as effective as Duloxetine at the launched dosage for treating SUI, which indicates that these concentrations are sufficiently effective. For example, Compound A' at 20 mg showed significant effects on urethra-closing function 30 minutes, 3 hours and 6 hours after administration, and plasma concentrations of Compound A at these time points were 29 ng/mL, 14 ng/mL and 3.5 ng/mL, respectively.

Plasma concentrations of Compound A required for the treatment of obesity were analyzed. Plasma concentration of Compound B at the launched dose (10 mg, bid) for the treatment of obesity varies in a day from 26 ng/mL (Cmin) to 56 ng/mL (Cmax), and the average level at steady state is 43 ng/mL. The plasma level of 43 ng/mL is 3.3 fold less than that showing greater body weight loss in obese rats than vehicle treatment by 3.8%. As 670 ng/mL of Compound A showed greater body weight loss in obese rats than placebo by 3.8%, 203 ng/mL (3.3-fold less than 670 ng/mL) as an average level at steady state in obese patients would be expected to show body weight lowering effects.

An additional analysis was performed to elucidate the plasma concentration of Compound A below which any sufficient therapeutic effects on obesity would not be expected. Compound B' at 10 mg, once daily administration (QD) shows insufficient body weight loss (difference of body weight loss from placebo is 2.6%; Blossom trial) and the average daily plasma level at steady state is 24 ng/mL. The plasma level of 24 ng/mL is 5.8 fold less than that showing greater body weight loss in obese rats than vehicle treatment (140 ng/mL). Therefore, Compound A at equal to and less than 116 ng/mL (5.8-fold less than 670 ng/mL) as an average daily plasma level at steady state in obese patients would not show sufficient body weight loss for anti-obesity therapy.

The plasma concentration ranges of Compound A and Compound B for treatment of SUI without showing body weight lowering effects was analyzed. The analysis described in the above paragraphs indicated that compound A at more than 2 ng/mL and less than 203 ng/mL and Compound B at more than 0.32 ng/mL and less than 43 ng/mL would show therapeutic effects on SUI without showing body weight loss to an extent as observed after 10 mg Compound B' administration (bid). In addition, compound A at more than 2 ng/mL and equal to or less than 116 ng/mL and Compound B at more than 0.32 ng/mL and equal to or less than 24 ng/mL would show effects for the treatment of SUI without showing body weight loss to an extent as observed after 10 mg Compound B' administration (qd). The plasma concentration ranges of each drug exerting urethra-closing function is unexpectedly and remarkably lower than the plasma concentration range showing sufficient body weight loss as an anti-obesity drug. In addition, adverse events (e.g., nausea in 8.8% subjects and headache in 32.5% subjects) were observed in 57.9% of patients in the Compound B' 10 mg administered group (bid), which means adverse effects should be drastically reduced at these plasma concentrations increasing urethra-closing function (showing therapeutic effects on SUI). Therefore, 5-HT$_{2C}$ receptor agonists used within the plasma concentration range shown here may be advantageously administered even to stress urinary incontinence patients having no obesity, and may serve as safe drugs with fewer adverse effects.

The preferred duration to keep the effective plasma concentration in a patient is variable, depending on the target diseases. For obesity, it is required to keep the plasma concentration of 5-HT$_{2C}$ agonist above the effective one for 24 hours in a patient and then keep the appetite of the patient reduced to effectively decrease body weight in the patient. Otherwise, the patient may eat too much at one time only to gain undesirable body weight. In contrast, a preferred duration to keep the effective plasma level suitable for SUI would depend on the patient's demand. In cases where patients want to avoid SUI only during a one- or three-hour trip, a medicament with at least 1- or 3-hour effective duration would be sufficient to meet the demand. In case of full-time employment, at least 8 to 12 hour-effectiveness would be preferred. Therefore, a preferred effective duration would depend on a patient's demand, and a medicament with variety of effective duration, for example, from 1 hour to 24 hours would be useful in the treatment of SUI. Therefore, the administration to a patient of Compound A, Compound A salt including Compound A', Compound B or Compound B salt including Compound B' which provides an effective plasma concentration for a duration from 1 hour to 24 hours in the patient may be used for the treatment of SUI. In addition, the medicament comprising Compound A, Compound A salt including Compound A', Compound B or Compound B salt including Compound B' which provides an effective plasma concentration for a duration from 1 hour to 24 hours also can be used for the treatment of SUI.

In summary, the administration of Compound A or Compound A salt including Compound A' and the medicament comprising Compound A or Compound A salt including Compound A' which provides plasma concentration at more than 2 ng/mL and less than 203 ng/mL for a duration from 1 hour to 24 hours can be used for the treatment of SUI, in an SUI patient with a normal, lower or larger BMI.

Furthermore, the administration of Compound A or Compound A salt including Compound A' and the medicament comprising Compound A or Compound A salt including Compound A' which provides plasma concentration range from more than 2 ng/mL to equal to or less than 116 ng/mL for a duration from 1 hour to 24 hours would be more preferable for the treatment of SUI to induce fewer adverse effects.

The administration of Compound B or Compound B salt including Compound B' and the medicament comprising Compound B or Compound B salt including Compound B' which provides plasma concentration at more than 0.32 ng/mL and less than 43 ng/mL for a duration from 1 hour to 24 hours can be used for the treatment of SUI in an SUI patient with a normal, lower or larger BMI.

Furthermore, the administration of Compound B or Compound B salt including Compound B' and the medicament comprising Compound B or Compound B salt including Compound B' which provides plasma concentration range from more than 0.32 ng/mL to equal to or less than 24 ng/mL for a duration from 1 hour to 24 hours are more preferable for the treatment of SUI to induce fewer adverse effects.

**Example 11: Effects of Compound A' and Compound B' on rat anus-closing functions measured by high speed infusion of saline into the rectum**

**Method**

Thirty six female SD rats were used. After overnight fasting, rats were intraperitoneally administered with urethane for anesthesia, and isoflurane inhalation was further added during surgery. The abdomen was incised to expose the rectum, and a small incision of the rectum was made. A polyethylene catheter (SP61, Natsume) was inserted into the rectum through the rectum incision, and the tip of the catheter was positioned around 5 mm from the anal orifice. The other end of the catheter was connected with a pressure transducer and an infusion pump via three-way stopcocks, and the change in pressure in the rectum was measured using the pressure transducer, an electric signal amplifier, and an analog-to-digital converter. For administrating drugs, a polyethylene catheter (SP45, Natsume) was inserted into the femoral vein after exposing the vein by small incision.

After recovery period from surgery, while saline was infused at a speed of 0.1 mL/sec into the rectum using an infusion pump, change in the measured pressure - was observed. The infusion was stopped when the leakage of the saline from the anal orifice was observed. In the procedure, sudden decrease in the pressure was observed during the infusion with the leakage of saline from the anal orifice. The peak value of the pressure recorded in the rectum during the course of the procedures was defined as "an Anal LPP" in this Example. This measurement was repeated at least twice to determine baseline Anal LPP, and after obtaining stable baseline, saline at 1 mL/kg, Compound A' at 0.3 or 1 mg/mL/kg, or Compound B' at 1 mg/mL/kg was intravenously administered, respectively. The Anal LPP was again measured in the same way 5 minutes after drug administration. The difference in Anal LPP from the Pre value (increased amount of Anal LPP) was calculated. Regarding change in Anal LPP before and after drug administration, the differences between the mean values of the drug-treated and vehicle-treated groups were analyzed by the one-tailed Williams' test or Student's *t*-test.

**Results**

The high speed infusion of saline into the rectum increased the measured pressure before leaking saline from the anal orifice. The intravenous administration of Compound A' dose-dependently increased the Anal LPP (see Table 16), and the increase in Anal LPP at 1 mg/kg was statistically significant. The intravenous administration of Compound B' significantly increased the Anal LPP (see Table 16). These results suggest that both Compound A' and Compound B' increase anus-closing functions.

[Table 16]

Table 16: Effects of Compound A' and Compound B' on Anal LPP in rats.

| Drug | Dose (mg/kg, i.v.) | Anal LPP Increase (cmH$_2$O) |
|---|---|---|
| Experiment 1 | | |
| Vehicle | - | 1.0 ± 4.1 |
| Compound A' | 0.3 | 6.2 ± 3.1 |
| Compound A' | 1 | 30.2 ± 9.6* |
| Experiment 2 | | |
| Vehicle | - | -3.8 ± 1.9 |
| Compound B' | 1 | 13.8 ± 5.7 # |

Data are expressed as mean ± SEM from 7 or 8 rats.

*: $p \leq 0.025$: The Anal LPP increase was compared with that of the vehicle-treated group using the one-tailed Williams' test.

#: $p \leq 0.05$: The Anal LPP increase was compared with that of the vehicle-treated group using the Student's *t*-test.

**Example 12**: **Influence of an antagonist on Compound A'-induced rat anus-closing function increasing effects**

In this Example, a 5-HT$_{2C}$ receptor antagonist SB 242084 (Abcam, Lot No. Ab120519) was used to investigate if the anus-closing function increasing effect of Compound A' is mediated by 5-HT$_{2C}$ receptor stimulations.

**Method**

Eighteen female SD rats were used. After overnight fasting, rats were intraperitoneally administered with urethane for anesthesia, and isoflurane inhalation was added during surgery. The abdomen was incised to expose the rectum, and a small incision of the rectum was made. A polyethylene catheter (SP61, Natsume) was inserted into the rectum through the rectum incision, and the tip of the catheter was positioned around 5 mm from the anal orifice. The other end of the catheter was connected with a pressure transducer and an infusion pump via three-way stopcocks, and the change in pressure in the rectum was measured using the pressure transducer, an electric signal amplifier, and an analog-to-digital converter. For administrating drugs, a polyethylene catheter (SP45, Natsume) was inserted into the femoral vein after exposing the vein by small incision.

After recovery period from surgery, while saline was infused at a speed of 0.1 mL/sec into the rectum using an infusion pump, change in the measured pressure - was observed. The infusion was stopped when the leakage of the saline from the anal orifice was observed. The peak value of the pressure recorded in the rectum during the course of the procedures was defined as "an Anal LPP" in this Example. This measurement was repeated twice to determine baseline Anal LPP, and after obtaining stable baseline, vehicle at 0.5 mL/kg or SB242084 at 0.3 mg/0.5mL/kg was intravenously administered. SB242084 was dissolved in a mixed solution of dimethylacetamide and polyethylene glycol 400 (1:1). Five minutes later, Compound A' at 1 mg/mL/kg was intravenously administered. The Anal LPP was again measured in the same way 5 minutes after the administration with Compound A'. The difference in Anal LPP from the Pre value (increased amount of Anal LPP) was calculated. In order to analyze the influence of the antagonists on the effect of Compound A', the differences in the mean Anal LPP values between the SB 242084- and Compound A'-administered group, and the vehicle- and Compound A'-administered group were analyzed by Student's *t*-test.

**Results**

Intravenous administration of Compound A' significantly increased Anal LPP ($9.2\pm3.0$ cmH$_2$O increase; n=9), whereas the Anal LPP increasing effect of Compound A' was not observed in the group in which SB242084 was intravenously treated before administering Compound A' ($-2.6 \pm4.1$ cmH$_2$O increase; n=9; p<0.05), indicating that the effects of Compound A' is mediated by 5-HT$_{2C}$ receptor stimulations.

[Industrial Applicability]

The therapeutic drug according to the present invention is useful because the therapeutic drug can be administered to stress urinary incontinence patients, regardless of the presence or absence of obesity, produces only fewer adverse effects, and may prevent reduction in quality of life (QOL) of patients during the treatment of their stress urinary incontinence. Further, the therapeutic drug according to the present invention is useful because the therapeutic drug can be administered to fecal incontinence patients, regardless of the presence or absence of obesity, produces only fewer adverse effects, and may prevent reduction in quality of life (QOL) of patients during the treatment of their incontinence of fees.

**Claims**

1. A medicament for use in treating or preventing incontinence of feces, comprising a 5-HT$_{2C}$ receptor agonist.

2. The medicament according to claim 1, wherein the 5-HT$_{2C}$ receptor agonist is *N*-methyl-*N*-(1-methylethyl)-6,7,8,9-tetrahydropyrazino[2,3-*f*][1,4]oxazepine-3-amine or a salt thereof.

3. The medicament according to Claim 1, wherein the 5-HT$_{2C}$ receptor agonist is (*R*)-8-chloro-1-methyl-2,3,4,5-tetrahydro-1H-3-benzazepine or a salt thereof.

4. A medicament for use in treating stress urinary incontinence or incontinence of feces, comprising a 5-HT$_{2C}$ receptor agonist, wherein the medicament is administered at a dosage lower than the minimum dosage of the agonist as an anti-obesity drug.

5. A medicament for use in treating stress urinary incontinence or incontinence of feces, comprising a therapeutically effective amount of a 5-HT$_{2C}$ receptor agonist for treating stress urinary incontinence or incontinence of feces, wherein the therapeutically effective amount of the agonist shows no body weight lowering effect.

6. The medicament according to Claim 4 or 5, wherein the 5-HT$_{2C}$ receptor agonist is *N*-methyl-*N*-(1-methylethyl)-6,7,8,9-tetrahydropyrazino[2,3-*f*][1,4]oxazepine-3-amine or a salt thereof.

7. The medicament according to Claim 4 or 5, wherein the 5-HT$_{2C}$ receptor agonist is (*R*)-8-chloro-1-methyl-2,3,4,5-tetrahydro-1H-3-benzazepine or a salt thereof.

8. The medicament according to Claim 3, wherein the medicament is administered such that the plasma concentration of the 5-HT$_{2C}$ receptor agonist is more than 2 ng/mL and less than 203 ng/mL for the duration from 1 hour to 24 hours.

9. The medicament according to Claim 3, wherein the medicament is administered such that the plasma concentration of the 5-HT$_{2C}$ receptor agonist is more than 2 ng/mL and equal or less than 116 ng/mL for the duration from 1 hour to 24 hours.

10. The medicament according to Claim 4, wherein the medicament is administered such that the plasma concentration of the serotonin 5-HT$_{2C}$ receptor agonist is more than 0.32 ng/mL and less than 43 ng/mL for the duration from 1 hour to 24 hours.

11. The medicament according to Claim 4, wherein the medicament is administered such that the plasma concentration of the 5-HT$_{2C}$ receptor agonist is more than 0.32 ng/mL and equal or less than 24 ng/mL for the duration from 1 hour to 24 hours.

12. A method of treating or preventing incontinence of feces in a subject in need thereof, comprising administering to the subject an effective amount of a 5-HT$_{2C}$ receptor agonist.

13. A method of treating or preventing stress urinary incontinence or incontinence of feces in a subject in need thereof, comprising
administering to the subject an effective amount of a 5-HT$_{2C}$ receptor agonist, wherein the effective amount is a dosage lower than the minimum dosage of the agonist as an anti-obesity drug.

14. A method of treating or preventing stress urinary incontinence or incontinence of feces in a subject in need thereof, comprising
administering to the subject an effective amount of a 5-HT$_{2C}$ receptor agonist, wherein the effective amount of the agonist shows no body weight lowering effect.

15. A 5-HT$_{2C}$ receptor agonist for use in a method of treating or preventing incontinence of feces.

16. A 5-HT$_{2C}$ receptor agonist for use in a method of treating or preventing stress urinary incontinence or incontinence of feces, **characterized in that** the agonist is administered at a dosage lower than the minimum dosage of the agonist as an anti-obesity drug.

17. A 5-HT$_{2C}$ receptor agonist for use in a method of treating or preventing stress urinary incontinence or incontinence of feces, **characterized in that** the agonist is administered at a dosage that show no body weight lowering effect.

18. Use of a 5-HT$_{2C}$ receptor agonist in a manufacture of a medicament for use in treating or preventing incontinence of feces.

19. Use of a 5-HT$_{2C}$ receptor agonist in a manufacture of a medicament for use in treating or preventing stress urinary incontinence or incontinence of feces, **characterized in that** the agonist is administered at a dosage lower than the minimum dosage of the agonist as an anti-obesity drug.

20. Use of a 5-HT$_{2C}$ receptor agonist in a manufacture of a medicament for use in treating or preventing stress urinary incontinence or incontinence of feces, **characterized in that** the agonist is administered at a dosage that show no body weight lowering effect.

[Figure 1]

Intravesical pressure

Urethra-closing
reflex response

Pre

Post

50 cmH$_2$O

40 cmH$_2$O

30 s

30 s

[Figure 2]

[Figure 3]

$$F2 = F2_{int} * e^{(Dose * F2exp)}$$

$$F1 = 1$$

※ = between subject variability included

$$CL_{tot} = C * (CL + V_{max}/(K_m + C))$$

[Figure 4]

EP 3 733 204 A1

[Figure 5]

[Figure 6]

[Figure 7]

[Figure 8]

[Figure 9]

[Figure 10]

[Figure 11]

EP 3 733 204 A1

[Figure 12]

with placebo

90% prediction interval in PK-PD plots with placebo

[Figure 13]

# without placebo

## 90% prediction interval in PK-PD plots without placebo

EP 3 733 204 A1

[Figure 14]

## INTERNATIONAL SEARCH REPORT

| International application No. |
|---|
| PCT/JP2018/048229 |

A. CLASSIFICATION OF SUBJECT MATTER
Int.Cl. A61K45/00(2006.01)i, A61K31/55(2006.01)i, A61K31/553(2006.01)i,
A61P1/12(2006.01)i, A61P13/00(2006.01)i, A61P43/00(2006.01)i

According to International Patent Classification (IPC) or to both national classification and IPC

B. FIELDS SEARCHED

Minimum documentation searched (classification system followed by classification symbols)
Int.Cl. A61K45/00, A61K31/55, A61K31/553, A61P1/12, A61P13/00, A61P43/00

Documentation searched other than minimum documentation to the extent that such documents are included in the fields searched
Published examined utility model applications of Japan　　　　　1922–1996
Published unexamined utility model applications of Japan　　　　1971–2019
Registered utility model specifications of Japan　　　　　　　　1996–2019
Published registered utility model applications of Japan　　　　1994–2019

Electronic data base consulted during the international search (name of data base and, where practicable, search terms used)
JSTPlus/JMEDPlus/JST7580 (JDreamIII), CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2009-520776 A (UROSPHERE SAS) 28 May 2009, entire text, in particular, claim 10, paragraphs [0016], [0027], [0031], [0033], [0034], [0037], examples 4, 5 & US 2008/0305162 A1 claim 23, paragraphs [0005], [0016], [0030], [0034], [0036], [0037], [0040], examples 4, 5 & WO 2007/074291 A2 & EP 1965800 A2 | 1, 4, 5, 10-20<br>1-20 |

☒ Further documents are listed in the continuation of Box C.　　☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>25 January 2019 (25.01.2019) | Date of mailing of the international search report<br>12 February 2019 (12.02.2019) |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

## INTERNATIONAL SEARCH REPORT

| International application No. |
|---|
| PCT/JP2018/048229 |

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2002-522496 A (MILKHAUS LABORATORY, INC.) 23 July 2002, entire text, in particular, paragraphs [0006], [0015], [0018]-[0022], [0026] & US 6156780 A column 1, lines 36-44, column 2, lines 38-52, examples 2-6, 10 & WO 2000/009128 A1 & EP 1105127 A1 | 1, 3-5, 7-20 |
| Y | | 1, 2, 4-6, 8-20 |
| X | JP 2012-530051 A (TAKEDA PHARMACEUTICAL COMPANY LIMITED) 29 November 2012, entire text, in particular, paragraphs [0002], [0319]-[0321], [0414]-[0416], [0419], [0420], [0422], [0423] & US 2010/0317651 A1 paragraphs [0003], [0773]-[0778], [0904]-[0906], [0911], [0911], [0912], [0916]-[0920] & EP 2442870 A1 | 4, 5, 10-17, 19, 20 |
| Y | | 1-20 |
| X | STORER, R. I. et al., "Multiparameter optimization in CNS drug discovery: design of pyrimido[4,5-d]azepines as potent 5-hydroxytryptamine 2C (5-HT2C) receptor agonists with exquisite functional selectivity over 5-HT2A and 5-HT2B receptors.", Journal of Medicinal Chemistry, 2014, vol. 57, no. 12, pp. 5258-5269, ISSN:0022-2623 abstract, page 5258, left column, line 2 to page 5258, right column, line 4, page 5259, left column, lines 11-19, page 5263, left column, line 1 to page 5263, right column, line 6, page 5265, right column, lines 4-25, fig. 1, 7, 8, table 3 | 4, 5, 10-17, 19, 20 |
| Y | | 1, 3-5, 7-20 |
| X | ANDREWS, M. D. et al., "Pyrimido [4, 5-d] azepines as potent and selective 5-HT2C receptor agonists: design, synthesis, and evaluation of PF-3246799 as a treatment for urinary incontinence.", Bioorganic and Medicinal Chemistry Letters, 2011, vol. 21, no. 9, pp. 2715-2720, ISSN: 0960-894X abstract, page 2715, left column, line 1 to page 2715, right column, line 4, page 2719, left column, lines 1-9, page 2719, left column, line 16 to page 2719, right column, line 9, fig. 4, table 2 | 4, 5, 10-17, 19, 20 |
| Y | | 1, 3-5, 7-20 |
| X | BRENNAN, P. E. et al., "Discovery of a novel azepine series of potent and selective 5-HT2C agonists as potential treatments for urinary incontinence.", Bioorganic and Medicinal Chemistry Letters, 2009, vol. 19, no. 17, pp. 4999-5003, ISSN: 0960-894X abstract, page 5002, right column, lines 3-13, fig. 1, table 3 | 4, 5, 10-17, 19, 20 |

Form PCT/ISA/210 (continuation of second sheet) (January 2015)

**INTERNATIONAL SEARCH REPORT**

International application No.

PCT/JP2018/048229

C (Continuation). DOCUMENTS CONSIDERED TO BE RELEVANT

| Category* | Citation of document, with indication, where appropriate, of the relevant passages | Relevant to claim No. |
|---|---|---|
| X | JP 2017-031088 A (TORAY INDUSTRIES, INC.) 09 February 2017, paragraphs [0059]-[0067], fig. 1 (Family: none) | 4, 5, 10-17, 19, 20 |
| Y | | 1, 3-5, 7-20 |
| X | JP 2017-100953 A (TORAY INDUSTRIES, INC.) 08 June 2017, paragraphs [0138]-[0147], fig. 1 (Family: none) | 4, 5, 10-17, 19, 20 |
| Y | | 1, 3-5, 7-20 |
| X | JP 2016-222570 A (TORAY INDUSTRIES, INC.) 28 December 2016, paragraphs [0078]-[0086], fig. 1 (Family: none) | 4, 5, 10-17, 19, 20 |
| Y | | 1, 3-5, 7-20 |
| Y | HIGGINS, G. A. et al., "Characterization of the 5-HT2C receptor agonist lorcaserin on efficacy and safety measures in a rat model of diet-induced obesity.", Pharmacology Research and Perspectives, 2014, vol. 3, no. 1, e00084, pp. 1-14, ISSN:2052-1707 abstract, page 4, left column, line 6 from the bottom to page 4, right column, line 20, page 6, left column, line 9 from the bottom to page 6, right column, line 7, fig. 2A-2D | 1, 3, 5, 7-20 |
| Y | US 2009/0076565 A1 (SURWIT, Earl A.) 19 March 2009, paragraphs [0001], [0025], [0032], [0033], [0052], [0078], [0090]-[0092] (Family: none) | 1, 3, 5, 7-20 |
| Y | WO 2006/022420 A1 (TAKEDA PHARMACEUTICAL COMPANY LIMITED) 02 March 2006, examples 1, 3 & US 2007/0274913 A1 examples 1, 3 & EP 1792629 A1 | 1, 3, 5, 7-20 |
| Y | WO 2007/132841 A1 (TAKEDA PHARMACEUTICAL COMPANY LIMITED) 22 November 2007, paragraphs [0027], [0165], [0567]-[0571] & US 2009/0131402 A1 paragraphs [0016], [0017], [0219]-[0221], [1011]-[1017] & EP 2018863 A1 | 1, 3, 5, 7-20 |
| Y | WO 2008/108445 A1 (TAKEDA PHARMACEUTICAL COMPANY LIMITED) 12 September 2008, paragraphs [0509]-[0517] & US 2010/0087418 A1 paragraphs [0877]-[0886] & EP 2123644 A1 | 1, 3, 5, 7-20 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010147226 A
- WO 2006022420 A
- WO 2003086306 A
- WO 2007132841 A
- WO 2011111817 A
- WO 2008108445 A
- EP 0572863 A
- EP 0863136 A
- EP 1213017 A
- US 3253989 A
- US 3676558 A
- US 3652588 A
- US 4082844 A
- US 4971969 A
- US 5494928 A
- US 5646173 A
- US 6310208 B
- WO 9742183 A
- WO 9830546 A
- WO 9830548 A
- WO 9833504 A
- WO 9902159 A
- WO 9943647 A
- US 6281243 B
- WO 0012475 A
- US 6380238 B
- WO 0012502 A
- US 6365598 B
- WO 0012510 A
- US 6433175 B
- WO 0012481 A
- US 6552062 B
- WO 0012482 A
- WO 0016761 A
- WO 0017170 A
- WO 0028993 A
- WO 0035922 A
- US 6372745 B
- WO 0044737 A
- WO 0044753 A
- WO 0064899 A
- WO 0077001 A
- WO 0077002 A
- WO 0077010 A
- WO 0076984 A
- US 6465467 B
- WO 0109111 A
- WO 0109122 A
- WO 0109123 A
- US 6638936 B
- WO 0109126 A
- WO 0112602 A
- WO 0112603 A
- US 6706750 B
- WO 0140183 A
- WO 0166548 A
- US 6583134 B
- WO 0170207 A
- WO 0170223 A
- WO 0172752 A
- US 6734301 B
- WO 0183487 A
- WO 0204456 A
- WO 0204457 A
- WO 0208178 A
- WO 0210169 A
- WO 0224700 A
- WO 0224701 A
- WO 0236596 A
- WO 0240456 A
- WO 0240457 A
- WO 0242304 A
- WO 0244152 A
- US P6479534 B
- WO 0248124 A
- WO 0251844 A
- US P6610685 B
- WO 0259124 A
- WO 0259127 A
- WO 0259129 A
- WO 0272584 A
- WO 0274746 A
- WO 0283863 A
- WO 0298350 A
- WO 0298400 A
- WO 0298860 A
- WO 0300663 A
- WO 0300666 A
- WO 0304501 A
- WO 0306466 A
- WO 0311281 A
- WO 0314118 A
- WO 0314125 A
- WO 0324976 A
- WO 0328733 A
- WO 0333497 A
- WO 0357161 A
- WO 0357213 A

- WO 0357673 A
- WO 0357674 A
- WO 0362205 A
- WO 0364423 A
- WO 0386306 A
- WO 0387086 A
- WO 0389409 A
- WO 0391250 A
- WO 0391251 A
- WO 0391257 A
- WO 0397636 A
- WO 0400829 A
- WO 0400830 A
- US 6667303 B
- WO 0456324 A
- WO 0478718 A
- WO 0481010 A
- WO 04087156 A
- WO 0487662 A
- WO 0487692 A
- WO 0489897 A
- WO 04096196 A
- WO 0496201 A
- WO 04112769 A
- US 2004192754 A
- WO 0500849 A
- WO 0503096 A
- EP 1500391 A
- WO 0516902 A
- WO 0519180 A
- US 2005080074 A
- WO 0540146 A
- WO 0541856 A
- WO 0542490 A
- WO 0542491 A
- WO 0544812 A
- WO 05082859 A
- WO 05000309 A
- WO 05019179 A
- WO 05121113 A
- WO 05049623 A
- WO 05105082 A
- WO 05109987 A
- WO 05113535 A
- US 2006003990 A
- US 2006014777 A
- US 2006014778 A
- WO 06000902 A
- WO 06028961 A
- WO 06020817 A
- WO 06020049 A
- WO 06019940 A
- WO 06004931 A
- US 2006025601 A
- WO 06044762 A
- WO 06047032 A
- WO 06050007 A
- WO 06052887 A
- WO 06077025 A
- WO 06065600 A
- WO 06103511 A
- WO 06116165 A
- WO 06047228 A
- WO 06117304 A
- US 2006241172 A
- US 2006241176 A
- WO 06116136 A
- WO 06116148 A
- WO 06116151 A
- WO 06116171 A
- WO 06116170 A
- WO 06116218 A
- WO 06116169 A
- US 2007032481 A
- WO 07025144 A
- WO 07028082 A
- WO 07028132 A
- WO 07028131 A
- WO 07028083 A
- WO 07030150 A
- US 20070049613 A
- US 8404675 B
- US 8846906 B
- US 20070179155 A
- US 20070275949 A
- US 20080009478 A
- US 20080119477 A
- US 20080255137 A
- US 20180186797 A
- US 20180214455 A
- WO 2015066344 A
- WO 2018035477 A
- US 8153621 B
- JP H09263545 B
- WO 2012030927 A

**Non-patent literature cited in the description**

- *Expert Opinion on Investigational Drugs,* 2006, vol. 15, 257-266
- LORCASERIN HYDROCHLORIDE, Briefing Document for FDA Advisory Committee Meeting. Center for Drug Evaluation and Research, 06 April 2012
- *Clinical Therapeutics,* 2016, vol. 38 (10), 2227-2238
- FDA Briefing Document : NDA 22529. FDA, 16 September 2010
- *World Journal of Urology,* 2012, vol. 30, 437-43

- *British Journal of Urology,* 1997, vol. 79, 892-7
- *Central European Journal of Urology,* 2011, vol. 64, 110-9
- *Journal of Clinical Oncology,* 1994, vol. 12, 213-225
- *British Journal of Cancer,* 1993, vol. 68, 314-318
- Japanese Pharmacopoeia
- **BOY S. et al.** *Eur. Urol.,* 2006, vol. 50 (1), 119-125
- **YONO M.** LUTS: Lower Urinary Tract Symptoms. John Wiley & Sons Australia, Ltd, 2014, vol. 2